# EUROPÄISCHE PATENTANMELDUNG

(11) **EP 1 938 686 A1**
(43) Veröffentlichungstag der Anmeldung: **02.07.2008**
(21) Anmeldenummer: 06027044.4
(22) Anmeldetag: 29.12.2006
(51) Int. Cl.: A01N 43/56, C07D 401/04, C07D 231/12, A01P 13/00, A01P 21/00

(54) **Substituierte 1-(3-Pyridinyl)pyrazol-4-yl-essigsäuren, Verfahren zu deren Herstellung und deren Verwendung als Herbizide und Pflanzenwachstumsregulatoren**

(71) Anmelder: Bayer CropScience AG, 40789 Monheim (DE)
(72) Erfinder: Jakobi, Harald, Dr., 60598 Frankfurt (DE); Ort, Oswald, Dr., 51375 Leverkusen (DE); Hills, Martin, 65510 Idstein (DE); Kehne, Heinz, Dr., 65719 Hofheim (DE); Rosinger, Christopher, Dr., 65719 Hofheim (DE); Feucht, Dieter, Dr., 65760 Eschborn (DE)

(57) **Zusammenfassung**

Die Erfindung betrifft die Verwendung von Verbindungen der Formel (I) oder deren Salzen, 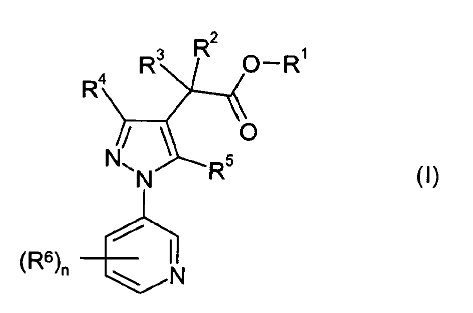 in welcher
R¹, R², R³, R⁴, R⁵, R⁶ und n wie in Anspruch 1 definiert sind
als Herbizide und Pflanzenwachstumsregulatoren.

Die Erfindung betrifft auch neue Verbindungen der Formel (I) und ihre Salze (siehe Anspruch 5.

## Beschreibung

Die Erfindung betrifft das technische Gebiet der Herbizide und Pflanzenwachstumsregulatoren, beispielsweise der Herbizide zur Bekämpfung von Unkräutern und Ungräsern in Nutzpflanzenkulturen oder der Pflanzenwachstumsregulatoren, welche zur Beeinflussung des Wachstums von Kulturpflanzen eingesetzt werden können.

Bisher bekannte Pflanzenschutzmittel zur selektiven Bekämpfung von Schadpflanzen in Nutzpflanzenkulturen oder Wirkstoffe zur Bekämpfung von unerwünschtem Pflanzenwuchs weisen bei ihrer Anwendung teilweise Nachteile auf, sei es (a) dass sie keine oder aber eine unzureichende herbizide Wirkung gegen bestimmte Schadpflanzen, (b) ein zu geringes Spektrum der Schadpflanzen, das mit einem Wirkstoff bekämpft werden kann, (c) zu geringe Selektivität in Nutzpflanzenkulturen oder ein toxikologisch ungünstiges Profil besitzen. Andere Wirkstoffe, die als Pflanzenwachstumsregulatoren bei einigen Nutzpflanzen eingesetzt werden können, führen bei anderen Nutzpflanzen zu unerwünscht verminderten Ernteerträgen oder sind mit der Kulturpflanze nicht oder nur in einem engen Aufwandmengenbereich verträglich. Andere bekannte Wirkstoffe lassen sich wegen schwer zugänglicher Vorprodukte und Reagenzien im industriellen Maßstab nicht wirtschaftlich herstellen oder besitzen nur unzureichende chemische Stabilitäten.
Bei anderen Wirkstoffen hängt die Wirkung zu stark von Umweltbedingungen, wie Wetter- und Bodenverhältnissen ab.

Aus den genannten Gründen besteht weiterhin ein Bedarf nach alternativen wirkungsstarken Herbiziden für die selektive Anwendung in Pflanzenkulturen oder die Anwendung auf Nichtkulturland. Auch ist es wünschenswert, alternative chemische Wirkstoffe bereitzustellen, die gegebenenfalls mit Vorteilen als Herbizide oder Pflanzenwachstumsregulatoren eingesetzt werden können.

Gegenstand der vorliegenden Erfindung ist die Verwendung von Verbindungen der Formel (I) oder deren Salzen, 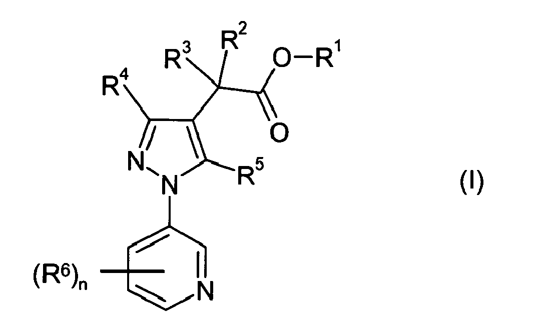 in welcher
- R¹: Wasserstoff oder einen hydrolysierbaren Rest, vorzugsweise Wasserstoff oder einen gegebenenfalls substituierten Kohlenwasserstoffrest oder einen gegebenenfalls substituierten Heterocyclylrest, wobei jeder der beiden letztgenannten kohlenstoffhaltigen Reste inklusive Substituenten 1 bis 30 C-Atome, vorzugsweise 1 bis 24 C-Atome, insbesondere 1 bis 20 C-Atome aufweist, oder einen Rest der Formel SiR^{a}R^{b}R^{c}, -NR^{a}R^{b} oder -N=CR^{c}R^{d},
wobei in den letztgenannten 3 Formel jeder der Reste R^{a}, R^{b}, R^{c} und R^{d} unabhängig voneinander Wasserstoff oder einen gegebenenfalls substituierten Kohlenwasserstoffrest bedeutet oder R^{a} und R^{b} zusammen mit dem N-Atom einen 3- bis 9-gliedrigen Heterocyclus, welcher zusätzlich zum N-Atom ein oder zwei weitere Heteroringatome aus der Gruppe N, O und S enthalten kann und welcher unsubstituiert oder substituiert ist, bedeuten oder R^{c} und R^{d} zusammen mit dem C-Atom einen 3- bis 9-gliedrigen carbocyclischen Rest oder einen heterocyclischen Rest, welcher 1 bis 3 Heteroringatome aus der Gruppe N, O und S enthalten kann, wobei der carbocyclische oder heterocyclischen Rest unsubstituiert oder substituiert ist, bedeuten, wobei jeder der Reste R^{a}, R^{b}, R^{c} und R^{d} inklusive Substituenten bis 30 C-Atome, vorzugsweise bis 24 C-Atome, insbesondere bis 20 C-Atome aufweist,
- R²: Wasserstoff, Halogen oder (C₁-C₆)Alkyl, das unsubstituiert oder durch einen oder mehrere Reste aus der Gruppe, welche aus Halogen, (C₁-C₄)Alkoxy, (C₁-C₄)Alkylthio und (C₁-C₄)Haloalkoxy besteht, substituiert ist,
- R³: Wasserstoff, Halogen oder (C₁-C₆)Alkyl, das unsubstituiert oder durch einen oder mehrere Reste aus der Gruppe, welche aus Halogen, (C₁-C₄)Alkoxy, (C₁-C₄)Alkylthio und (C₁-C₄)Haloalkoxy besteht, substituiert ist, oder
- R² und R³: zusammen mit dem C-Atom, an das sie gebunden sind, einen carbocyclischen gesättigten oder teilweise ungesättigten Ring mit 3 bis 6 C-Atomen, der unsubstituiert oder durch einen oder mehrere Reste aus der Gruppe Halogen und (C₁-C₄)Alkyl substituiert ist, und
- R⁴: Wasserstoff, Halogen, Cyano, (C₁-C₆)Alkyl, (C₂-C₆)Alkenyl oder (C₂-C₆)Alkinyl, wobei jeder der letztgenannten drei Reste unsubstituiert oder durch einen oder mehrere Reste aus der Gruppe bestehend aus Halogen, Cyano, (C₁-C₄)Alkoxy, (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₉)Cycloalkyl substituiert ist, oder
(C₃-C₉)Cycloalkyl, (C₅-C₉)Cycloalkenyl oder (C₅-C₉)Cycloalkinyl, wobei jeder der 3 letztgenannten Reste unsubstituiert oder durch einen oder mehrere Reste aus der Gruppe bestehend aus Halogen, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy und (C₁-C₄)Alkylthio substituiert ist, oder
Phenyl, das unsubstituiert oder durch einen oder mehrere Reste aus der Gruppe bestehend aus Halogen, Cyano, Nitro, Carboxy, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy, (C₁-C₄)Alkoxy-(C₁-C₄)alkoxy, (C₁-C₄)Alkylthio, (C₁-C₄)Alkanoyl, (C₁-C₄)Haloalkanoyl, [(C₁-C₄)Alkoxy]-carbonyl und [(C₁-C₄)Haloalkoxy]-carbonyl substituiert ist, oder
(C₁-C₆)Alkanoyl, das unsubstituiert oder durch einen oder mehrere Reste aus der Gruppe bestehend aus Halogen, Cyano, (C₁-C₄)Alkoxy, (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₆)Cycloalkyl substituiert ist, oder
[(C₁-C₄)Alkoxy]-carbonyl, unsubstituiert oder durch einen oder mehrere Reste aus der Gruppe bestehend aus Halogen, Cyano, (C₁-C₄)Alkoxy, (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₆)Cycloalkyl substituiert ist, oder
[(C₃-C₉)Cycloalkoxy]-carbonyl, das unsubstituiert oder durch einen oder mehrere Reste aus der Gruppe bestehend aus Halogen, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy und (C₁-C₄)Alkylthio substituiert ist,
- R⁵: einen Arylrest, der unsubstituiert oder substituiert ist und inklusive Substituenten 6 bis 30 C-Atome, vorzugsweise 6 bis 24 C-Atome, insbesondere 6 bis 20 C-Atome aufweist, oder
einen heteroaromatischen Rest mit 1 bis 4 Heteroringatomen aus der Gruppe N, O und S, der unsubstituiert oder substituiert ist und inklusive Substituenten 1 bis 30 C-Atome, vorzugsweise 1 bis 24 C-Atome, insbesondere 1 bis 20 C-Atome aufweist, und
- (R⁶)ₙ: n Substituenten R⁶, wobei R⁶, im Falle dass n = 1 ist, oder jeder der Substituenten R⁶ unabhängig voneinander, im Falle dass n größer als 1 ist, einen Rest Halogen, Hydroxy, Amino, Nitro, Carboxy, Cyano, Carbamoyl, (C₁-C₆)Alkyl, (C₁-C₆)Haloalkyl, (C₁-C₄)Alkoxy-(C₁-C₄)alkyl, (C₁-C₄)Alkylthio-(C₁-C₄)alkyl, Mono- oder Di-[(C₁-C₄)alkyl]-aminoalkyl, Hydroxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₁-C₆)Haloalkoxy, (C₁-C₄)Alkoxy-(C₁-C₄)alkoxy, (C₁-C₆)Alkylthio, [(C₁-C₆)Alkoxy]-carbonyl, [(C₁-C₆)Haloalkoxy]-carbonyl, (C₁-C₆)Alkanoyl, (C₁-C₆)Haloalkanoyl, Mono- oder Di-[(C₁-C₄)alkyl]-aminocarbonyl, Mono- oder Di-[(C₁-C₆)acyl]-amino, Mono- oder Di-[(C₁-C₄)alkyl]-amino, N-[(C₁-C₆)Acyl]-N-[(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, (C₃-C₉)Cycloalkyl oder (C₅-C₉)Cycloalkenyl, wobei jeder der letztgenannten beiden Reste unsubstituiert oder durch einen oder mehrere Reste aus der Gruppe bestehend aus Halogen, (C₁-C₄)Alkyl und (C₁-C₄)Haloalkyl substituiert ist, bedeutet, und
- n: 0, 1, 2, 3 oder 4
bedeuten,
als Herbizide oder Pflanzenwachstumsregulatoren.

Die Verbindungen der Formel (I) können durch Anlagerung einer geeigneten anorganischen oder organischen Säure, wie beispielsweise HCl, HBr, H₂SO₄ oder HNO₃, aber auch Oxalsäure oder Sulfonsäuren an eine basische Gruppe, wie z.B. Amino oder Alkylamino, Salze bilden. Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden. Salze können ebenfalls dadurch gebildet werden, dass bei geeigneten Substituenten, wie z.B. Sulfonsäuren oder Carbonsäuren, der Wasserstoff durch ein für die Landwirtschaft geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze.

In der Formel (I) und allen nachfolgenden Formeln werden Bezeichnungen für chemische Reste verwendet, die insbesondere die nachstehend erläuterten Bedeutungen haben.

Ein hydrolysierbarer Rest (siehe Definition von R¹) bedeutet einen unter den Anwendungsbedingungen hydrolysierbaren Rest, beispielsweise einen schon in der Spritzbrühe oder insbesondere unter den physiologischen Bedingungen in Pflanzen hydrolysierbaren Rest, wobei eine Verbindung der Formel (I) mit der Carbonsäureestergruppe -CO-OR¹ (R¹ ungleich Wasserstoff) zur Verbindung der Formel (I) mit der Carbonsäuregruppe -CO-OH (d. h. Verbindung (I) mit R¹ = H) hydrolysiert wird. In der Definition der hydrolysierbaren Reste sind auch ausdrücklich die Reste mit R¹ = Kohlenwasserstoffrest oder Heterocyclylrest, wobei die beiden letztgenannten Reste unsubstituiert oder substituiert sind, umfasst, auch wenn sie teilweise vergleichsweise langsam hydrolysierbar sind.

Ein Kohlenwasserstoffrest ist ein aliphatischer, cycloaliphatischer oder aromatischer monocyclischer oder, im Falle eines gegebenenfalls substituierten Kohlenwasserstoffrestes, auch ein bicyclischer oder polycyclischer organischer Rest auf Basis der Elemente Kohlenstoff und Wasserstoff, beispielsweise umfassend die Reste Alkyl, Alkenyl, Alkinyl, Cycloalkyl, Cycloalkenyl, Aryl, Phenyl, Naphthyl, Indanyl, Indenyl, etc.; Entsprechendes gilt für Kohlenwasserstoffreste in zusammengesetzte Bedeutungen wie Kohlenwasserstoffoxyresten oder anderen über Heteroatomgruppen gebundene Kohlenwasserstoffresten.

Wenn nicht näher definiert, weisen die Kohlenwasserstoffreste vorzugsweise 1 bis 20 C-Atome, weiter bevorzugt 1 bis 16 C-Atome, insbesondere 1 bis 12 C-Atome auf.

Die Kohlenwasserstoffreste, auch in den speziellen Resten Alkyl, Alkoxy, Haloalkyl, Haloalkoxy, Alkylamino und Alkylthio, sowie die entsprechenden ungesättigten und/oder substituierten Reste können im Kohlenstoffgerüst jeweils geradkettig oder verzweigt sein.

Der Ausdruck "(C₁-C₄)Alkyl" bedeutet eine Kurzschreibweise für Alkyl mit einem bis 4 Kohlenstoffatomen, d. h. umfasst die Reste Methyl, Ethyl, 1-Propyl, 2-Propyl, 1-Butyl, 2-Butyl, 2-Methylpropyl or tert-Butyl. Allgemeine Alkylreste mit einem größeren angegebenen Bereich von C-Atomen, z. B. "(C₁-C₆)Alkyl" umfassen entsprechend auch gradkettige oder verzweigte Alkylreste mit einer größeren Zahl von C-Atomen, d. h. gemäß Beispiel auch die Alkylreste mit 5 und 6 C-Atomen.
Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Vinyl, Allyl, 1-Methyl-2-propenyl, 2-Methyl-2-propenyl, 2-Butenyl, Pentenyl, 2-Methylpentenyl oder Hexenyl group, vorzugsweise Allyl, 1-Methylprop-2-en-1-yl, 2-Methyl-prop-2-en-1-yl, But-2-en-1-yl, But-3-en-1-yl, 1-Methyl-but-3-en-1-yl oder 1-Methyl-but-2-en-1-yl. Alkenyl schließt insbesondere auch geradkettige oder verweigte Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl;

Alkinyl bedeutet z.B. Propargyl, But-2-in-1-yl, But-3-in-1-yl, 1-Methyl-but-3-in-1-yl. Alkinyl schließt insbesondere auch geradkettige oder verweigte Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl.

Ein 3- bis 9-gliedriger carbocyclischer Ring bedeutet (C₃-C₉)Cycloalkyl oder (C₅-C₉)Cycloalkenyl.
(C₃-C₉)Cycloalkyl bedeutet ein carbocyclisches, gesättigtes Ringsystem mit vorzugsweise 3-9 C-Atomen, z.B. Cyclopropyl, Cyclobutyl, Cyclopentyl, Cyclohexyl, Cycloheptyl, Cyclooctyl oder Cyclononyl. Im Falle von substituiertem Cycloalkyl werden cyclische Systeme mit Substituenten umfaßt, wobei die Substituenten auch mit einer Doppelbindung am Cycloalkylrest, z. B. eine Alkylidengruppe wie Methyliden, gebunden sein können.
(C₅-C₉)Cycloalkenyl bedeutet ein carbocyclisches, nicht aromatisches, partiell ungesättigtes Ringsystem mit 5-9 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl. Im Falle von substituiertem Cycloalkenyl gelten die Erläuterungen für substituiertes Cycloalkyl entsprechend

Alkyliden, z. B. auch in der Form (C₁-C₁₀)Alkyliden, bedeutet den Rest eines geradkettigen oder verzweigten Alkans, der über eine Zweifachbindung gebunden ist, wobei die Position der Bindungsstelle noch nicht festgelegt ist. Im Falle eines verzweigten Alkans kommen naturgemäß nur Positionen in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅.

Halogen bedeutet beispielsweise Fluor, Chlor, Brom oder lod. Haloalkyl, -alkenyl und -alkinyl bedeuten durch gleiche oder verschiedene Halogenatome, vorzugsweise aus der Gruppe Fluor, Chlor und Brom, insbesondere aus der Gruppe Fluor und Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; Entsprechendes gilt für Haloalkenyl und andere durch Halogen substituierte Reste.

Aryl bedeutet ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl.

Ein heterocyclischer Rest oder Ring (Heterocyclyl) kann gesättigt, ungesättigt oder heteroaromatisch sein; wenn nicht anders definiert, enthält er vorzugsweise ein oder mehrere, insbesondere 1, 2 oder 3 Heteroatome im heterocyclischen Ring, vorzugsweise aus der Gruppe N, O, und S; vorzugsweise ist er ein aliphatischer Heterocyclylrest mit 3 bis 7 Ringatomen oder ein heteroaromatischer Rest mit 5 oder 6 Ringatomen. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält. Vorzugsweise ist er ein heteroaromatischer Ring mit einem Heteroatom aus der Gruppe N, O und S, beispielsweise Pyridyl, Pyrrolyl, Thienyl oder Furyl; weiterhin bevorzugt ist er ein entsprechender heteroaromatischer Ring mit 2 oder 3 Heteroatomen, z. B. Pyrimidinyl, Pyridazinyl, Pyrazinyl, Triazinyl, Thiazolyl, Thiadiazolyl, Oxazolyl, Isoxazolyl, Pyrazolyl, Imidazolyl und Triazolyl. Weiterhin bevorzugt ist er ein partiell oder vollständig hydrierter heterocyclischer Rest mit einem Heteroatom aus der Gruppe N, O und S, beispielsweise Oxiranyl, Oxetanyl, Oxolanyl (= Tetrahydrofuryl), Oxanyl, Pyrrolinyl, Pyrrolidyl oder Piperidyl,

Weiterhin bevorzugt ist er ein partiell oder vollständig hydrierter heterocyclischer Rest mit 2 Heteroatomen aus der Gruppe N, O und S, beispielsweise Piperazinyl, Dioxolanyl, Oxazolinyl, Isoxazolinyl, Oxazolidinyl, Isoxazolidinyl und Morpholinyl.
Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Bevorzugte Beispiele für Heterocyclyl sind ein heterocyclischer Rest mit 3 bis 6 Ringatomen aus der Gruppe Pyridyl, Thienyl, Furyl, Pyrrolyl, Oxiranyl, 2-Oxetanyl, 3-Oxetanyl, Oxolanyl (= Tetrahydrofuryl), Pyrrolidyl, Piperidyl, insbesondere Oxiranyl, 2-Oxetanyl, 3-Oxetanyl oder Oxolanyl, oder ist ein heterocyclischer Rest mit zwei oder drei Heteroatomen, beispielsweise Pyrimidinyl, Pyridazinyl, Pyrazinyl, Triazinyl, Thiazolyl, Thiadiazolyl, Oxazolyl, Isoxazolyl, Pyrazolyl, Triazolyl, Piperazinyl, Dioxolanyl, Oxazolinyl, Isoxazolinyl, Oxazolidinyl, Isoxazolidinyl oder Morpholinyl.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Substituierte Reste, wie ein substituierter Alkyl-, Alkenyl-, Alkinyl-, Cycloalkyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Alkylsulfonyl und, im Falle cyclischer Reste, auch Alkyl, Haloalkyl, Alkylthio-alkyl, Alkoxy-alkyl, gegebenfalls substituiertes Mono- und Dialkylaminoalkyl und Hydroxy-alkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden substituiert sind.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

### Bevorzugt Substituenten für die Subtituentenebenen sind beispielsweise

Amino, Hydroxy, Halogen, Nitro, Cyano, Mercapto, Carboxy, Carbonamid, SF₅, Aminosulfonyl, Alkyl, Cycloalkyl, Alkenyl, Cycloalkenyl, Alkinyl, Monoalkyl-amino, Dialkyl-amino, N-Alkanoyl-amino, Alkoxy, Alkenyloxy, Alkinyloxy, Cycloalkoxy, Cycloalkenyloxy, Alkoxy-carbonyl, Alkenyloxy-carbonyl, Alkinyloxy-carbonyl, Aryloxycarbonyl, Alkanoyl, Alkenyl-carbonyl, Alkinyl-carbonyl, Aryl-carbonyl, Alkylthio, Cycloalkylthio, Alkenylthio, Cycloalkenylthio, Alkinylthio, Alkylsulfinyl, Alkylsulfonyl, Monoalkyl-aminosulfonyl, Dialkyl-aminosulfonyl, N-Alkylaminocarbonyl, N,N-Dialkyl-aminocarbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino und Benzylamino.

Gegebenenfalls substituiertes Phenyl bedeutet vorzugsweise Phenyl oder Phenyl, das unsubstituiert oder durch einen oder mehrere Reste aus der Gruppe Halogen, Cyano, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkoxy-(C₁-C₄)alkoxy, (C₁-C₄)Alkylthio und Nitro substituiert ist, insbesondere Phenyl, das gegebenenfalls durch einen oder mehrere Reste aus der Gruppe Halogen, (C₁-C₄)Alkyl, (C₁-C₄)Haloalkyl und (C₁-C₄)Alkoxy substituiert ist.

Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Mono- und Dialkyl-amino, Mono- und Diarylamino, Acylamino, N-Alkyl-N-arylamino, N-Alkyl-N-acylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Acyl bedeutet einen Rest einer organischen Säure, der formal durch Abtrennen einer Hydroxygruppe an der Säurefunktion entsteht, wobei der organische Rest in der Säure auch über ein Heteroatom mit der Säurefunktion verbunden sein kann. Beispiele für Acyl sind der Rest -CO-R einer Carbonsäure HO-CO-R und Reste davon abgeleiteter Säuren wie der Thiocarbonsäure, gegebenenfalls N-substituierten Iminocarbonsäuren oder der Rest von Kohlensäuremonoestern, N-substituierter Carbaminsäure, Sulfonsäuren, Sulfinsäuren, N-substituierter Sulfonamidsäuren, Phosphonsäuren oder Phosphinsäuren. Acyl bedeutet beispielsweise Formyl, Alkylcarbonyl wie [(C₁-C₄)Alkyl]-carbonyl, Phenylcarbonyl, Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren. Dabei können die Reste jeweils im Alkyl- oder Phenylteil noch weiter substituiert sein, beispielsweise im Alkylteil durch einen oder mehrere Reste aus der Gruppe Halogen, Alkoxy, Phenyl und Phenoxy; Beispiele für Substituenten im Phenylteil sind die bereits weiter oben allgemein für substituiertes Phenyl erwähnten Substituenten.
Acyl bedeutet vorzugsweise einen Acylrest im engeren Sinne, d. h. einen Rest einer organischen Säure, bei der die Säuregruppe direkt mit dem C-Atom eines organischen Restes verbunden ist, beispielsweise Formyl, Alkylcarbonyl wie Acetyl oder [(C₁-C₄)Alkyl]-carbonyl, Phenylcarbonyl, Alkylsulfonyl, Alkylsulfinyl und andere Reste von organischen Säuren.

Die "yl-Position" eines Restes bezeichnet das C-Atom mit der freien Bindung. Erfindungsgemäße bzw. erfindungsgemäß verwendete Verbindungen der Formel (I) (und ggf deren Salze) werden kurz auch als "Verbindungen (I)" bezeichnet.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in den allgemeinen Formeln (I) nicht gesondert angegeben sind. Die durch ihre spezifische Raumform definierten möglichen Stereoisomeren, wie Enantiomere, Diastereomere, Z- und E-Isomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Vor allem aus den Gründen der höheren herbiziden Wirkung, besseren Selektivität und/oder besseren Herstellbarkeit sind erfindungsgemäße Verbindungen der genannten Formel (I) oder deren Salze bzw. deren erfindungsgemäße Verwendung von besonderem Interesse, worin einzelne Reste eine der bereits genannten oder im folgenden genannten bevorzugten Bedeutungen haben, oder insbesondere solche, worin eine oder mehrere der bereits genannten oder im Folgenden genannten bevorzugten Bedeutungen kombiniert auftreten.

Unabhängig von den jeweils anderen Resten aus der Gruppe R¹, R², R³, R⁴, R⁵ und (R⁶)ₙ und der den allgemeinen Resten entsprechenden Unterbedeutungen, und vorzugsweise in Kombination mit bevorzugten Bedeutungen von einem oder mehreren dieser Reste, sind erfindungsgemäße Verbindungen bzw. erfindungsgemäße Verwendungen von Verbindungen mit nachfolgend aufgeführten bevorzugten Bedeutungen der betreffenden Reste von besonderem Interesse.

Bevorzugt sind die erfindungsgemäßen Anwendungen von Verbindungen der Formel (I) oder deren Salzen, 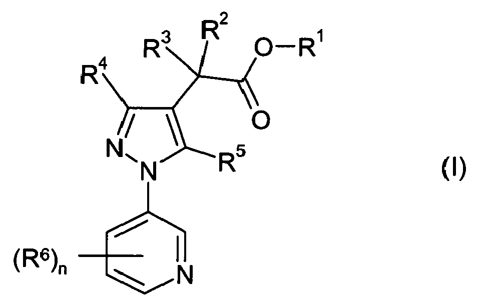 in welcher
- R¹: Wasserstoff, Alkyl, Alkenyl, Alkinyl, Cycloalkyl, Cycloalkenyl, Cycloalkinyl oder Aryl, wobei jeder der letztgenannten 7 Reste unsubstituiert oder substituiert ist und inklusive Substituenten bis zu 30 C-Atome, vorzugsweise bis 24 C-Atome, insbesondere bis 20 C-Atome aufweist, oder einen Heterocyclylrest mit 3 bis 9 Ringatomen, der 1 bis 4 Heteroatome aus der Gruppe N, O und S enthält und der unsubstituiert oder oder substituiert ist und der inklusive Substituenten 1 bis 30 C-Atome, vorzugsweise 1 bis 24 C-Atome, insbesondere 1 bis 20 C-Atome aufweist, bedeutet.
Weiter bevorzugt sind dabei auch Verbindungen (I) oder deren Salze, worin
- R¹: Wasserstoff bedeutet.

Weiter bevorzugt sind dabei auch Verbindungen (I) oder deren Salze, worin
- R¹: H, (C₁-C₁₈)Alkyl, (C₂-C₁₈)Alkenyl, (C₂-C₁₈)Alkinyl, (C₃-C₉)Cycloalkyl, (C₅-C₉)Cycloalkenyl, (C₅-C₉)Cycloalkinyl oder Phenyl, wobei jeder der letztgenannten 7 Reste unsubstituiert oder substituiert ist und inklusive Substituenten bis zu 30 C-Atome, vorzugsweise bis 24 C-Atome, insbesondere bis 20 C-Atome aufweist.

Weiter bevorzugt sind dabei auch Verbindungen (I) oder deren Salze, worin
- R¹: H, (C₁-C₁₈)Alkyl, (C₂-C₁₈)Alkenyl, (C₂-C₁₈)Alkinyl, (C₃-C₉)Cycloalkyl, (C₅-C₉)Cycloalkenyl, (C₅-C₉)Cycloalkinyl oder Phenyl,
wobei jeder der letztgenannten 7 Reste unsubstituiert oder durch einen oder mehrere Reste aus der Gruppe Halogen, Cyano, Thio, Nitro, Hydroxy, (C₁-C₈)Alkyl, (C₁-C₈)Haloalkyl, (C₁-C₄)Alkoxy-(C₁-C₄)alkyl, (C₂-C₈)Alkenyl, (C₂-C₈)Haloalkenyl, (C₂-C₈)Alkinyl, (C₂-C₈)Haloalkinyl, die letztgenannten 7 Reste nur im Falle cyclischer Basisreste, (C₁-C₈)Alkoxy, (C₂-C₈)Alkenyloxy, (C₂-C₈)Alkinyloxy, (C₁-C₈)Haloalkoxy, (C₁-C₄)Alkoxy-(C₁-C₄)alkoxy, (C₁-C₈)Alkylthio, (C₂-C₈)Alkenylthio, (C₂-C₈)Alkinylthio, Reste der Formeln -NR*R**, -CO-NR*R** und -O-CO-NR*R**,
wobei jeder der Reste R* und R** in den letztgenannten 3 Formeln unabhängig voneinander H, (C₁-C₈)Alkyl, (C₂-C₈)Alkenyl,
(C₂-C₈)Alkinyl, Benzyl, substituiertes Benzyl, Phenyl oder substituiertes Phenyl, oder zusammen mit dem N-Atom einen 3- bis
8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere
Reste aus der Gruppe (C₁-C₄)Alkyl und (C₁-C₄)Haloalkyl substituiert
ist, bedeutet,
und [(C₁-C₈)Alkoxy]-carbonyl, [(C₁-C₈)Alkoxy]-thiocarbonyl, [(C₂-C₈)Alkenyloxy]-carbonyl, [(C₂-C₈)Alkinyloxy]-carbonyl, [(C₁-C₈)Alkylthio]-carbonyl, [(C₂-C₈)Alkenylthio]-carbonyl, [(C₂-C₈)Alkinylthio]-carbonyl, (C₁-C₈)Alkanoyl, [(C₂-C₈)Alkenyl]-carbonyl, [(C₂-C₈)Alkinyl]-carbonyl, (C₁-C₄)Alkylimino, (C₁-C₄)Alkoxyimino, [(C₁-C₈)Alkyl]-carbonylamino, [(C₂-C₈)Alkenyl]-carbonylamino, [(C₂-C₈)Alkinyl]-carbonylamino, [(C₁-C₈)Alkoxy]-carbonylamino, [(C₂-C₈)Alkenyloxy]-carbonylamino, [(C₂-C₈)Alkinyloxy]-carbonylamino, [(C₁-C₈)Alkylamino]-carbonylamino, [(C₁-C₆)Alkyl]-carbonyloxy, [(C₂-C₆)Alkenyl]-carbonyloxy, [(C₂-C₆)Alkinyl]-carbonyloxy, [(C₁-C₈)Alkoxy]-carbonyloxy, [(C₂-C₈)Alkenyloxy]-carbonyloxy, [(C₂-C₈)Alkinyloxy]-carbonyloxy, (C₁-C₈)Alkylsulfinyl und (C₁-C₈)Alkylsulfonyl,
wobei jeder der letztgenannten 27 Reste unsubstituiert oder durch einen oder mehrere Reste aus der Gruppe Halogen, NO₂, (C₁-C₄)Alkoxy und gegebenenfalls substituiertes Phenyl substituiert ist,
und Phenyl, Phenyl-(C₁-C₆)alkoxy, Phenyl-[(C₁-C₆)alkoxy]-carbonyl, Phenoxy, Phenoxy-(C₁-C₆)alkoxy, Phenoxy-[(C₁-C₆)alkoxy]-carbonyl, Phenoxycarbonyl, Phenylcarbonyloxy, Phenylcarbonylamino, Phenyl-[(C₁-C₆)alkyl]-carbonylamino, Phenyl-[(C₁-C₆)alkyl]-carbonyloxy, (C₃-C₇)Cycloalkyl und (C₃-C₇)Cycloalkoxy,
wobei jeder der letztgenannten 13 Reste im Ring unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist,
und Reste der Formeln -SiR'₃, -O-SiR'₃, (R')₃Si-(C₁-C₆)alkoxy, -CO-O-NR'₂, -O-N=CR'₂, -N=CR'₂, -O-NR'₂, -CH(OR')₂ und -O-(CH₂)ₘ-CH(OR')₂,
in welchen jeder der Reste R' unabhängig voneinander H,
(C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und m eine ganze Zahl von 0 bis 6 bedeuten,
und Reste der Formel R"O-CHR"'CH(OR")-(C₁-C₆)alkoxy, in welcher jeder der Reste R" unabhängig voneinander H oder (C₁-C₄)Alkyl oder gemeinsam eine (C₁-C₆)Alkylengruppe bedeuten und R'" H oder (C₁-C₄)Alkyl bedeuten,
substituiert ist,
bedeutet.

Weiter bevorzugt sind dabei auch Verbindungen (I) oder deren Salze, worin
- R¹: H, (C₁-C₁₂)Alkyl, (C₂-C₁₂)Alkenyl, (C₂-C₁₂)Alkinyl, (C₃-C₆)Cycloalkyl, (C₅-C₆)Cycloalkenyl, (C₅-C₆)Cycloalkinyl oder Phenyl, wobei jeder der letztgenannten 7 Reste unsubstituiert oder durch einen oder mehrere Reste aus der Gruppe Halogen, Cyano, Thio, Nitro, Hydroxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, die letztgenannten 7 nur im Falle cyclischer Basisreste, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio, Reste der Formel -NR*R**, -CO-NR^{*}R^{**} und -O-CO-NR^{*}R^{**},
wobei jeder der Reste R^{*} und R^{**} in den letztgenannten 3 Formeln unabhängig voneinander H, (C₁-C₄)Alkyl, (C₂-C₄)Alkenyl, (C₂-C₄)Alkinyl, Benzyl, Phenyl, das unsubstituiert oder durch einen oder mehrere Reste aus der Gruppe Halogen, (C₁-C₄)Alkyl, (C₁-C₄)Haloalkyl und (C₁-C₄)Alkoxy substituiert ist, oder zusammen mit dem N-Atom einen Piperidin-, Piperazin-, Pyrrolidin-, Pyrazolidin-, Piperazolidin- oder Morpholinrest, welcher unsubstituiert oder durch einen oder mehrere Reste aus der Gruppe (C₁-C₄)Alkyl und (C₁-C₄)Haloalkyl substituiert ist, bedeutet,
und [(C₁-C₄)Alkoxy]-carbonyl, [(C₁-C₄)Alkyl]-carbonylamino, [(C₁-C₄)Alkoxy]-carbonylamino, [(C₁-C₄Alkylamino]-carbonylamino, [(C₁-C₄)Alkyl]-carbonyloxy, [(C₁-C₄)Alkoxy]-carbonyloxy und (C₁-C₄)Alkylsulfonyl,
wobei jeder der letztgenannten 7 Reste unsubstituiert oder durch einen oder mehrere Reste aus der Gruppe Halogen, NO₂, (C₁-C₄)Alkoxy und Phenyl, das unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist,
und Phenyl, Phenyl-(C₁-C₄)alkoxy, Phenyl-[(C₁-C₄)alkoxy]-carbonyl, Phenoxy, Phenoxy-(C₁-C₄)alkoxy, Phenoxy-[(C₁-C₄)alkoxy]-carbonyl, Phenoxycarbonyl, Phenylcarbonyloxy, Phenylcarbonylamino, Phenyl-[(C₁-C₄)alkyl]-carbonylamino, Phenyl-[(C₁-C₄)alkyl]-carbonyloxy, (C₃-C₆)Cycloalkyl und (C₃-C₆)Cycloalkoxy,
wobei jeder der letztgenannten 13 Reste im Ring unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist,
und Reste der Formeln -CO-O-NR'₂, -O-N=CR'₂, -N=CR'₂, -O-NR'₂, -CH(OR')₂ und -O-(CH₂)ₘ-CH(OR')₂,
in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und m eine ganze Zahl von 0 bis 6 bedeuten,
und Reste der Formel R"O-CHR"'CH(OR")-(C₁-C₆)alkoxy,
in welcher jeder der Reste R" unabhängig voneinander H oder (C₁-C₄)Alkyl oder gemeinsam eine (C₁-C₄)Alkylengruppe bedeuten und R'" H oder (C₁-C₂)Alkyl bedeuten,
substituiert ist,
bedeutet.

Weiter bevorzugt sind dabei auch Verbindungen (I) oder deren Salze, worin
- R¹: H, (C₁-C₆)Alkyl, (C₂-C₆)Alkenyl, (C₂-C₆)Alkinyl oder (C₃-C₆)Cycloalkyl, wobei jeder der letztgenannten 4 Reste unsubstituiert oder durch einen oder mehrere Reste aus der Gruppe Halogen, (C₁-C₆)Alkyl, letzterer nur Substituent im Fall cyclischer Basisreste, (C₁-C₄)Alkoxy, (C₁-C₄)Alkylthio, (C₃-C₆)Cycloalkyl, das unsubstituiert oder durch einen oder mehrere Reste aus der Gruppe Halogen und (C₁-C₄)Alkyl substituiert ist, und Phenyl, das unsubstituiert oder durch einen oder mehrere Reste aus der Gruppe Halogen, (C₁-C₄)Alkyl, (C₁-C₄)Alkoxy und (C₁-C₄)Haloalkyl substituiert ist, substituiert ist,
bedeutet.

Besonders bevorzugt sind dabei auch Verbindungen (I) oder deren Salze, worin
- R¹: H, (C₁-C₄)Alkyl, (C₂-C₄)Alkenyl oder (C₂-C₄)Alkinyl,
wobei jeder der letztgenannten 3 Reste unsubstituiert oder durch einen oder mehrere Reste aus der Gruppe Halogen, (C₁-C₄)Alkoxy, (C₁-C₄)Alkylthio, Cyclopropyl, Cyclobutyl, wobei jeder der letztgenannten beiden Reste unsubstituiert oder durch einen oder mehrere Reste aus der Gruppe Halogen und (C₁-C₄)Alkyl substituiert ist, und Phenyl, das unsubstituiert oder durch einen oder mehrere Reste aus der Gruppe Halogen, (C₁-C₄)Alkyl, (C₁-C₄)Alkoxy und (C₁-C₄)Haloalkyl substituiert ist, substituiert ist,
bedeutet, ganz besonders
- R¹: H, Methyl, Ethyl, n-Propyl, i-Propyl, Benzyl, Cyclopropylmethyl, 1-Cyclopropylethyl, (1-Methyl-cyclopropyl)-methyl, (2,2-Dimethyl-cyclopropyl)-methyl, Allyl, Propargyl, 2-Chlorprop-2-en-1-yl, 3-Phenylprop-2-in-1-yl, 3,3-Dichlorprop-2-en-1-yl, 3,3-Dichlor-2-fluor-prop-2-en-1-yl, But-2-en-1-yl, But-3-en-1-yl, But-2-in-1-yl, But-3-in-1-yl, 4-Chlor-but-2-in-1-yl, 3-Methyl-but-2-en-1-yl, 3-Methyl-but-2-en-1-yl, (E)-Pent-3-en-2-yl oder (Z)-Pent-3-en-2-yl
bedeutet.

Bevorzugt sind auch Verbindungen (I) oder deren Salze, worin
- R¹: einen gesättigten, teilweise ungesättigten oder heteroaromatischen Heterocyclylrest mit 3 bis 9 Ringatomen, vorzugsweise mit 5 oder 6 Ringatomen, der 1 bis 4 Heteroatome, vorzugsweise 1 bis 3 Heteroringatome aus der Gruppe N, O und S enthält und der unsubstituiert oder durch einen oder mehrere Reste aus der Gruppe Halogen, Cyano, Thio, Nitro, Hydroxy, (C₁-C₆)Alkyl, (C₁-C₆)Haloalkyl, (C₁-C₄)Alkoxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₂-C₆)Alkenyloxy, (C₂-C₆)Alkinyloxy, (C₁-C₆)Haloalkoxy, (C₁-C₄)Alkoxy-(C₁-C₄)alkoxy, (C₁-C₆)Alkylthio, (C₂-C₆)Alkenylthio, (C₂-C₆)Alkinylthio, (C₃-C₆)Cycloalkyl, (C₃-C₆)Cycloalkoxy, [(C₁-C₈)Alkoxy]-carbonyl, [(C₁-C₆)Haloalkoxy]-carbonyl und Oxo substituiert ist,
bedeutet.

Bevorzugt sind auch Verbindungen (I) oder deren Salze, worin
- R¹: einen Rest der Formel SiR^{a}R^{b}R^{c}, -NR^{a}R^{b} oder -N=CR^{c}R^{d}, vorzugsweise der Formel -NR^{a}R^{b} oder -N=CR^{c}R^{d},
wobei in den letztgenannten 5 Formeln jeder der Reste R^{a}, R^{b}, R^{c} und R^{d} unabhängig voneinander Wasserstoff, (C₁-C₄)Alkyl, (C₂-C₄)Alkenyl, (C₂-C₄)Alkinyl, Benzyl, substituiertes Benzyl, Phenyl oder substituiertes Phenyl bedeutet oder R^{a} und R^{b} zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl und (C₁-C₄)Haloalkyl substituiert ist, bedeuten oder R^{c} und R^{d} zusammen mit dem C-Atom einen 3- bis 8-gliedrigen carbocyclischen Rest oder heterocyclischen Rest, welcher 1 bis 3 Heteroringatome aus der Gruppe N, O und S enthalten kann, wobei der carbocyclische oder heterocyclischen Rest unsubstituiert oder durch einen oder mehrere Reste aus der Gruppe (C₁-C₄)Alkyl und (C₁-C₄)Haloalkyl substituiert ist, bedeuten,
bedeutet.

Bevorzugt sind auch die Anwendungen von Verbindungen der Formel (I) oder deren Salzen, worin
- R²: Wasserstoff, Halogen oder (C₁-C₄)Alkyl, das unsubstituiert oder durch einen oder mehrere Reste aus der Gruppe Halogen, wie Fluor oder Chlor substituiert ist, vorzugweise Wasserstoff oder (C₁-C₄)Alkyl, insbesondere Wasserstoff, Methyl oder Ethyl, ganz besonders Wasserstoff oder Methyl
bedeutet.

Bevorzugt sind auch die Anwendungen von Verbindungen der Formel (I) oder deren Salzen, worin
- R³: Wasserstoff, Halogen oder (C₁-C₄)Alkyl, das unsubstituiert oder durch einen oder mehrere Reste aus der Gruppe Halogen, wie Fluor oder Chlor, substituiert ist, vorzugweise Wasserstoff oder (C₁-C₄)Alkyl, insbesondere Wasserstoff oder Methyl, ganz besonders Wasserstoff
bedeutet.

Bevorzugt sind auch die Anwendungen von Verbindungen der Formel (I) oder deren Salzen, worin R² und R³ zusammen mit dem C-Atom, an das sie gebunden sind, (C₃-C₆)Cycloalkyl oder (C₅-C₆)Cycloalkenyl, vorzugsweise (C₃-C₆)Cycloalkyl, wobei jeder der letztgenannten 3 Reste unsubstituiert oder durch einen oder mehrere Reste aus der Gruppe Halogen und (C₁-C₄)Alkyl substituiert ist, bedeuten.
Bevorzugt bedeuten dabei R² und R³ zusammen mit dem C-Atom, an das sie gebunden sind, Cyclopropyl, Cyclobutyl, Cyclopentyl oder Cyclohexyl, insbesondere Cyclopropyl, wobei jeder der letztgenannten 4 Reste unsubstituiert oder durch einen oder mehrere Reste aus der Gruppe Halogen und Methyl, vorzugsweise Fluor, Chlor und Methyl substituiert ist.

Bevorzugt sind auch die Anwendungen von Verbindungen der Formel (I) oder deren Salzen, worin
- R⁴: Wasserstoff, Halogen, Cyano, (C₁-C₄)Alkyl, (C₂-C₄)Alkenyl oder (C₂-C₄)Alkinyl, wobei jeder der letztgenannten drei Reste unsubstituiert oder durch einen oder mehrere Reste aus der Gruppe bestehend aus Halogen, wie Fluor und Chlor substituiert ist, oder
(C₃-C₆)Cycloalkyl, das unsubstituiert oder durch einen oder mehrere Reste aus der Gruppe bestehend aus Halogen und (C₁-C₄)Alkyl substituiert ist, oder
Phenyl, das unsubstituiert oder durch einen oder mehrere Reste aus der Gruppe bestehend aus Halogen, Nitro, (C₁-C₄)Alkyl, (C₁-C₄)Haloalkyl, (C₁-C₄)Alkoxy, (C₁-C₄)Haloalkoxy, (C₁-C₄)Alkoxy-(C₁-C₄)alkoxy, (C₁-C₄)Alkylthio, [(C₁-C₄)Alkoxy]-carbonyl und [(C₁-C₄)Haloalkoxy]-carbonyl substituiert ist, oder
(C₁-C₄)Alkanoyl, das unsubstituiert oder durch einen oder mehrere Reste aus der Gruppe bestehend aus Halogen, wie Fluor und Chlor, Cyano, (C₁-C₄)Alkoxy, (C₁-C₄)Haloalkoxy und (C₁-C₂)Alkoxy-(C₁-C₂)alkoxy substituiert ist, oder
[(C₁-C₄)Alkoxy]-carbonyl, das unsubstituiert oder durch einen oder mehrere Reste aus der Gruppe bestehend aus Halogen, wie Fluor und Chlor, substituiert ist, oder
[(C₃-C₆)Cycloalkoxy]-carbonyl, das unsubstituiert oder durch einen oder mehrere Reste aus der Gruppe bestehend aus Halogen und (C₁-C₄)Alkyl substituiert ist,
bedeutet.

Weiter bevorzugt sind auch die Anwendungen von Verbindungen der Formel (I) oder deren Salzen, worin
- R⁴: Wasserstoff, Halogen, wie Fluor oder Chlor, (C₁-C₄)Alkyl, (C₁-C₄)Haloalkyl, Cyclopropyl oder Cyclobutyl, wobei jeder der letztgenannten beiden Reste unsubstituiert oder durch einen oder mehrere Reste aus der Gruppe bestehend aus Halogen, wie Fluor und Chlor, und (C₁-C₄)Alkyl substituiert ist, oder
Phenyl, das unsubstituiert oder durch einen oder mehrere Reste aus der Gruppe bestehend aus Halogen, (C₁-C₄)Alkyl, (C₁-C₄)Haloalkyl, (C₁-C₄)Alkoxy und (C₁-C₄)Alkylthio substituiert ist, oder
(C₁-C₄)Alkanoyl, das unsubstituiert oder durch einen oder mehrere Reste aus der Gruppe bestehend aus Halogen, wie Fluor und Chlor, substituiert ist, oder
[(C₁-C₄)Alkoxy]-carbonyl oder [(C₁-C₄)Haloalkoxy]-carbonyl
bedeutet, vorzugsweise
- R⁴: Wasserstoff, Halogen, wie Fluor oder Chlor, Methyl, Ethyl, n-Propyl, i-Propyl, CH₂Cl, CHCl₂, CCl₃, CH₂F, CHF₂ oder CF₃
bedeutet.

Bevorzugt sind auch die Anwendungen von Verbindungen der Formel (I) oder deren Salzen, worin
- R⁵: Phenyl, das unsubstituiert oder substituiert ist und inklusive Substituenten 6 bis 24 C-Atome, insbesondere 6 bis 20 C-Atome aufweist, oder
einen 5- oder 6-gliedrigen heteroaromatischen Rest mit 1 bis 3 Heteroringatomen aus der Gruppe N, O und S, der unsubstituiert oder substituiert ist und inklusive Substituenten 1 bis 24 C-Atome, insbesondere 1 bis 20 C-Atome aufweist,
bedeutet.

Weiter bevorzugt sind auch die Anwendungen von Verbindungen der Formel (I) oder deren Salzen, worin
- R⁵: Phenyl,
das unsubstituiert oder durch einen oder mehrere Reste aus der Gruppe bestehend aus Halogen, Hydroxy, Amino, Nitro, Carboxy, Cyano, Carbamoyl, (C₁-C₆)Alkyl, (C₁-C₆)Haloalkyl, (C₁-C₄)Alkoxy-(C₁-C₄)alkyl, (C₁-C₄)Alkylthio-(C₁-C₄)alkyl, Mono- und Di-[(C₁-C₄)alkyl]-aminoalkyl, Hydroxy-(C₁-C₄)alkyl, Carboxy-(C₁-C₄)alkyl, Cyano-(C₁-C₄)alkyl, (C₁-C₆)Alkoxy, (C₁-C₄)Alkoxy-(C₁-C₄)alkoxy, (C₁-C₆)Alkylthio, [(C₁-C₆)Alkoxy]-carbonyl, [(C₁-C₆)Haloalkoxy]-carbonyl, (C₁-C₆)Alkanoyl, (C₁-C₆)Haloalkanoyl, Mono- und Di-[(C₁-C₄)alkyl]-aminocarbonyl, Mono- und Di-[(C₁-C₆)acyl]-amino, Mono- und Di-[(C₁-C₄)alkyl]-amino, N-[(C₁-C₆)Acyl]-N-[(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, (C₃-C₆)Cycloalkyl, (C₃-C₆)Cycloalkyloxy, Phenyl und Phenoxy,
wobei jeder der letztgenannten 4 Reste unsubstituiert oder durch einen oder mehrere Reste aus der Gruppe bestehend aus Halogen, (C₁-C₄)Alkyl und (C₁-C₄)Haloalkyl substituiert ist,
substituiert ist und wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen und (C₁-C₆)Alkyl substituiert ist,
oder
- R⁵: einen 5- oder 6-gliedrigen heteroaromatischen Rest mit 1 bis 3 Heteroringatomen aus der Gruppe N, O und S,
der unsubstituiert oder durch einen oder mehrere Reste aus der Gruppe bestehend aus Halogen, Hydroxy, Amino, Nitro, Carboxy, Cyano, Carbamoyl, (C₁-C₆)Alkyl, (C₁-C₆)Haloalkyl, (C₁-C₄)Alkoxy-(C₁-C₄)alkyl, (C₁-C₄)Alkylthio-(C₁-C₄)alkyl, Mono- und Di-[(C₁-C₄)alkyl]-aminoalkyl, Hydroxy-(C₁-C₄)alkyl, Carboxy-(C₁-C₄)alkyl, Cyano-(C₁-C₄)alkyl, (C₁-C₆)Alkoxy, (C₁-C₄)Alkoxy-(C₁-C₄)alkoxy, (C₁-C₆)Alkylthio, [(C₁-C₆)Alkoxy]-carbonyl, [(C₁-C₆)Haloalkoxy]-carbonyl, (C₁-C₆)Alkanoyl, (C₁-C₆)Haloalkanoyl, Mono- und Di-[(C₁-C₄)alkyl]-aminocarbonyl, Mono- und Di-[(C₁-C₆)acyl]-amino, Mono- und Di-[(C₁-C₄)alkyl]-amino, N-[(C₁-C₆)Acyl]-N-(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, (C₃-C₆)Cycloalkyl, (C₃-C₆)Cycloalkyloxy, Phenyl und Phenoxy,
wobei jeder der letztgenannten 4 Reste unsubstituiert oder durch einen oder mehrere Reste aus der Gruppe bestehend aus Halogen, (C₁-C₄)Alkyl und (C₁-C₄)Haloalkyl substituiert ist,
substituiert ist,
bedeutet.

Weiter bevorzugt sind dabei auch die Anwendungen von Verbindungen der Formel (I) oder deren Salzen, worin
- R⁵: Phenyl, das unsubstituiert oder durch einen oder mehrere Reste aus der Gruppe bestehend aus Halogen, Hydroxy, Nitro, Carboxy, Cyano, (C₁-C₄)Alkyl, (C₁-C₄)Haloalkyl, (C₁-C₄)Alkoxy-(C₁-C₄)alkyl, (C₁-C₄)Alkylthio-(C₁-C₄)alkyl, Hydroxy-(C₁-C₄)alkyl, (C₁-C₄)Alkoxy, (C₁-C₄)Alkoxy-(C₁-C₄)alkoxy, (C₁-C₄)Alkylthio, [(C₁-C₄)Alkoxy]-carbonyl, [(C₁-C₆)Haloalkoxy]-carbonyl, (C₁-C₄)Alkylsulfinyl, (C₁-C₄)Haloalkylsulfinyl, (C₁-C₄)Alkylsulfonyl, (C₁-C₄)Haloalkylsulfonyl, (C₃-C₆)Cycloalkyl, (C₃-C₆)Cycloalkyloxy, Phenyl und Phenoxy,
wobei jeder der letztgenannten 4 Reste unsubstituiert oder durch einen oder mehrere Reste aus der Gruppe bestehend aus Halogen, (C₁-C₄)Alkyl und (C₁-C₄)Haloalkyl substituiert ist,
substituiert ist und wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen und (C₁-C₄)Alkyl substituiert ist, oder
- R⁵: einen 5- oder 6-gliedrigen heteroaromatischen Rest mit 1 bis 3 Heteroringatomen aus der Gruppe N, O und S,
der unsubstituiert oder durch einen oder mehrere Reste aus der Gruppe bestehend aus Halogen, Hydroxy, Carboxy, Cyano, (C₁-C₄)Alkyl, (C₁-C₄)Haloalkyl, (C₁-C₄)Alkoxy-(C₁-C₄)alkyl, (C₁-C₄)Alkylthio-(C₁-C₄)alkyl, Hydroxy-(C₁-C₄)alkyl, (C₁-C₄)Alkoxy, (C₁-C₄)Alkoxy-(C₁-C₄)alkoxy, (C₁-C₄)Alkylthio, [(C₁-C₆)Alkoxy]-carbonyl, [(C₁-C₆)Haloalkoxy]-carbonyl, (C₁-C₄)Alkylsulfinyl, (C₁-C₄)Haloalkylsulfinyl, (C₁-C₄)Alkylsulfonyl, (C₁-C₄)Haloalkylsulfonyl und (C₃-C₆)Cycloalkyl, das unsubstituiert oder durch einen oder mehrere Reste aus der Gruppe bestehend aus Halogen, (C₁-C₄)Alkyl und (C₁-C₄)Haloalkyl substituiert ist,
substituiert ist,
bedeutet.

Noch weiter bevorzugt sind dabei auch die Anwendungen von Verbindungen der Formel (I) oder deren Salzen, worin
- R⁵: Phenyl, das unsubstituiert oder durch einen oder mehrere Reste aus der Gruppe bestehend aus Halogen, Cyano, (C₁-C₄)Alkyl, (C₁-C₄)Haloalkyl, (C₁-C₄)Alkoxy und (C₁-C₄)Alkylthio substituiert ist,
oder
- R⁵: einen 5- oder 6-gliedrigen heteroaromatischen Rest mit 1 bis 3 Heteroringatomen aus der Gruppe N, O und S,
der unsubstituiert oder durch einen oder mehrere Reste aus der Gruppe bestehend aus Halogen, Hydroxy, (C₁-C₄)Alkyl, (C₁-C₄)Haloalkyl, (C₁-C₄)Alkoxy und (C₁-C₄)Alkylthio substituiert ist,
bedeutet.

Besonders bevorzugt sind dabei Anwendungen von Verbindungen der Formel (I) oder deren Salzen, worin
- R⁵: Phenyl, das unsubstituiert oder durch einen oder mehrere Reste aus der Gruppe bestehend aus Halogen, wie Fluor, Chlor, Brom und Iod, Cyano, Methyl, Ethyl, n-Propyl, i-Propyl, n-Butyl, sec-Butyl, i-Butyl, t-Butyl, CF₃, CCl₃, Methoxy und Ethoxy substituiert ist, bedeutet, vorzugsweise
- R⁵: Phenyl, 2-Fluorphenyl, 2-Chlorphenyl, 2-Bromphenyl, 2-lodphenyl, 2-Methylphenyl, 2-CF₃-phenyl, 2-Methoxyphenyl, 3-Fluorphenyl, 3-Chlorphenyl, 3-Bromphenyl, 3-lodphenyl, 3-Methylphenyl, 3-CF₃-phenyl, 3-Methoxyphenyl, 4-Fluorphenyl, 4-Chlorphenyl, 4-Bromphenyl, 4-lodphenyl, 4-Methylphenyl, 4-CF₃-phenyl, 4-Methoxyphenyl, 2,3-Dichlorphenyl, 2,3-Dimethylphenyl, 2,4-Dichlorphenyl, 2,4-Dimethylphenyl, 2,5-Dichlorphenyl, 2,5-Dimethylphenyl, 2,6-Dichlorphenyl, 2,6-Dimethylphenyl, 3,4-Dichlorphenyl, 3,4-Dimethylphenyl, 3,5-Dichlorphenyl, 3,5-Dimethylphenyl, 2-Chlor-3-methylphenyl, 2-Chlor-4-methylphenyl, 2-Chlor-5-methylphenyl, 2-Chlor-6-methylphenyl, 3-Chlor-4-methylphenyl, 3-Chlor-5-methylphenyl, 3-Chlor-2-methylphenyl, 4-Chlor-2-methylphenyl, 4-Chlor-3-methylphenyl oder 5-Chlor-2-methylphenyl
bedeutet.

Bevorzugt sind dabei auch Anwendungen von Verbindungen der Formel (1) oder deren Salzen, worin
- R⁵: 2-Pyridyl, 3-Pyridyl, 4-Pyridyl, 2-Thienyl, 3-Thienyl, 2-Furyl, 3-Furyl, 2-Pyrrolyl, 2-Pyrrolyl, 2-Pyrimidinyl, 4-Pyrimidinyl, 5-Pyrimidinyl, 3-Pyridazinyl, 4-Pyridazinyl, 2-Pyrazinyl, 3-Pyrazinyl, 2-Imidazolinyl, 4-Imidazolinyl, 3-Pyrazol, 4-Pyrazol, 1,3,5-Triazin-2-yl, 1,2,4-Triazin-3-yl, 1,2,4-Triazin-5-yl, 2-Thiazolyl, 4-Thiazolyl, 5-Thiazolyl, 3-Isothiazolyl, 4-Isothiazolyl, 5-Isothiazolyl, 2-Oxazolyl, 4-Oxazolyl, 5-Oxazolyl, 3-Isoxazolyl, 4-Isoxazolyl, 5-Isoxazolyl, Pyrazolyl, Thiadiazolyl oder Triazolyl, vorzugsweise 2-Pyridyl, 3-Pyridyl, 4-Pyridyl, 2-Thienyl, 3-Thienyl oder 2-Furyl, wobei jeder der vorstehend genannten heteroaromatischen Reste unsubstituiert oder substituiert ist, vorzugsweise durch die oben bereits bevorzugt genannten Reste substituiert ist, insbesondere durch einen oder mehrere Reste aus der Gruppe bestehend aus Halogen, Hydroxy, (C₁-C₄)Alkyl, (C₁-C₄)Haloalkyl, (C₁-C₄)Alkoxy, (C₁-C₄)Alkylthio substituiert ist,
bedeutet.

Besonders bevorzugt sind dabei auch Anwendungen von Verbindungen der Formel (I) oder deren Salzen, worin
- R⁵: 2-Pyridyl, 3-Fluor-pyrid-2-yl, 3-Chlor-pyrid-2-yl, 3-Brom-pyrid-2-yl, 3-Methyl-pyrid-2-yl, 3-Methoxy-pyrid-2-yl, 3-Trifluormethyl-pyrid-2-yl, 4-Fluor-pyrid-2-yl, 4-Chlor-pyrid-2-yl, 4-Brom-pyrid-2-yl, 4-Methyl-pyrid-2-yl, 4-Methoxy-pyrid-2-yl, 4-Trifluormethyl-pyrid-2-yl, 5-Fluor-pyrid-2-yl, 5-Chlor-pyrid-2-yl, 5-Brom-pyrid-2-yl, 5-Methyl-pyrid-2-yl, 5-Methoxy-pyrid-2-yl, 5-Trifluormethyl-pyrid-2-yl, 6-Fluor-pyrid-2-yl, 6-Chlor-pyrid-2-yl, 6-Brom-pyrid-2-yl, 6-Methyl-pyrid-2-yl, 6-Methoxy-pyrid-2-yl, 6-Trifluormethyl-pyrid-2-yl,
3-Pyridyl, 2-Fluor-pyrid-3-yl, 2-Chlor-pyrid-3-yl, 2-Brom-pyrid-3-yl, 2-Methyl-pyrid-3-yl, 2-Methoxy-pyrid-3-yl, 2-Trifluormethyl-pyrid-3-yl, 4-Fluor-pyrid-3-yl, 4-Chlor-pyrid-3-yl, 4-Brom-pyrid-3-yl, 4-Methyl-pyrid-3-yl, 4-Methoxy-pyrid-3-yl, 4-Trifluormethyl-pyrid-3-yl, 5-Fluor-pyrid-3-yl, 5-Chlor-pyrid-3-yl, 5-Brom-pyrid-3-yl, 5-Methyl-pyrid-3-yl, 5-Methoxy-pyrid-3-yl, 5-Trifluormethyl-pyrid-3-yl, 6-Fluor-pyrid-3-yl, 6-Chlor-pyrid-3-yl, 6-Brom-pyrid-3-yl, 6-Methyl-pyrid-3-yl, 6-Methoxy-pyrid-3-yl, 6-Trifluormethyl-pyrid-3-yl,
4-Pyridyl, 2-Fluor-pyrid-4-yl, 2-Chlor-pyrid-4-yl, 2-Brom-pyrid-4-yl, 2-Methyl-pyrid-4-yl, 2-Methoxy-pyrid-4-yl, 2-Trifluormethyl-pyrid-4-yl, 3-Fluor-pyrid-4-yl, 3-Chlor-pyrid-4-yl, 3-Brom-pyrid-4-yl, 3-Methyl-pyrid-3-yl, 3-Methoxy-pyrid-4-yl, 3-Trifluormethyl-pyrid-4-yl,
2-Thienyl, 3-Fluor-thien-2-yl, 3-Chlor-thien-2-yl, 3-Brom-thien-2-yl, 3-Methyl-thien-2-yl, 3-Methoxy-thien-2-yl, 3-Trifluormethyl-thien-2-yl, 4-Fluor-thien-2-yl, 4-Chlor-thien-2-yl, 4-Brom-thien-2-yl, 4-Methyl-thien-2-yl, 4-Methoxy-thien-2-yl, 4-Trifluormethyl-thien-2-yl, 5-Fluor-thien-2-yl, 5-Chlor-thien-2-yl, 5-Brom-thien-2-yl, 5-Methyl-thien-2-yl, 5-Methoxy-thien-2-yl, 5-Trifluormethyl-thien-2-yl,
3-Thienyl, 2-Fluor-thien-3-yl, 2-Chlor-thien-3-yl, 2-Brom-thien-3-yl, 2-Methyl-thien-3-yl, 2-Methoxy-thien-3-yl, 2-Trifluormethyl-thien-3-yl, 4-Fluor-thien-3-yl, 4-Chlor-thien-3-yl, 4-Brom-thien-3-yl, 4-Methyl-thien-3-yl, 4-Methoxy-thien-3-yl, 4-Trifluormethyl-thien-3-yl, 5-Fluor-thien-3-yl, 5-Chlor-thien-3-yl, 5-Brom-thien-3-yl, 5-Methyl-thien-3-yl, 5-Methoxy-thien-3-yl, 5-Trifluormethyl-thien-3-yl,
2-Furyl, 3-Fluor-fur-2-yl, 3-Chlor-fur-2-yl, 3-Brom-fur-2-yl, 3-Methyl-fur-2-yl, 3-Methoxy-fur-2-yl, 3-Trifluormethyl-fur-2-yl, 4-Fluor-fur-2-yl, 4-Chlor-fur-2-yl, 4-Brom-fur-2-yl, 4-Methyl-fur-2-yl, 4-Methoxy-fur-2-yl, 4-Trifluormethyl-fur-2-yl, 5-Fluor-fur-2-yl, 5-Chlor-fur-2-yl, 5-Brom-fur-2-yl, 5-Methyl-fur-2-yl, 5-Methoxy-fur-2-yl oder 5-Trifluormethyl-fur-2-yl
bedeutet, vorzugsweise
- R⁵: 2-Pyridyl, 5-Fluor-pyrid-2-yl, 5-Chlor-pyrid-2-yl, 5-Brom-pyrid-2-yl, 5-Methyl-pyrid-2-yl, 5-Methoxy-pyrid-2-yl, 5-Trifluormethyl-pyrid-2-yl, 3-Pyridyl, 6-Fluor-pyrid-3-yl, 6-Chlor-pyrid-3-yl, 6-Brom-pyrid-3-yl, 6-Methyl-pyrid-3-yl, 6-Methoxy-pyrid-3-yl oder 6-Trifluormethyl-pyrid-3-yl, 2-Thienyl, 3-Chlor-thien-2-yl, 3-Methyl-thien-2-yl, 4-Chlor-thien-2-yl, 4-Methyl-thien-2-yl, 5-Chlor-thien-2-yl, 5-Brom-thien-2-yl oder 5-Methyl-thien-2-yl
bedeutet.

Bevorzugt sind auch die Anwendungen von Verbindungen der Formel (I) oder deren Salzen, worin
- (R⁶)ₙ: n Substituenten R⁶, wobei R⁶, im Falle dass n = 1 ist, oder jeder der Substituenten R⁶ unabhängig voneinander, im Falle dass n größer als 1 ist, einen Rest Halogen, (C₁-C₄)Alkyl, (C₁-C₄)Haloalkyl, (C₁-C₄)Alkoxy, (C₁-C₄)Alkylthio, (C₁-C₄)Alkylsulfinyl, (C₁-C₄)Haloalkylsulfinyl, (C₁-C₄)Alkylsulfonyl oder (C₁-C₄)Haloalkylsulfonyl bedeutet, und
- n: 0, 1, 2, 3 oder 4, vorzugweise 0, 1 oder 2
bedeuten.

Bevorzugt sind dabei auch die Anwendungen von Verbindungen der Formel (I) oder deren Salzen, worin
- (R⁶)ₙ: n Substituenten R⁶, wobei R⁶, im Falle dass n = 1 ist, oder jeder der Substituenten R⁶ unabhängig voneinander, im Falle dass n größer als 1 ist, einen Rest Halogen, wie Fluor, Chlor, Brom oder Iod, Methyl, Ethyl, CF₃, Methoxy, Ethoxy, Methylthio, Methylsulfinyl, Ethylsulfinyl, Methylsulfonyl oder Ethylsulfonyl bedeutet, und
- n: 0, 1, 2, 3 oder 4, vorzugweise 0, 1 oder 2
bedeuten.

Weiter bevorzugt sind dabei die Anwendungen von Verbindungen der Formel (I) oder deren Salzen, worin
- n: 0 (= die Zahl Null, d. h. keine Substituenten R⁶ vorhanden, d. h. alle freien Bindungen am Ring sind mit Wasserstoff besetzt) oder
- (R⁶)ₙ: 2-Fluor, 2-Chlor, 4-Fluor, 4-Chlor, 5-Fluor, 5-Chlor, 6-Fluor, 6-Chlor, 2-Methyl, 2-Ethyl, 4-Methyl, 4-Ethyl, 5-Methyl, 5-Ethyl, 6-Methyl, 6-Ethyl, 2-CF₃, 2-Methoxy, 2-Ethoxy, 2-Methylsulfonyl, 2-Methylsulfinyl, 2-Methylthio, 4-CF₃, 4-Methoxy, 4-Ethoxy, 4-Methylsulfonyl, 4-Methylsulfinyl, 4-Methylthio, 5-CF₃, 5-Methoxy, 5-Ethoxy oder 5-Methylsulfonyl, 5-Methylthio, 6-CF₃, 6-Methoxy, 6-Ethoxy oder 6-Methylsulfonyl, 6-Methylthio, 2,6-Dimethyl, 2,4-Dimethyl, 2,5-Dimethyl, 4,5-Dimethyl, 4,6-Dimethyl, 5,6-Dimethyl, 2,6-Dichlor, 2,4-Dichlor, 2,5-Dichlor, 4,5-Dichlor, 4,6-Dichlor oder 5,6-Dichlor
bedeutet,
wobei die Bezifferung der Reste sich auf die Position des Restes am 3-Pyridylrest bezieht (Stickstoffatom = Position 1).

Besonders bevorzugt sind dabei die Anwendungen von Verbindungen der Formel (I) oder deren Salzen, worin n = 0 oder worin
- (R⁶)ₙ: 4-Fluor, 4-Chlor, 4-Methyl, 4-CF₃, 4-Methoxy, 4-Methylsulfonyl, 4-Methylsulfinyl oder 4-Methylthio, vorzugsweise 4-Methyl,
bedeutet.

Bevorzugt sind auch die Anwendungen von Verbindungen der Formel (I) oder deren Salzen, worin die Reste R¹, R², R³, R⁴, R⁵, R⁶ und n gemäß zwei oder mehreren der genannten bevorzugten Bedeutungen ausgewählt worden sind.

Besonders bevorzugt sind die Verbindungen der allgemeinen Formel (I) oder deren Salze, worin
- R¹: Wasserstoff bedeutet und R², R³, R⁴, R⁵, R⁶ und n wie in Formel (I) definiert sind [= Verbindungen der Formel (I")] oder
- R¹: Methyl bedeutet und R², R³, R⁴, R⁵, R⁶ und n wie in Formel (I) definiert sind, [= Verbindungen der Formel (I"')] oder
R² und R³ jeweils Wasserstoff bedeuten und R¹, R⁴, R⁵, R⁶ und n wie in Formel (I) definiert sind, jedoch [= Verbindungen der Formel (I"")].

Dabei sind besonders die Verbindungen der Formel (I) und deren Salze bevorzugt,
in welcher ein oder mehrere der Reste R¹ bis R⁶ die in den Beispieltabellen verwendeten Restebedeutungen aufweisen.

Die erfindungsgemäßen Verbindungen der Formel (I) umfassen alle Stereoisomeren, welche aufgrund der Asymmetriezentren oder Doppelbindungen im Molekül, deren Konfiguration in der Formel nicht speziell bezeichnet oder die nicht speziell angegeben sind, auftreten können, und deren Mischung, inklusive der racemischen Verbindungen und der teilweise mit bestimmten Stereoisomeren angereicherten Mischungen.

Die Erfindung umfasst auch alle Tautomeren, wie Keto- und Enol-Tautomeren, und deren Mischungen und Salze, wenn entsprechende funktionelle Gruppen vorhanden sind.

Einige Verbindungen der Formel (I) sind bereits bekannt. So wird in DE-A-2141124 (US-A-4,325,962, US-A-4146721) heteroaromatisch substituierte Pyrazole und deren Anwendung als Antiphlogistika, Analgetika und Antipyretika beschrieben. Speziell beschrieben wird darin die Herstellung von:
a) 3,5-Diphenyl-1-(3-pyridyl)-pyrazol-4-essigsäure (siehe DE-A-2141124, Beispiel 1, siehe US-A-4,146,721, Beispiel 52), welches die Verbindung der Formel (I) mit R¹ = H, R² = H, R³ = H, R⁴ = Phenyl, R⁵ = Phenyl und n = 0 bedeutet,
b) 3,5-Diphenyl-1-(3-pyridyl)-pyrazol-4-essigsäureethylester [siehe DE-A-2141124, Beispiel 1, siehe US-A-4,146,721, Beispiel 52, als Zwischenprodukt zur Herstellung von Verbindung aus Absatz a) angenommen], welches die Verbindung der Formel (I) mit R¹ = Ethyl, R² = H, R³ = H, R⁴ = Phenyl, R⁵ = Phenyl und n = 0 bedeutet,
c) 3-Methyl-5-phenyl-1-(3-pyridyl)-pyrazol-4-essigsäure [siehe DE-A-2141124, Beispiel 3, US-A-4,146,721, Beispiel 47(c)], welches die Verbindung der Formel (I) mit R¹ = H, R² = H, R³ = H, R⁴ = Methyl, R⁵ = Phenyl und n = 0 bedeutet,
d) 3-Methyl-5-phenyl-1-(3-pyridyl)-pyrazol-4-essigsäure-ethylester (siehe DE-A-2141124, Beispiel 3, US-A-4,146,721, Beispiel 47(c)), welches die Verbindung der Formel (I) mit R¹ = Ethyl, R² = H, R³ = H, R⁴ = Methyl, R⁵ = Phenyl und n = 0 bedeutet,

Die erfindungsgemäße Verwendung dieser Verbindungen als Herbizide und Pflanzenwachstumsregulatoren ist jedoch bisher nicht bekannt gewesen.

Gegenstand der Erfindung sind daher auch neue Verbindungen der Formel (I) und deren Salze, wie sie oben für die erfindungsgemäße Verwendung definiert oder bevorzugt definiert worden sind, ausgenommen die Verbindungen 3,5-Diphenyl-1-(3-pyridyl)-pyrazol-4-essigsäure, 3,5-Diphenyl-1-(3-pyridyl)-pyrazol-4-essigsäureethylester, 3-Methyl-5-phenyl-1-(3-pyridyl)-pyrazol-4-essigsäure, 3-Methyl-5-phenyl-1-(3-pyridyl)-pyrazol-4-essigsäureethylester und Salze der genannten Verbindungen.

Gegenstand der Erfindung sind auch Verfahren zur Herstellung der neuen Verbindungen der Formel (I) und deren Salze, dadurch gekennzeichnet, dass man
(a) eine Verbindung der Formel (II), 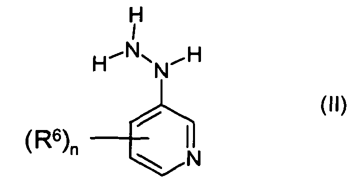 worin (R⁶)ₙ wie in Formel (I) definiert ist,
   mit einer Verbindung der Formel (III), 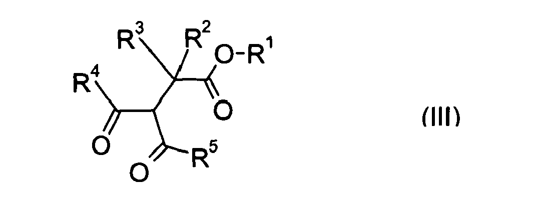 worin R¹, R², R³, R⁴ und R⁵ wie in Formel (I) definiert sind,
   zur Verbindung der Formel (I) oder deren Salz umsetzt oder
(b) im Falle, dass R¹ in Formel (I) von Wasserstoff verschieden ist, eine Verbindung der Formel (I'), 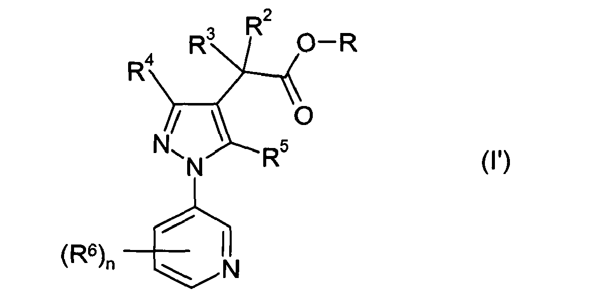 in welcher R¹, R², R³, R⁴ und R⁵ wie in Formel (I) definiert sind und
   - R: einen vom Rest R¹ unterschiedlichen, von Wasserstoff verschiedenen Rest, der aus der Gruppe der Reste, wie sie für R¹ definiert ist, ausgewählt ist, oder ein Anhydrid, Säurehalogenid oder einen aktivierten Ester der Verbindung der Formel (I'), worin R = H bedeutet, bedeutet,
   mit einer Verbindung der Formel (IV),

   R¹ - OH (IV)

   worin R¹ wie in Formel (I) definiert ist,
   zur Verbindung der Formel (I) umsetzt oder
(c) im Falle, dass R¹ in Formel (I) von Wasserstoff verschieden ist, eine Verbindung der Formel (I") 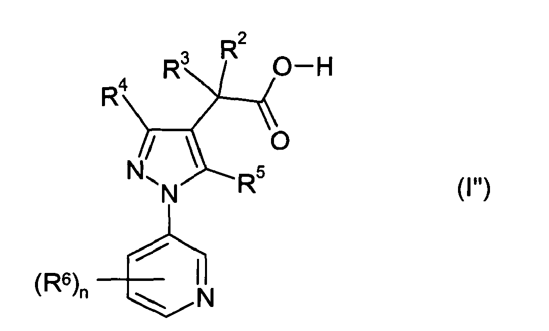 in welcher R², R³, R⁴ und R⁵ wie in Formel (I) definiert sind,
   gegebenenfalls nach Aktivierung der Säuregruppe, mit einer Verbindung der Formel (IV),

   R¹ - OH (IV)

   worin R¹ wie in Formel (I) definiert ist,
   zur Verbindung der Formel (I) verestert oder
(d) im Falle, dass die Verbindung der Formel (I), worin R = H, oder deren Salz hergestellt wird, eine Verbindung der Formel (I') [siehe Definition in Variante (b)] zur Verbindung der Formel (I) oder deren Salz hydrolysiert.

Die Ausgangsverbindungen der Formeln (II), (III) und (IV) sind in der Regel bekannt oder können analog bekannten Verfahren hergestellt werden.

Die Verbindungen der Formel (III) lassen sich beispielsweise herstellen durch Umsetzung einer Dicarbonylverbindung der Formel (V)

R⁴ - CO -CH₂ - CO - R⁵ (V)

mit einer Verbindung der Formel (VI),

R²R³C(Hal)-CO-OR¹ (VI)

wobei R¹, R², R³, R⁴ und R⁵ wie in Formel (III) definiert sind, vorzugsweise R¹ Methyl oder Ethyl bedeutet, und Hal eine Abgangsgruppe, vorzugsweise ein reaktives Halogen wie ein Chlor-atom oder insbesondere ein Brom-atom, bedeutet.

Die Verfahren sind in analoger Weise bereits bekannt. So können erfindungsgemäße Verbindungen der Formel (I) analog bekannten Methoden dargestellt werden, wie sie z. B. in Methoden der organischen Chemie (Houben-Weyl, E. Schaumann, Ed.) Band E8b, Hetarene III, Teil 2, S. 399-710, Georg Thieme Verlag, Stuttgart 1994 und dort zitierter Literatur beschrieben sind, wobei die Synthesen nach Methoden der organischen Chemie (Houben-Weyl, E. Schaumann, Ed.) Band E8b, Hetarene III, Teil 2, S. 420 ff., Georg Thieme Verlag, Stuttgart 1994 und dort zitierte Literatur; Synthesis, 1986, 409; J. Chinese Chem. Soc., 2001, 48, 45 und besonders US 4146721, DE2141124 , DOS 1946370 und Justus Liebigs Ann. Chem. 1973, 1919 von besonderem Interesse sind.

Die beim erfindungsgemäßen Verfahren (a) zur Herstellung von Verbindungen der Formel (I) als Ausgangsstoffe verwendeten substituierten 1,3-Dicarbonylverbindungen der Formel (III) sind vorzugsweise solche, bei denen die Reste R¹, R², R³, R⁴ und R⁵ die bevorzugten Bedeutungen aufweisen, die bereits oben im Zusammenhang mit der Beschreibung der erfindungsgemäßen Verbindungen der Formel (I) als bevorzugt angegeben worden sind. Die beim erfindungsgemäßen Verfahren (a) zur Herstellung von Verbindungen der Formel (I) als Ausgangsstoffe verwendeten substituierten 3-Pyridylhydrazine der Formel (II) hat daher auch vorzugsweise diejenigen Bedeutungen für (R⁶)ₙ, die bereits oben im Zusammenhang mit der Beschreibung der erfindungsgemäßen Verbindungen der Formel (I) vorzugsweise für (R⁶)ₙ, angegeben worden sind.

Die Ausgangsstoffe der allgemeinen Formel (III) können nach allgemein bekannten Verfahren durch Alkylierung entsprechender 1,3-Diketone mit 2-halogenierten Essigsäurederivaten, beispielsweise Bromessigsäure-Derivaten erhalten werden (vgl. z.B. DE-OS 1946370, S. 13). Die hierfür als Ausgangsstoffe verwendeten 1,3-Diketone (V) sind kommerziell erhältlich oder bekannt und/oder können nach bekannten Verfahren hergestellt werden (siehe z.B. US 4146721, DE2141124, DOS1946370 oder J. Am. Chem. Soc., 1948, 70, 4023*;* Justus Liebigs Ann. Chem. 1973, 1919; Justus Liebigs Ann. Chem. 1976, 13; J. Chem. Soc. Perkin Trans. 2, 1993, 6, 1067; Heteroatom Chemistry, 1997, 8, 147).

Hydrazine der Formel (II) oder deren Salze als Ausgangsstoffe sind bekannt und/oder können nach bekannten Verfahren hergestellt werden (vgl. z.B. Methoden der organischen Chemie (Houben-Weyl, D. Klamann, Ed.) Band E16a, Teil 1, S. 421 ff., Georg Thieme Verlag, Stuttgart 1990 und dort zitierte Literatur; J. Am. Chem. Soc., 1954, 76, 596; Monatshefte für Chemie 1988, 119, 333; J. Heterocyclic Chem. 1988, 25, 1055; J. Heterocyclic Chem. 1989, 26, 475; Heterocycles 1994, 37, 379).

Die Umsetzung der Verbindungen der Formel (II) und (III) kann ohne Katalysator oder in Gegenwart von Katalysatoren, bespielsweise von einer Säure als Katalysator, erfolgen, vorzugsweise in einem organischen Lösungsmittel, wie z.B. Tetrahydrofuran (THF), Dioxan, Acetonitril, Dimethylformamid (DMF), Methanol und Ethanol, bei Temperaturen zwischen 20 °C bis zum Siedepunkt des Lösungsmittels, vorzugsweise bei 50 °C bis 150 °C. Falls Säureadditionssalze der Formel (II) verwendet werden, setzt man diese in der Regel mit Hilfe einer Base in situ frei. Als Basen bzw. basische Katalysatoren eignen sich Alkalihydroxide, Alkalihydride, Alkalicarbonate, Alkalihydrogencarbonate, Alkalialkoholate, Erdalkalihydroxide, Erdalkalihydride, Erdalkalicarbonate oder organische Basen wie Triethylamin, Diisopropyl-ethylamin oder 1,8-Diazabicyclo[5.4.0]undec-7-en (DBU).

Die Umsetzung der Verbindungen der Formel (I') und (IV) kann nach Standardmethoden der Umesterung oder der Veresterung über aktivierte Carbonsäuren erfolgen.
Die Umsetzung der Verbindungen der Formel (I") und (IV) kann nach Standardmethoden der Versterungen oder gegebenenfalls über aktivierte Carbonsäuren erfolgen.
Die Herstellung von Verbindungen der Formel (I") aus Verbindungen (I') kann nach Standardmethoden der Verseifung erfolgen.

Für die Herstellung von Enantiomeren der Verbindungen (I) kommen auch übliche Racemattrennungsmethoden in Frage (vgl. Handbücher der Stereochemie), z. B. im Anschluss an Verfahren zur Trennung von Gemischen in Diastereomere, z. B. physikalische Verfahren wie Kristallisation, Chromatographieverfahren, vor allem Säulenchromatographie und Hochdruckflüssigchromatographie, Destillation, gegebenenfalls unter reduziertem Druck , Extraktion und andere Verfahren, können verbleibende Gemische von Enantiomeren in der Regel durch chromatographische Trennung an chiralen Festphasen getrennt werden. Für präparative Mengen oder im industriellen Maßstab kommen Verfahren wie die Kristallisation diastereomerer Salze, die aus den Verbindungen (I) mit optisch aktiven Säuren und gegebenenfalls bei vorhandenen sauren Gruppen mit optisch aktiven Basen erhalten werden können, in Frage.

Zur Racemattrennung durch Kristallisation diastereomerer Salze kommen als optisch aktive Säure z. B. Camphersulfonsäure, Camphersäure, Bromcamphersulfonsäure, Chinasäure, Weinsäure, Dibenzoylweinsäure und andere analoge Säure in Betracht; als optisch aktive Basen kommen z. B. Chinin, Cinchonin, Chinidin, Brucin, 1-Phenylethylamin und andere analoge Basen in Frage.
Die Kristallisationen werden dann meist in wässrigen oder wässrig-organischen Lösungsmittel durchgeführt, wobei das Diastereomer mit der geringeren Löslichkeit gegebenenfalls nach Animpfen zunächst ausfällt. Das eine Enantiomer der Verbindung der Formel (I) wird danach aus dem ausgefällten Salz oder das andere aus dem Kristallisat durch Ansäuern bzw. mit Base freigesetzt.

Zur Herstellung der Säureadditionssalze der Verbindungen der Formel (I) kommen folgende Säuren in Frage: Halogenwasserstoffsäuren wie Chlorwasserstoffsäure oder Bromwasserstoffsäure, weiterhin Phosphorsäure, Salpetersäure, Schwefelsäure, mono- oder bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphtalindisulfonsäure. Die Säureadditionsverbindungen der Formel (I) können in einfacher Weise nach den üblichen Salzbildungsmethoden, z.B. durch Lösen einer Verbindung der Formel (I) in einem geeigneten organischen Lösungsmittel wie z.B. Methanol, Aceton, Methylenchlorid oder Benzol und Hinzufügen der Säure bei Temperaturen von 0 bis 100 °C erhalten werden und in bekannter Weise, z.B. durch Abfiltrieren, isoliert und gegebenfalls durch Waschen mit einem inerten organischen Lösemittel gereinigt werden.

Die Basenadditionssalze der Verbindungen der Formel (I) werden vorzugsweise in inerten polaren Lösungsmitteln wie z.B. Wasser, Methanol oder Aceton bei Temperaturen von 0 bis 100 °C hergestellt. Geeignete Basen zur Herstellung der erfindungsgemäßen Salze sind beispielsweise Alkalicarbonate, wie Kaliumcarbonat, Alkali- und Erdalkalihydroxide, z.B. NaOH oder KOH, Alkali- und Erdalkalihydride, z.B. NaH, Alkali- und Erdalalkoholate, z.B. Natriummethanolat oder Kalium-tert.-butylat, oder Ammoniak, Ethanolamin oder quartäres Ammoniumhydroxid der Formel [NRR'R"R''']⁺ OH-.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Eine Kollektion aus Verbindungen der Formel (I), die nach den obengenannten Verfahren synthetisiert werden können, können zusätzlich in parallelisierter Weise hergestellt werden, wobei dies in manueller, teilweise automatisierter oder vollständig automatisierter Weise geschehen kann. Dabei ist es möglich, sowohl die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch S. H. DeWitt in "Annual Reports in Combinatorial Chemistry and Molecular Diversity: Automated Synthesis", Band 1, Verlag Escom, 1997, Seite 69 bis 77 beschrieben wird.

Zur parallelisierten Reaktionsdurchführung und Aufarbeitung können eine Reihe von im Handel erhältlichen Geräten verwendet werden wie sie beispielsweise von den Firmen Stem Corporation, Woodrolfe Road, Tollesbury, Essex, CM9 8SE, England oder H + P Labortechnik GmbH, Bruckmannring 28, 85764 Oberschleißheim, Deutschland angeboten werden. Für die parallelisierte Aufreinigung von Verbindungen (I) oder von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA. Die aufgeführten Apparaturen ermöglichen eine modulare Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise von Roboter bedient werden. Derartige Automationssysteme können zum Beispiel von der Firma Zymark Corporation, Zymark Center, Hopkinton, MA 01748, USA bezogen werden.

Neben den beschriebenen Methoden kann die Herstellung von Verbindungen der Formel (I) vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepaßten Synthese an ein Syntheseharz gebunden. Festphasen unterstützte Synthesemethoden sind in der Fachliteratur hinreichend beschrieben, z. B.: Barry A. Bunin in "The Combinatorial Index", Verlag Academic Press, 1998.
Die Verwendung von festphasen-unterstützten Synthesemethoden erlaubt eine Reihe von literaturbekannten Protokollen, die wiederum manuell oder automatisiert ausgeführt werden können. Zum Beispiel kann die "Teebeutelmethode" (Houghten, US 4,631,211; Houghten et al., Proc. Natl. Acad. Sci., 1985, 82, 5131 - 5135) mit Produkten der Firma IRORI, 11149 North Torrey Pines Road, La Jolla, CA 92037, USA teilweise automatisiert werden. Die Automatisierung von Festphasen unterstützter Parallelsynthese gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland.

Die Herstellung gemäß der hier beschriebenen Verfahren liefert Verbindungen der Formel (I) in Form von Substanzkollektionen oder -bibliotheken. Gegenstand der vorliegenden Erfindung sind daher auch Bibliotheken der Verbindungen der Formel (I), die mindestens zwei Verbindungen der Formel (I) enthalten, und deren Vorprodukte.

Die erfindungsgemäßen Verbindungen der Formel (I) und deren Salze, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im Einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.
Auf der Seite der monokotylen Unkrautarten werden z.B. Agrostis, Alopecurus, Apera, Avena, Brachicaria, Bromus, Dactyloctenium, Digitaria, Echinochloa, Eleocharis, Eleusine, Festuca, Fimbristylis, Ischaemum, Lolium, Monochoria, Panicum, Paspalum, Phalaris, Phleum, Poa, Sagittaria, Scirpus, Setaria, Sphenoclea sowie Cyperusarten aus der annuellen Gruppe und auf seiten der perennierenden Spezies Agropyron, Cynodon, lmperata sowie Sorghum und auch ausdauernde Cyperusarten gut erfaßt.

Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.
Herbizide Wirkung wird auch erreicht bei dikotylen Schadpflanzen wie Ambrosia, Anthemis, Carduus, Centaurea, Chenopodium, Cirsium, Convolvulus, Datura, Emex, Galeopsis, Galinsoga, Kochia, Lepidium, Lindernia, Papaver, Portlaca, Polygonum, Ranunculus, Rorippa, Rotala, Seneceio, Sesbania, Solanum, Sonchus, Taraxacum, Trifolium, Urtica und Xanthium.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.
Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Soja, insbesondere Plantagenkulturen wie Ölpalme, Olive, Kokosnuss, Gummibaum, Zitrus, Ananas, Apfel, Birne, Kirsche, Baumwolle, Kaffee, Kakau, Wein und andere vergleichbare Obst und Plantagenkulturen nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen inklusive Zierpflanzungen. Auch eignen sich die Wirkstoffe, gegebenenfalls in Kombination mit anderen Wirkstoffen für die Anwendung im Nichtkulturland, wie auf Wegen, Plätzen, Beeten, Rasenflächen, Bahndämmen, Industrieanlagen zur Bekämpfung von unerwünschtem Pflanzenwuchs.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen oder zur Wachstumsregulation in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Emteguts bekannt.

Bevorzugt ist die Anwendung der erfindungsgemäßen Verbindungen der Formel (I) oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.
Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Herbizide aus der Gruppe der Imidazolinone, Sulfonylharnstoffe, Glufosinate-ammonium oder Glyphosateisopropylammonium und analoge Wirkstoffe resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen (I) als Herbizide zur Bekämpfung von Schadpflanzen oder zur Pflanzenwachstumsregulation in transgenen Kulturpflanzen.

Die erfindungsgemäße Verwendung zur Bekämpfung von Schadpflanzen oder zur Wachstumsregulierung von Pflanzen schließt auch den Fall ein, bei dem der Wirkstoff der Formel (I) oder dessen Salz erst nach der Ausbringung auf der Pflanze, in der Pflanze oder im Boden aus einer Vorläufersubstanz ("Prodrug") gebildet wird.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage:
Spritzpulver (WP), wasserlösliche Pulver (SP), wasserlösliche Konzentrate, emulgierbare Konzentrate (EC), Emulsionen (EW), wie Öl-in-Wasser- und Wasserin-Öl-Emulsionen, versprühbare Lösungen, Suspensionskonzentrate (SC), Dispersionen auf Öl- oder Wasserbasis, ölmischbare Lösungen,
Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesellschaft, Stuttgart 1976; Winnacker-Küchler, "Chemische Technologie", Band 7, C. Hanser Verlag München, 4. Aufl. 1986.
Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.
Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen
Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt. Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-% .

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel. Beispiele für Formulierungshilfsmittel sind unter anderem in "Chemistry and Technology of Agrochemical Formulations", ed. D. A. Knowles, Kluwer Academic Publishers (1998) beschrieben.

Die Verbindungen der Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen. Die Kombinationsformulierungen können dabei auf Basis der obengenannten Formulierungen hergestellt werden,
wobei die physikalischen Eigenschaften und Stabilitäten der zu kombinierenden Wirkstoffe zu berücksichtigen sind.
Als Kombinationspartner für die erfindungsgemäßen Wirkstoffe in Mischungsformulierungen oder im Tank-Mix sind beispielsweise bekannte Wirkstoffe, die auf einer Inhibition von beispielsweise Acetolactat-Synthase, Acetyl-Coenzyl-A-Carboxylase, PS I, PS II, HPPDO, Phytoene-Desaturase, Protoporphyrinogen-Oxidase, Glutamine-Synthetase, Cellulosebiosynthese, 5-Enolpyruvylshikimat-3-phosphat-Synthetase beruhen, einsetzbar. Solche Verbindungen und auch andere einsetzbare Verbindungen mit teilweise unbekanntem oder anderem Wirkungsmechanismus sind z.B. in Weed Research 26, 441-445 (1986), oder "The Pesticide Manual", 14. Auflage 2006/2007, herausgegeben vom British Crop Protection Council, (im Folgenden auch kurz "PM"), und dort zitierter Literatur beschrieben. Als literaturbekannte Herbizide, Pflanzenwachstumsregulatoren und Herbizid-Safenern, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet): acetochlor; acibenzolar-S-methyl, acifluorfen(-sodium); aclonifen; AD-67, AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim(-sodium); ametryn; amicarbazone, amidochlor, amidosulfuron; aminopyralid, amitrole; AMS, d.h. Ammoniumsulfamat; ancymidol, anilofos; asulam; atrazine; aviglycine, azafenidin, azimsulfuron (DPX-A8947); aziprotryn; barban; BAS 516 H, d.h. 5-Fluor-2-phenyl-4H-3,1-benzoxazin-4-on; beflubutamid, benazolin(-ethyl); bencarbzone, benfluralin; benfuresate; benoxacor, bensulfuron(-methyl); bensulide; bentazone; benzfendizone, benzobicyclon, benzofenap; benzofluor; benzoylprop(-ethyl); benzthiazuron; bialaphos; bifenox; bilanafos (bialaphos), bispyribac(-sodium), borax, bromacil; bromobutide; bromofenoxim; bromoxynil; bromuron; buminafos; busoxinone; butachlor; butafenacil, butamifos; butenachlor; buthidazole; butralin; butroxydim, butylate; cafenstrole (CH-900); carbetamide; carfentrazone(-ethyl) (ICI-A0051); caloxydim, CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlorflurenol(-methyl), chlormequat (-chloride), chlormesulon (ICI-A0051); chlorbromuron; chlorbufam; chlorfenac; chloridazon; chlorimuron(-ethyl); chlornitrofen; chlorotoluron; chloroxuron; chlorpropham; chlorsulfuron; chlorthal-dimethyl; chlorthiamid; chlortoluron, cinidon(-methyl und -ethyl), cinmethylin; cinosulfuron; clefoxydim, clethodim; clodinafop und dessen Esterderivate (z.B. clodinafop-propargyl); clofencet, clomazone; clomeprop; cloprop, cloproxydim; clopyralid; clopyrasulfuron(-methyl), cloquintocet(-mexyl), cloransulam(-methyl), cumyluron (JC 940); cyanamide, cyanazine; cyclanilide, cycloate; cyclosulfamuron (AC 104); cycloxydim; cycluron; cyhalofop und dessen Esterderivate (z.B. Butylester, DEH-112); cyperquat; cyprazine; cyprazole; cyprosulfamide, daimuron; 2,4-D, 2,4-DB; 2,4-DB, dalapon; daminozide, dazomet, n-decanol, desmedipham; desmetryn; di-allate; dicamba; dichlobenil; dichlormid, dichlorprop(-P) (-salze); diclofop und dessen Ester wie diclofop-methyl; diclosulam, diethatyl(-ethyl); difenoxuron; difenzoquat(-metilsulfate); diflufenican; diflufenzopyr, dimefuron; dimepiperate, dimethachlor; dimethametryn; dimethazone, dimethenamid (SAN-582H); dimethenamid-P, dimethylarsinic acid, dimexyflam, dimethipin; dimetrasulfuron, dinitramine; dinoseb; dinoterb; diphenamid; dipropetryn; diquat (-salze); dithiopyr; diuron; DNOC; eglinazine-ethyl; EL 77, d.h.
5-Cyano-1-(1,1-dimethylethyl)-N-methyl-1H-pyrazole-4-carboxamid; endothal; epoprodan, EPTC; esprocarb; ethalfluralin; ethametsulfuron-methyl; ethephon, ethidimuron; ethiozin; ethofumesate; ethoxyfen und dessen Ester (z.B. Ethylester, HN-252); ethoxysulfuron, etobenzanid (HW 52); F5231, d.h. N-[2-Chlor-4-fluor-5-[4-(3-fluorpropyl)-4,5-dihydro-5-oxo-1H-tetrazol-1-yl]-phenyl]-ethansulfonamid; fenchlorazole(-ethyl), fenclorim, fenoprop; fenoxan, fenoxaprop und fenoxaprop-P sowie deren Ester, z.B. fenoxaprop-P-ethyl und fenoxaprop-ethyl; fenoxydim; fentrazamide, fenuron; ferrous sulfate, flamprop(-methyl oder -isopropyl oder -isopropyl-L); flazasulfuron; floazulate, florasulam, fluazifop und fluazifop-P und deren Ester, z.B. fluazifop-butyl und fluazifop-P-butyl; fluazolate, flucarbazone(-sodium), flucetosulfuron, fluchloralin; flufenacet, flufenpyr(-ethyl), flumetralin, flumetsulam; flumeturon; flumidorac(-pentyl), flumioxazin (S-482); flumipropyn; fluometuron, fluorochloridone, fluorodifen; fluoroglycofen(-ethyl); flupoxam (KNW-739); flupropacil (UBIC-4243); flupropanoate, flupyrsulfuron(-methyl)(-sodium), flurazole, flurenol(-butyl), fluridone; flurochloridone; fluroxypyr(-meptyl); flurprimidol, flurtamone; fluthiacet(-methyl), fluthiamide, fluxofenim, fomesafen; foramsulfuron, forchlorfenuron, fosamine; furilazole, furyloxyfen; gibberellic acid, glufosinate(-ammonium); glyphosate(-isopropylammonium); halosafen; halosulfuron(-methyl) und dessen Ester (z.B. Methylester, NC-319); haloxyfop und dessen Ester; haloxyfop-P (= R-haloxyfop) und dessen Ester; HC-252, hexazinone; imazamethabenz(-methyl); imazamox, imazapic, imazapyr; imazaquin und Salze wie das Ammoniumsalz; imazamethapyr, imazapic, imazethamethapyr; imazethapyr; imazosulfuron; inabenfide, indanofan, indol-3-ylacetic acid, 4-indol-3-ylbutyric acid, iodosulfuron-methyl(-sodium), ioxynil; isocarbamid; isopropalin; isoproturon; isouron; isoxaben; isoxachlortole, isoxadifen(-ethyl), isoxaflutole, isoxapyrifop; karbutilate; lactofen; lenacil; linuron; maleic hydrazide, MCPA; MCPB; mecoprop(-P), mefenacet; mefenpyr-diethyl, mefluidid; mepiquat (chloride), mesotrione, mesosulfuron(-methyl), mesotrione, metam, metamifop, metamitron; metazachlor; methabenzthiazuron; methazole; methoxyphenone; methylarsonic acid, 1-methyl-cyclopropene, methyldymron; methyl isothiocyanate, metobenzuron, metobromuron; (alpha-)metolachlor; metosulam (XRD 511); metoxuron; metribuzin; metsulfuron-methyl; MH; MK-616, molinate; monalide; monocarbamide dihydrogensulfate; monolinuron; monuron; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; 2-(1-naphthyl)acetamide, 1-naphthylacetic acid, 2-naphthyloxyacetic acid, naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrophenolate mixture, nitrofluorfen; nonanoic acid, norflurazon; orbencarb; orthasulfamuron, oryzalin; oxabetrinil, oxadiargyl (RP-020630); oxadiazon; oxasulfuron, oxaziclomefone, oxyfluorfen; paclobutrazol, paraquat(dichloride); pebulate; pelargonic acid, pendimethalin; penoxulam, pentachlorophenol, pentanochlor, pentoxazone, perfluidone; pethoxamid, phenisopham; phenmedipham; picloram; picolinafen, pinoxaden, piperophos; piributicarb; pirifenop-butyl; pretilachlor; primisulfuron(-methyl); probenazole, procarbazone-(sodium), procyazine; prodiamine; profluralin; profoxydim, prohexadione(-calcium), prohydrojasmon, proglinazine(-ethyl); prometon; prometryn; propachlor; propanil; propaquizafop und dessen Ester; propazine; propham; propisochlor; propoxycarbazone(-sodium), n-propyl dihydrojasmonate, propyzamide; prosulfalin; prosulfocarb; prosulfuron (CGA-152005); prynachlor; pyraclonil, pyraflufen(-ethyl), pyrasulfotole, pyrazolynate; pyrazon; pyrazosulfuron(-ethyl); pyrazoxyfen; pyribenzoxim, pyributicarb, pyridafol, pyridate; pyriftalid, pyriminobac(-methyl), pyrimisulfan, pyrithiobac(-sodium) (KIH-2031); pyroxasulfone, pyroxofop und dessen Ester (z.B. Propargylester); pyroxsulam, quinclorac; quinmerac; quinoclamine, quinofop und dessen Esterderivate, quizalofop und quizalofop-P und deren Esterderivate z.B. quizalofop(-ethyl); quizalofop(-P-tefuryl und -ethyl); renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; sintofen, SN 106279, d.h. 2-[[7-[2-Chlor-4-(trifluormethyl)-phenoxy]-2-naphthalenyl]-oxy]-propansäure und -methylester; sulcotrione, sulfentrazone (FMC-97285, F-6285); sulfazuron; sulfometuron(-methyl); sulfosate (ICI-A0224); sulfosulfuron, 2,3,6-TBA, TCA; tebutam (GCP-5544); tebuthiuron; tecnazene, tefurlyltrione, tembotrione, tepraloxydim, terbacil; terbucarb; terbuchlor; terbumeton; terbuthylazine; terbutryn; TFH 450, d.h. N,N-Diethyl-3-[(2-ethyl-6-methylphenyl)-sulfonyl]-1 H-1,2,4-triazol-1-carboxamid; thenylchlor (NSK-850); thiafluamide, thiazafluron; thiazopyr (Mon-13200); thidiazimin (SN-24085); thidiazuron, thiencarbazone, thifensulfuron(-methyl); thiobencarb; TI-35, tiocarbazil; topramezone, tralkoxydim; tri-allate; triasulfuron; triaziflam, triazofenamide; tribenuron(-methyl); triclopyr; tridiphane; trietazine; trifloxysulfuron(-sodium), trifluralin; triflusulfuron und Ester (z.B. Methylester, DPX-66037); trimeturon; trinexapac(-ethyl), tritosulfuron, tsitodef; uniconazole, vernolate; WL 110547, d.h. 5-Phenoxy-1-[3-(trifluormethyl)-phenyl]-1 H-tetrazol; BAY MKH 6561, UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023

Von besonderem Interesse ist die selektive Bekämpfung von Schadpflanzen in Kulturen von Nutz- und Zierpflanzen. Obgleich die erfindungsgemäßen Verbindungen (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen (I) von besonderem Interesse, welche die Verbindungen (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z. B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide. Folgende Gruppen von Verbindungen kommen beispielsweise als Safener für die Verbindungen (I) und deren Kombinationen mit weiteren Pestiziden in Frage:
a) Verbindungen vom Typ der Dichlorphenylpyrazolin-3-carbonsäure, vorzugsweise Verbindungen wie 1-(2,4-Dichlorphenyl)-5-(ethoxycarbonyl)-5-methyl-2-pyrazolin-3-carbonsäureethylester (S1-1) ("Mefenpyr-diethyl", PM), und verwandte Verbindungen, wie sie in der WO 91/07874 beschrieben sind,
b) Derivate der Dichlorphenylpyrazolcarbonsäure, vorzugsweise Verbindungen wie 1-(2,4-Dichlorphenyl)-5-methyl-pyrazol-3-carbonsäureethylester (S1 -2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1 -4), 1-(2,4-Dichlorphenyl)-5-phenyl-pyrazol-3-carbonsäureethylester (S1-5) und verwandte Verbindungen, wie sie in EP-A-333 131 und EP-A-269 806 beschrieben sind.
c) Verbindungen vom Typ der Triazolcarbonsäuren, vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-6), und verwandte Verbindungen EP-A-174 562 und EP-A-346 620);
d) Verbindungen vom Typ der 5-Benzyl- oder 5-Phenyl-2-isoxazolin-3- carbonsäure, oder der 5,5-Diphenyl-2-isoxazolin-3-carbonsäure vorzugsweise Verbindungen wie 5-(2,4-Dichlorbenzyl)-2-isoxazolin-3-carbonsäureethylester (S1-7) oder 5-Phenyl-2-isoxazolin-3-carbonsäureethylester (S1-8) und verwandte Verbindungen, wie sie in WO 91/08202 beschrieben sind, bzw. der 5,5-Diphenyl-2-isoxazolin-carbonsäureethylester (S 1-9) ("Isoxadifen-ethyl") oder - n-propylester (S1-10) oder der 5-(4-Fluorphenyl)-5-phenyl-2-isoxazolin-3-carbonsäureethylester (S1-11), wie sie in der deutschen Patentanmeldung (WO-A-95/07897) beschrieben sind.
e) Verbindungen vom Typ der 8-Chinolinoxyessigsäure (S2), vorzugsweise (5-Chlor-8-chinolinoxy)-essigsäure-(1-methyl-hex-1 -yl)-ester (Common name "Cloquintocet-mexyl" (S2-1) (siehe PM) (5-Chlor-8-chinolinoxy)-essigsäure-(1,3-dimethyl-but-1-yl)-ester (S2-2), (5-Chlor-8-chinolinoxy)-essigsäure-4-allyl-oxy-butylester (S2-3), (5-Chlor-8-chinolinoxy)-essigsäure-1-allyloxy-prop-2-ylester (S2-4), (5-Chlor-8-chinolinoxy)-essigsäureethylester (S2-5), (5-Chlor-8-chinolinoxy)-essigsäuremethylester (S2-6), (5-Chlor-8-chinolinoxy)-essigsäureallylester (S2-7), (5-Chlor-8-chinolinoxy)-essigsäure-2-(2-propyliden-iminoxy)-1-ethylester (S2-8), (5-Chlor-8-chinolinoxy)-essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind.
f) Verbindungen vom Typ der (5-Chlor-8-chinolinoxy)-malonsäure, vorzugsweise Verbindungen wie (5-Chlor-8-chinolinoxy)-malonsäurediethylester, (5-Chlor-8-chinolinoxy)-malonsäurediallylester, (5-Chlor-8-chinolinoxy)-malonsäure-methyl-ethylester und verwandte Verbindungen, wie sie in EP-A-0 582 198 beschrieben sind.
g) Wirkstoffe vom Typ der Phenoxyessig- bzw. -propionsäurederivate bzw. der aromatischen Carbonsäuren, wie z.B. 2,4-Dichlorphenoxyessigsäure(ester) (2,4-D), 4-Chlor-2-methyl-phenoxy-propionester (Mecoprop), MCPA oder 3,6-Dichlor-2-methoxy-benzoesäure(ester) (Dicamba).
h) Wirkstoffe vom Typ der Pyrimidine, die als bodenwirksame Safener in Reis angewendet werden, wie z. B. "Fenclorim" (PM) (= 4,6-Dichlor-2-phenylpyrimidin), das als Safener für Pretilachlor in gesätem Reis bekannt ist,
i) Wirkstoffe vom Typ der Dichloracetamide, die häufig als Vorauflaufsafener (bodenwirksame Safener) angewendet werden, wie z. B. "Dichlormid" (PM) (= N,N-Diallyl-2,2-dichloracetamid),
   "R-29148" (= 3-Dichloracetyl-2,2,5-trimethyl-1,3-oxazolidin von der Firma Stauffer),
   "Benoxacor" (PM) (= 4-Dichloracetyl-3,4-dihydro-3-methyl-2H-1,4-benzoxazin).
   "PPG-1292" (= N-Allyl-N-[(1,3-dioxolan-2-yl)-methyl]-dichloracetamid von der Firma PPG Industries),
   "DK-24" (= N-Allyl-N-[(allylaminocarbonyl)-methyl]-dichloracetamid von der Firma Sagro-Chem),
   "AD-67" oder "MON 4660" (= 3-Dichloracetyl-1-oxa-3-aza-spiro[4,5]decan von der Firma Nitrokemia bzw. Monsanto),
   "Diclonon" oder "BAS145138" oder "LAB145138" (= (= 3-Dichloracetyl-2,5,5-trimethyl-1,3-diazabicyclo[4.3.0]nonan von der Firma BASF) und
   "Furilazol" oder "MON 13900" (siehe PM) (= (RS)-3-Dichloracetyl-5-(2-furyl)-2,2-dimethyloxazolidin)
j) Wirkstoffe vom Typ der Dichloracetonderivate, wie z. B. "MG 191" (CAS-Reg. Nr. 96420-72-3) (= 2-Dichlormethyl-2-methyl-1,3-dioxolan von der Firma Nitrokemia), das als Safener für Mais bekannt ist,
k) Wirkstoffe vom Typ der Oxyimino-Verbindungen, die als Saatbeizmittel bekannt sind, wie z. B.
   "Oxabetrinil" (PM) (= (Z)-1,3-Dioxolan-2-ylmethoxyimino(phenyl)acetonitril), das als Saatbeiz-Safener für Hirse gegen Schäden von Metolachlor bekannt ist,
   "Fluxofenim" (PM) (= 1-(4-Chlorphenyl)-2,2,2-trifluor-1-ethanon-O-(1,3-dioxolan-2-ylmethyl)-oxim, das als Saatbeiz-Safener für Hirse gegen Schäden von Metolachlor bekannt ist, und
   "Cyometrinil" oder "-CGA-43089" (PM) (= (Z)-Cyanomethoxyimino(phenyl)acetonitril), das als Saatbeiz-Safener für Hirse gegen Schäden von Metolachlor bekannt ist,
l) Wirkstoffe vom Typ der Thiazolcarbonsäureester, die als Saatbeizmittel bekannt sind, wie z. B.
   "Flurazol" (PM) (= 2-Chlor-4-trifluormethyl-1,3-thiazol-5-carbonsäurebenzylester), das als Saatbeiz-Safener für Hirse gegen Schäden von Alachlor und Metolachlor bekannt ist,
m) Wirkstoffe vom Typ der Naphthalindicarbonsäurederivate, die als
   Saatbeizmittel bekannt sind, wie z. B.
   "Naphthalic anhydrid" (PM) (= 1,8-Naphthalindicarbonsäureanhydrid), das als Saatbeiz-Safener für Mais gegen Schäden von Thiocarbamatherbiziden bekannt ist,
n) Wirkstoffe vom Typ Chromanessigsäurederivate, wie z. B.
   "CL 304415" (CAS-Reg. Nr. 31541-57-8) (= 2-(4-Carboxy-chroman-4-yl)-essigsäure von der Firma American Cyanamid), das als Safener für Mais gegen Schäden von lmidazolinonen bekannt ist,
o) Wirkstoffe, die neben einer herbiziden Wirkung gegen Schadpflanzen auch Safenerwirkung an Kulturpflanzen wie Reis aufweisen, wie z. B.
   "Dimepiperate" oder "MY-93" (PM) (= Piperidin-1-thiocarbonsäure-S-1-methyl-1-phenylethylester), das als Safener für Reis gegen Schäden des Herbizids Molinate bekannt ist,
   "Daimuron" oder "SK 23" (PM) (= 1-(1-Methyl-1-phenylethyl)-3-p-tolyl-harnstoff), das als Safener für Reis gegen Schäden des Herbizids
   Imazosulfuron bekannt ist,
   "Cumyluron" = "JC-940" (= 3-(2-Chlorphenylmethyl)-1-(1-methyl-1-phenylethyl)-harnstoff, siehe JP-A-60087254), das als Safener für Reis gegen Schäden einiger Herbizide bekannt ist,
   "Methoxyphenon" oder "NK 049" (= 3,3'-Dimethyl-4-methoxy-benzophenon), das als Safener für Reis gegen Schäden einiger Herbizide bekannt ist,
   "CSB" (= 1-Brom-4-(chlormethylsulfonyl)-benzol) (CAS-Reg. Nr. 54091-06-4 von Kumiai), das als Safener gegen Schäden einiger Herbizide in Reis bekannt ist,
p) N-Acylsulfonamide der Formel (S3) und ihre Salze, 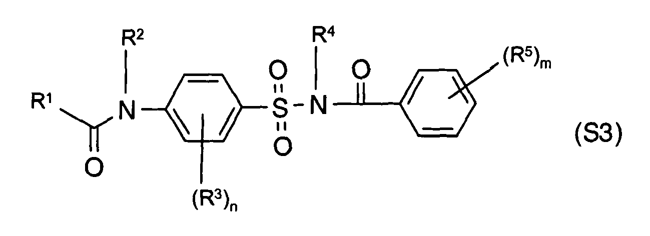 wie sie in WO-A-97/45016 beschrieben sind,
q) Acylsulfamoylbenzoesäureamide der allgemeinen Formel (S4), gegebenenfalls auch in Salzform, 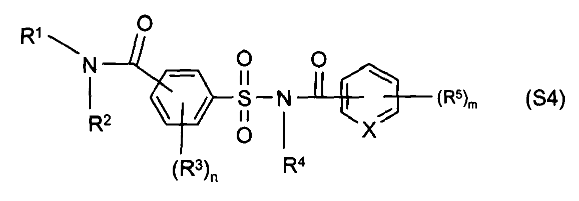 wie sie in der Internationalen Anmeldung Nr. PCT/EP98/06097 beschrieben sind, beispielsweise "cyprosulfamide" (S4-1) und
r) Verbindungen der Formel (S5), 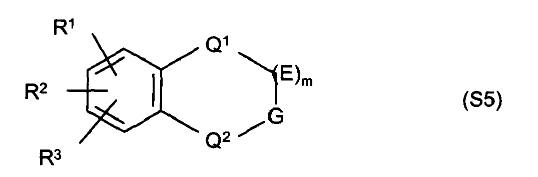 wie sie in der WO-A 98/13 361 beschrieben sind, einschließlich der Stereoisomeren und den in der Landwirtschaft gebräuchlichen Salzen.

Von besonderem Interesse sind unter den genannten Safenern sind (S1-1), (S1-9), (S2-1) und (S4-1).
Einige der Safener sind bereits als Herbizide bekannt und entfalten somit neben der Herbizidwirkung bei Schadpflanzen zugleich auch Schutzwirkung bei den Kulturpflanzen.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Herbizid- oder Herbizid-Safener-Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken. Für die Anwendung als Herbizid zur Bekämpfung von Schadpflanzen liegt sie beispielsweise im Bereich von 0,001 bis 10,0 kg/ha oder mehr Aktivsubstanz, vorzugsweise im Bereich von 0,005 bis 5 kg/ha, insbesondere im Bereich von 0,01 bis 1 kg/ha Aktivsubstanz. Dies gilt sowohl für die Anwendung im Vorauflauf oder im Nachauflauf.

Bei der Anwendung als Pflanzenwachstumsregulator, beispielsweise als Halmverkürzer bei Kulturpflanzen, wie sie oben genannt worden sind, vorzugsweise Getreidepflanzen wie Weizen, Gerste, Roggen, Triticale, Hirse, Reis oder Mais, liegt die Aufwandmenge beispielsweise im Bereich von 0,001 bis 2 kg/ha oder mehr Aktivsubstanz, vorzugsweise im Bereich von 0,005 bis 1 kg/ha, insbesondere im Bereich von 10 bis 500 g/ha Aktivsubstanz, ganz besonders von 20 bis 250 g/ha Aktivsubstanz. Dies gilt sowohl für die Anwendung im Vorauflauf oder im Nachauflauf, wobei die Nachauflaufbehandlung in der Regel bevorzugt ist.

Die Applikation als Halmverkürzer kann in verschiedenen Stadien des Wachstums der Pflanzen erfolgen. Bevorzugt ist beispielsweise die Anwendung nach der Bestockung am Beginn des Längenwachstums.

Alternativ kommt bei der Anwendung als Pflanzenwachstumsregulator auch die Behandlung des Saatguts in Frage, welche die unterschiedlichen Saatgutbeiz- und Beschichtungstechniken einschließt. Die Aufwandmenge hängt dabei von den einzelnen Techniken ab und kann in Vorversuchen ermittelt werden.

Nachfolgend sind beispielhaft einige Synthesebeispiele von Verbindungen der allgemeinen Formel (I) beschrieben.

In den Beispielen beziehen sich Mengenangaben (auch Prozentangaben) auf das Gewicht, sofern nichts anderes speziell angegeben ist. Wenn im Rahmen der Beschreibung und der Beispiele Bezeichnungen "R" und "S" für die absolute Konfiguration an einem Chiralitätszentrum der Stereoisomeren der Formel (I) angegeben ist, folgt diese der RS-Nomenklatur nach der Cahn-Ingold- Prelog-Regel, wenn nichts anderes näher definiert ist.

### (A) Synthesebeispiele

### Beispiel A1 [5-(3-Chlorphenyl)-3-methyl-1-pyridin-3-yl-1 H-pyrazol-4-yl] essigsäuremethylester (siehe Tabelle 2, Beispiel 2-17)

Zu 0,650 g (2,419 mmol) Methyl 3-(3-chlorbenzoyl)-4-oxopentanoat gab man 0,577 g (4,233 mmol) 3-Hydrazinopyridin in 5,00 ml Ethanol und rührte 1,5 h bei 130°C in einem geschlossenen Gefäß im Mikrowellenofen. Man entfernte das Lösemittel im Vakuum, nahm den Rückstand in Dichlormethan auf und wusch zweimal mit je 25 ml Wasser. Die vereinigten organischen Phasen wurden über Natriumsulfat getrocknet und das Lösemittel im Vakuum entfernt. Nach Chromatographie des Rückstands erhielt man 0,364 g (42% d. Th.) eines hellgelben Öls. ¹H-NMR (CDCl₃): Siehe Tabelle 2.

### Beispiel A2 [5-(3-Chlorphenyl)-3-methyl-1-pyridin-3-yl-1H-pyrazol-4-yl]essigsäure (siehe Tabelle 1, Beispiel 1-17)

Zu 0,114 g (0,334 mmol) [5-(3-chlorphenyl)-3-methyl-1-pyridin-3-yl-1H-pyrazol-4-yl]-essigsäuremethylester (siehe A1) gab man 0,053 g (1,334 mmol) 2 molare wässrige Natronlauge und rührte 1 h bei 20°C. Man entfernte das bei der Reaktion entstandene Methanol im Vakuum und goss den Rückstand auf ein Gemisch von 10 ml Wasser und 15 ml Dichlormethan. Die wässrige Phase wurde mit 15 ml Dichlormethan extrahiert, mit konzentrierter Salzsäure angesäuert (pH = 3) und dreimal mit je 15 ml Dichlormethan extrahiert. Nach Trocknen der vereinigten organischen Phasen und Entfernen des Lösemittels im Vakuum erhielt man 0,050 g (43% d. Th.) eines hellbeigen Feststoffs mit Schmelzpunkt 163°C.

### Beispiel A3 [3-Methyl-1-(4-methylpyridin-3-yl)-5-phenyl-1H-pyrazol-4-yl]-essigsäuremethylester (siehe Tabelle 2, Beispiel 2-10)

Zu 0,400 g (1,708 mmol) 3-benzoyl-4-oxopentansäuremethylester gab man 0,315 g (2,049 mmol) 3-Hydrazino-4-methylpyridin in 5,00 ml Ethanol und rührte 8 h unter Rückfluss. Man entfernte das Lösemittel im Vakuum und erhielt nach Chromatographie des Rückstands 0,305 g (47% d. Th.) eines rötlichen wachsartigen Feststoffs. ¹H-NMR (CDCl₃): Siehe Tabelle 2.

### Beispiel A4 [3-Methyl-1-(4-methylpyridin-3-yl)-5-phenyl-1 H-pyrazol-4-yl]-essigsäure (siehe Tabelle 1, Beispiel 1-10)

Zu 0,200 g (0,498 mmol) [3-methyl-1-(4-methylpyridin-3-yl)-5-phenyl-1H-pyrazol-4-yl]-essigsäuremethylester in 5,00 ml Methanol gab man 0,100 g (2,489 mmol) 2 molare wässrige Natronlauge und rührte 1 h bei 20°C. Man entfernte das Methanol im Vakuum und goss den Rückstand auf ein Gemisch von 10 ml Wasser und 15 ml Dichlormethan. Die wässrige Phase wurde mit 15 ml Dichlormethan extrahiert, mit konzentrierter Salzsäure angesäuert (pH = 3) und dreimal mit je 15 ml Dichlormethan extrahiert. Nach Trocknen der vereinigten organischen Phasen und Entfernen des Lösemittels im Vakuum erhielt man 0,108 g (71% d. Th.) eines gelben Feststoffs mit Schmelzpunkt 246°C.

### Beispiel A5 [3-Methyl-1-(4-methylpyridin-3-yl)-5-phenyl-1H-pyrazol-4-yl]-essigsäure-prop-2-in-1-ylester (siehe Tabelle 3, Beispiel 3-76)

Zu 0,130 g (0,423 mmol) [3-Methyl-1-(4-methylpyridin-3-yl)-5-phenyl-1 H-pyrazol-4-yl]-essigsäure gab man 0,445 g (6,345 mmol) Prop-2-in-1-ol sowie einen Tropfen konzentrierte Schwefelsäure und rührte 4 h unter Rückfluss. Man entfernte das Lösemittel im Vakuum, versetzte den Rückstand mit einem Gemisch aus 10 ml Dichlormethan, 1,5 ml Diisopropylethylamin und 10 ml Wasser und extrahierte die wässrige Phase mit 15 ml Dichlormethan. Die vereinigten organischen Phasen wurden über Natriumsulfat getrocknet und das Lösemittel im Vakuum entfernt. Nach Chromatographie des Rückstands erhielt man 0,083 g (57% d. Th.) eines hellbeigen Feststoffs mit Schmelzpunkt 106°C.

### Beispiel A6 [3-Methyl-1-(4-methylpyridin-3-yl)-5-phenyl-1H-pyrazol-4-yl]-essigsäure-cyclopropylmethylester (siehe Tabelle 3, Beispiel 3-99)

Zu 0,175 g (0,569 mmol) [3-Methyl-1-(4-methylpyridin-3-yl)-5-phenyl-1H-pyrazol-4-yl]-essigsäure gab man 0,296 g (2,575 mmol) Dichlor(methoxy)methan und rührte 2 h bei 60°C. Man entfernte das überschüssige Dichlor(methoxy)methan im Vakuum, gab zum Rückstand 0,660 g (9,153 mmol) Cyclopropylmethanol und rührte 0,5 h bei 20°C. Man entfernte das Lösemittel im Vakuum, versetzte den Rückstand mit einem Gemisch aus 10 ml Dichlormethan, 1,5 ml Diisopropylethylamin und 10 ml Wasser und extrahierte die wässrige Phase mit 15 ml Dichlormethan. Die vereinigten organischen Phasen wurden über Natriumsulfat getrocknet und das Lösemittel im Vakuum entfernt. Nach Chromatographie des Rückstands erhielt man 0,123 g (59% d. Th.) eines farblosen Feststoffs mit Schmp. 87°C.

Die in den nachfolgenden Tabellen beschriebenen Verbindungen erhält man gemäß den oder analog zu den oben beschriebenen Beispielen.

In den Tabellen bedeuten:
- F, Cl, Br, J =: Fluor, Chlor, Brom bzw. Jod entsprechend den üblichen chemischen Atomsymbolen
- Me =: Methyl
- MeO oder OMe =: Methoxy
- 2,6-Me₂ =: 2,6-Dimethyl (z. B. als Substitution am Phenylring)
- Et =: Ethyl
- Pr =: n-Propyl
- iPr =: Isopropyl
- iOPr =: Isopropyloxy
- cyPr =: Cyclopropyl
- tBu =: tertiär-Butyl
- Ph =: Phenyl
- PhO =: Phenoxy
- Ac =: COCH₃ = Acetyl
- Allyl =: Prop-2-en-1-yl
- COOH =: Carboxy
Im übrigen gelten die üblichen chemischen Symbole, wie z. B. CH₂ für Methylen oder CF₃ für Trifluormethyl oder OH für Hydroxy. Zusammengesetzte Bedeutungen sind entsprechend aus den genannten Abkürzungen zusammengesetzt definiert.

In der Spalte zu physikalische Daten ("Daten") der Tabellen bedeuten:
- "NMR" =: Daten gemäß ¹H-NMR-Spektum (¹H-Kernresonanz-Daten) sind am Ende der jeweiligen Tabelle angegeben.
- "Schmp." =: Schmelzpunkt
- "(R⁶)ₙ = H" =: unsubstituierter Cyclus (n = 0)

**Tabelle 1: Verbindungen der Formel (I")**

| | | | | | | |
|---|---|---|---|---|---|---|
| 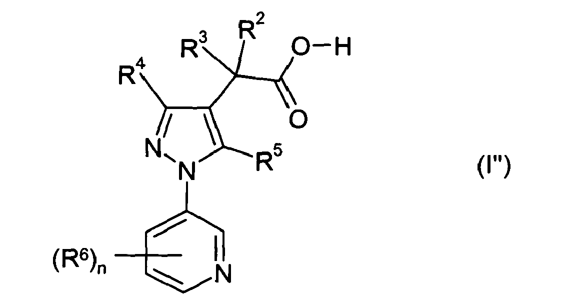 | | | | | | |

| Nr. | R² | R³ | R⁴ | R⁵ | (R⁶)ₙ | Daten |
|---|---|---|---|---|---|---|
| 1-1 | H | H | Ph | Ph | H | Schmp. 172°C |
| 1-2 | H | H | Me | Ph | H | Schmp. 192°C |
| 1-3 | H | H | Me | 2-Thienyl | 5-F | |
| 1-4 | H | H | Me | 2-Furyl | H | |
| 1-5 | H | H | Me | Ph | 6-OMe | Schmp. 55°C |
| 1-6 | Me | H | Me | Ph | 4-Me | |
| 1-7 | H | H | Me | Ph | 6-Cl | NMR |
| 1-8 | H | H | Me | Ph | 4-CF₃ | Schmp. 158°C |
| 1-9 | H | H | Me | Ph | 6- CF₃ | NMR |
| 1-10 | H | H | Me | Ph | 4-Me | Schmp. 246°C |
| 1-11 | H | H | Me | Ph | 2,6-Me₂ | NMR |
| 1-12 | H | H | Me | Ph | 2,6-Cl₂ | Schmp. 147°C |
| 1-13 | H | H | Me | 4-MeO-Ph | 4-Me | Schmp. 202°C |
| 1-14 | H | H | Me | 4-MeO-Ph | H | NMR |
| 1-15 | Me | H | Me | Ph | H | |
| 1-16 | H | H | Me | 4-Me-Ph | H | NMR |
| 1-17 | H | H | Me | 3-Cl-Ph | H | Schmp. 163°C |
| 1-18 | H | H | Me | 3-CF₃-Ph | H | NMR |
| 1-19 | H | H | Me | 3-CF₃-Ph | 4-Me | Schmp. 202°C |
| 1-20 | H | H | Me | 3,4-Cl₂-Ph | 4-Me | Schmp. 192°C |
| 1-21 | H | H | Me | 3-Cl-Ph | 4-Me | Schmp. 172°C |
| 1-22 | H | H | Me | 2-Cl-Ph | 4-Me | NMR |
| 1-23 | H | H | Me | 2,4-Cl₂-Ph | 4-Me | NMR |
| 1-24 | H | H | Me | 4-CF₃-Ph | 4-Me | Schmp. 208°C |
| 1-25 | H | H | Me | 4-Cl-Ph | 4-Me | Schmp. 238°C |
| 1-26 | H | H | Me | 4-Cl-Ph | H | Schmp. 155°C |
| 1-27 | H | H | Me | 3,4-Dichlor-Ph | H | Schmp. 147°C |
| 1-28 | H | H | Me | 4-CF₃-Ph | H | Schmp. 156°C |
| 1-29 | H | H | Me | 4-Cl-Ph | 4-Cl | Schmp. 228°C |
| 1-30 | H | H | Me | Ph | 4-Cl | Schmp. 223°C |
| 1-31 | H | H | Me | 2-Cl-Ph | H | Schmp. 238°C |
| 1-32 | H | H | Me | 4-tBu-Ph | 4-Me | Schmp. 256°C, NMR |
| 1-33 | H | H | Me | 3,5-Me₂-Ph | 4-Me | Schmp. 221°C |
| 1-34 | H | H | Me | Ph | 4-OMe | NMR |
| 1-35 | H | H | Me | 4-Cl-Ph | 4-OMe | Schmp. 224°C |
| 1-36 | H | H | Me | 4-Me-Ph | 4-Me | Schmp. 234°C |
| 1-37 | H | H | Me | 4-F-Ph | 4-Me | Schmp. 227°C |
| 1-38 | H | H | Me | 3-Me-Ph | 4-Me | Schmp. 204°C |
| 1-39 | H | H | Me | 4-(COOH)-Ph | 4-Me | Schmp. 269°C |
| 1-40 | H | H | Me | 3-Br-Ph | 4-Me | Schmp. 184°C |
| 1-41 | H | H | Me | 4-Ph-Ph | 4-Me | Schmp. 232°C |
| 1-42 | H | H | Me | 4-(COOH)-Ph | H | Schmp. 169°C |
| 1-43 | H | H | Me | 3,5-Me₂-Ph | H | Schmp. 192°C |
| 1-44 | H | H | Me | Ph | 4-SMe | |
| 1-45 | H | H | Me | 4-Cl-Ph | 4-SMe | Schmp. 238°C |
| 1-46 | H | H | Me | 3-Cl-4-Me-Ph | H | Schmp. 187°C |
| 1-47 | H | H | Me | 3-CF₃-4-Cl-Ph | H | Schmp. 177°C |
| 1-48 | H | H | Me | 3-CF₃-4-Cl-Ph | 4-Me | Schmp. 176°C |
| 1-49 | H | H | Me | 3-Cl-4-Me-Ph | 4-Me | Schmp. 191°C |
| 1-50 | H | H | Me | Ph | 5-Me | Schmp. 165°C |
| 1-51 | H | H | Me | 4-Cl-Ph | 5-Me | Schmp. 185°C |
| 1-52 | H | H | Me | 2-Thienyl | 4-Me | Schmp. 243°C |
| 1-53 | H | H | Me | 3-Me-2-thienyl | 4-Me | Schmp. 211 °C |
| 1-54 | H | H | Me | 4-Me-2-thienyl | 4-Me | Schmp. 246°C |
| 1-55 | H | H | Me | 5-Cl-2-thienyl | 4-Me | Schmp. 266°C |
| 1-56 | H | H | Me | 3-Thienyl | 4-Me | Schmp. 247°C |
| 1-57 | H | H | Me | 2-Thienyl | H | Schmp. 190°C |
| 1-58 | H | H | Me | 3-Me-2-thienyl | H | Schmp. 145°C |
| 1-59 | H | H | Me | 4-Me-2-thienyl | H | Schmp. 170°C |
| 1-60 | H | H | Me | 5-Cl-2-thienyl | H | Schmp. 210°C |
| 1-61 | H | H | Me | 5-Me-2-thienyl | H | Schmp. 216°C |
| 1-62 | H | H | Me | 6-MeO-pyridin-3-yl | H | Schmp. 162°C |
| 1-63 | H | H | Me | 3-Thienyl | H | Schmp. 189°C |
| 1-64 | H | H | Me | 4-Cl-Ph | 4-S(O)Me | Schmp. 216°C |
| 1-65 | H | H | Me | 4-Br-Ph | 4-Me | |
| 1-66 | H | H | Me | 1,3-Benzodioxol-5-yl | 4-Me | |
| 1-67 | H | H | Me | 4-J-Ph | 4-Me | |
| 1-68 | H | H | Me | 3,5-Cl₂-Ph | 4-Me | |
| 1-69 | H | H | Me | 4-PhO-Ph | 4-Me | |
| 1-70 | H | H | Me | 6-OH-pyridin-3-yl | H | Schmp. 255°C |
| 1-71 | H | H | Me | Ph | 4-S(O)Me | NMR |
| 1-72 | H | H | H | Ph | H | Schmp. 145°C |
| 1-73 | H | H | H | Ph | 4-Me | Schmp. 208°C |
| 1-74 | H | H | Et | Ph | H | |
| 1-75 | H | H | n-Pr | Ph | H | |
| 1-76 | H | H | CH₂Cl | Ph | H | |
| 1-77 | H | H | CHCl₂ | Ph | H | |
| 1-78 | H | H | CH₂F | Ph | H | |
| 1-79 | H | H | CHF₂ | Ph | H | |
| 1-80 | H | H | Cl | Ph | H | |
| 1-81 | H | H | Et | Ph | 4-Me | |
| 1-82 | H | H | n-Pr | Ph | 4-Me | |
| 1-83 | H | H | CH₂Cl | Ph | 4-Me | |
| 1-84 | H | H | CHCl₂ | Ph | 4-Me | |
| 1-85 | H | H | CH₂F | Ph | 4-Me | |
| 1-86 | H | H | CHF₂ | Ph | 4-Me | |
| 1-87 | H | H | Cl | Ph | 4-Me | |
| 1-88 | H | H | Et | 4-Cl-Ph | H | |
| 1-89 | H | H | n-Pr | 4-Cl-Ph | H | |
| 1-90 | H | H | CH₂Cl | 4-Cl-Ph | H | |
| 1-91 | H | H | CHCl₂ | 4-Cl-Ph | H | |
| 1-92 | H | H | CH₂F | 4-Cl-Ph | H | |
| 1-93 | H | H | CHF₂ | 4-Cl-Ph | H | |
| 1-94 | H | H | Cl | 4-Cl-Ph | H | |
| 1-95 | H | H | Et | 4-Me-Ph | H | |
| 1-96 | H | H | n-Pr | 4-Me-Ph | H | |
| 1-97 | H | H | CH₂Cl | 4-Me-Ph | H | |
| 1-98 | H | H | CHCl₂ | 4-Me-Ph | H | |
| 1-99 | H | H | CH₂F | 4-Me-Ph | H | |
| 1-100 | H | H | CHF₂ | 4-Me-Ph | H | |
| 1-101 | H | H | Cl | 4-Me-Ph | H | |
| 1-102 | H | H | Et | 2-Pyridyl | H | |
| 1-103 | H | H | n-Pr | 2-Pyridyl | H | |
| 1-104 | H | H | CH₂Cl | 2-Pyridyl | H | |
| 1-105 | H | H | CHCl₂ | 2-Pyridyl | H | |
| 1-106 | H | H | CH₂F | 2-Pyridyl | H | |
| 1-107 | H | H | CHF₂ | 2-Pyridyl | H | |
| 1-108 | H | H | Cl | 2-Pyridyl | H | |
| 1-109 | H | H | Me | 2-Pyridyl | H | |
| 1-110 | H | H | Me | 5-Cl-pyridin-2-yl | H | |
| 1-111 | H | H | Me | 5-Br-pyridin-2-yl | H | |
| 1-112 | H | H | Me | 5-F-pyridin-2-yl | H | |
| 1-113 | H | H | Me | 5-Me-pyridin-2-yl | H | |
| 1-114 | H | H | Me | 2,4-Cl₂-Ph | H | |
| 1-115 | H | H | Me | 4-CH₂COOH-Ph | 4-Me | |
| 1-116 | H | H | Me | 3,4-Me₂-Ph | 4-Me | |
| 1-117 | H | H | Me | 4-Br-Ph | 4-Me | |
| 1-118 | H | H | Me | 3,4- Me₂-Ph | H | |
| 1-119 | H | H | Me | 3-Me-Ph | H | |
| 1-120 | H | H | Me | 4-F-Ph | H | NMR |
| 1-121 | H | H | Me | 4-(Me-CO)-Ph | H | |
| 1-122 | H | H | Me | 4-tBu-Ph | H | |
| 1-123 | H | H | Me | 4-Cl-3-Me-Ph | H | |
| 1-124 | H | H | n-Pr | 4-Cl-Ph | 4-Me | NMR |
| 1-125 | H | H | Me | 3-Pyridyl | H | |
| 1-126 | H | H | Me | 4-Pyridyl | H | |
| 1-127 | H | H | C(O)OMe | Ph | H | |
| 1-128 | H | H | Me | 6-Me-pyridin-3-yl | H | |
| 1-129 | H | H | Me | 4-Cl-Ph | 4-SO₂Me | |
| 1-130 | H | H | Me | 3-Pyridyl | 4-Me | |
| 1-131 | H | H | Me | 2,3-Cl₂-Ph | 4-Me | |
| 1-132 | H | H | Me | 2-Pyridyl | 4-Me | |
| 1-133 | H | H | H | 4-Cl-Ph | 4-Me | |
| 1-134 | H | H | Me | 6-Cl-pyridin-3-yl | H | |

"NMR" der Beispielverbindungen wurden jeweils als ¹H-NMR-Spektum bei 300 MHz (CDCl₃) (¹H-Kernresonanz-Daten) gemessen. Nachfolgend sind charakterische chemische Verschiebungen δ (ppm) für einige Beispielverbindungen aufgeführt:

### Beisp. Nr.: δ (ppm)

- 1-7:: 2,37 (s, 3H), 3,41 (s, 2H), 7,23 (m, 3H), 7,4 (m, 3H), 7,57 (dd, 1 H), 8,21 (d, 1H)
- 1-9:: 7,60 (d, 1 H), 7,77 (dd, 1 H), 8,58 (d, 1 H)
- 1-11:: 2,18 (s, 3H), 2,23 (s, 3H), 2,50 (s, 3H), 7,85 (d, 1H)
- 1-14:: 3,80 (s, 3H), 6,90 (d, 2H), 7,17 (d, 2H)
- 1-16:: 2,37 (s, 3H), 2,38 (s, 3H), 3,42 (s, 2H), 7,10 (d, 2H), 7,18 (d, 2H)
- 1-18:: 2,38 (s, 3H), 3,41 (s, 2H), 7,41 (d, 1 H), 7,51 (t, 1 H), 7,55 (s,1H), 7,63 (d, 1 H), 8,49 (m, 2H)
- 1-22:: 2,27 (s, 3H), 2,38 (s, 3H), 3,35 (d, 1 H), 3,42 (d, 1 H), 7,18 (d, 1 H), 8,23 (s, 1 H), 8,35 (d, 1 H)
- 1-23:: 2,25 (s, 3H), 2,39 (s, 3H), 3,35 (d, 1 H), 3,42 (d, 1H), 7,37 (s, 1 H), 8,25 (s, 1 H), 8,39 (d, 1 H)
- 1-31:: 2,40 (s, 3H), 3,30 (d, 1H), 3,41 (d, 1H), 7,61 (m, 1 H), 8,45 (m, 2H)
- 1-34:: 2,38 (s, 3H), 3,46 (s, 2H), 3,59 (s, 3H), 6,78 (d, 1 H), 8,45 (d, 1 H), 8,50 (s, 1 H)
- 1-71:: 2,37 (s, 3H), 2,95 (s, 3H), 3,40 (d, 1 H), 3,48 (d, 1 H), 8,08 (d, 1 H), 8,12 (s, 1 H), 8,70 (d, 1 H)
- 1-120:: 2,39 (s, 3H), 3,42 (s, 2H), 7,08 (t, 1 H)
- 1-124:: 1,02 (t, 3H), 1,77 (sext, 2H), 2,08 (s, 3H), 2,70 (t, 2H), 3,44 (s, 2H), 7,12 (d, 2H), 7,19 (d, 1 H), 7,27 (d, 2H), 8,39 (s, 1 H), 8,42 (d, 1 H)

**Tabelle 2: Verbindungen der Formel (I'")**

| | | | | | | |
|---|---|---|---|---|---|---|
| 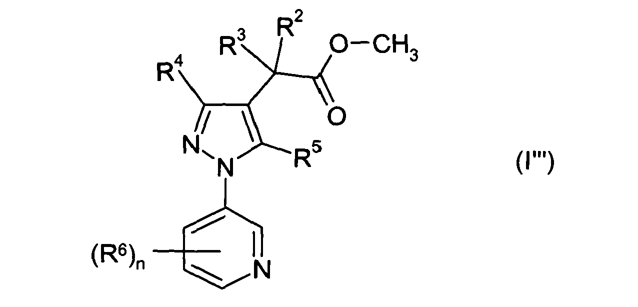 | | | | | | |

| Nr. | R² | R³ | R⁴ | R⁵ | (R⁶)ₙ | Daten |
|---|---|---|---|---|---|---|
| 2-1 | H | H | Ph | Ph | H | NMR |
| 2-2 | H | H | Me | Ph | H | NMR |
| 2-3 | H | H | Me | 2-Thienyl | 5-F | |
| 2-4 | H | H | Me | 2-Furyl | H | |
| 2-5 | H | H | Me | Ph | 6-OMe | NMR |
| 2-6 | Me | H | Me | Ph | 4-Me | NMR |
| 2-7 | H | H | Me | Ph | 6-Cl | NMR |
| 2-8 | H | H | Me | Ph | 4-CF3 | NMR |
| 2-9 | H | H | Me | Ph | 6-CF3 | NMR |
| 2-10 | H | H | Me | Ph | 4-Me | NMR |
| 2-11 | H | H | Me | Ph | 2,6-Me₂ | |
| 2-12 | H | H | Me | Ph | 2,6-Cl₂ | NMR |
| 2-13 | H | H | Me | 4-MeO-Ph | 4-Me | NMR |
| 2-14 | H | H | Me | 4-MeO-Ph | H | NMR |
| 2-15 | Me | H | Me | Ph | H | |
| 2-16 | H | H | Me | 4-Me-Ph | H | NMR |
| 2-17 | H | H | Me | 3-Cl-Ph | H | NMR |
| 2-18 | H | H | Me | 3-CF₃-Ph | H | NMR |
| 2-19 | H | H | Me | 3-CF₃-Ph | 4-Me | NMR |
| 2-20 | H | H | Me | 3,4-Cl₂-Ph | 4-Me | NMR |
| 2-21 | H | H | Me | 3-Cl-Ph | 4-Me | NMR |
| 2-22 | H | H | Me | 2-Cl-Ph | 4-Me | NMR |
| 2-23 | H | H | Me | 2,4-Cl₂-Ph | 4-Me | NMR |
| 2-24 | H | H | Me | 4-CF₃-Ph | 4-Me | Schmp. 96°C |
| 2-25 | H | H | Me | 4-Cl-Ph | 4-Me | NMR |
| 2-26 | H | H | Me | 4-Cl-Ph | H | NMR |
| 2-27 | H | H | Me | 3,4-Cl₂-Ph | H | NMR |
| 2-28 | H | H | Me | 4-CF₃-Ph | H | NMR |
| 2-29 | H | H | Me | 4-Cl-Ph | 4-Cl | NMR |
| 2-30 | H | H | Me | Ph | 4-Cl | Schmp. 100°C |
| 2-31 | H | H | Me | 2-Cl-Ph | H | NMR |
| 2-32 | H | H | Me | 4-tBu-Ph | 4-Me | NMR |
| 2-33 | H | H | Me | 3,5-Me₂-Ph | 4-Me | |
| 2-34 | H | H | Me | Ph | 4-OMe | Schmp. 144°C |
| 2-35 | H | H | Me | 4-CI-Ph | 4-OMe | NMR |
| 2-36 | H | H | Me | 4-Me-Ph | 4-Me | NMR |
| 2-37 | H | H | Me | 4-F-Ph | 4-Me | NMR |
| 2-38 | H | H | Me | 3-Me-Ph | 4-Me | NMR |
| 2-39 | H | H | Me | 4-COOH-Ph | 4-Me | |
| 2-40 | H | H | Me | 3-Br-Ph | 4-Me | |
| 2-41 | H | H | Me | 4-Ph-Ph | 4-Me | Schmp. 153°C |
| 2-42 | H | H | Me | 4-COOH-Ph | H | |
| 2-43 | H | H | Me | 3,5-Me₂-Ph | H | NMR |
| 2-44 | H | H | Me | Ph | 4-SMe | NMR |
| 2-45 | H | H | Me | 4-Cl-Ph | 4-SMe | NMR |
| 2-46 | H | H | Me | 3-Cl-4-Me-Ph | H | NMR |
| 2-47 | H | H | Me | 3-CF₃-4-chlor-Ph | H | NMR |
| 2-48 | H | H | Me | 3-CF₃-4-CI-Ph | 4-Me | NMR |
| 2-49 | H | H | Me | 3-Cl-4-Me-Ph | 4-Me | NMR |
| 2-50 | H | H | Me | Ph | 5-Me | NMR |
| 2-51 | H | H | Me | 4-Cl-Ph | 5-Me | NMR |
| 2-52 | H | H | Me | 2-Thienyl | 4-Me | Schmp. 99°C |
| 2-53 | H | H | Me | 3-Me-2-thienyl | 4-Me | NMR |
| 2-54 | H | H | Me | 4-Me-2-thienyl | 4-Me | Schmp. 94°C |
| 2-55 | H | H | Me | 5-Cl-2-thienyl | 4-Me | Schmp. 105°C |
| 2-56 | H | H | Me | 3-Thienyl | 4-Me | Schmp. 104°C |
| 2-57 | H | H | Me | 2-Thienyl | H | NMR |
| 2-58 | H | H | Me | 3-Me-2-thienyl | H | NMR |
| 2-59 | H | H | Me | 4-Me-2-thienyl | H | NMR |
| 2-60 | H | H | Me | 5-Cl-2-thienyl | H | NMR |
| 2-61 | H | H | Me | 5-Me-2-thienyl | H | NMR |
| 2-62 | H | H | Me | 6-MeO-pyridin-3-yl | H | NMR |
| 2-63 | H | H | Me | 3-Thienyl | H | NMR |
| 2-64 | H | H | Me | 4-Cl-Ph | 4-S(O)Me | Schmp. 161°C |
| 2-65 | H | H | Me | 4-Br-Ph | 4-Me | |
| 2-66 | H | H | Me | 1,3-Benzodioxol-5-yl | 4-Me | Schmp. 91°C |
| 2-67 | H | H | Me | 4-J-Ph | 4-Me | |
| 2-68 | H | H | Me | 3,5-Cl₂-Ph | 4-Me | Schmp. 112°C |
| 2-69 | H | H | Me | 4-PhO-Ph | 4-Me | |
| 2-70 | H | H | Me | 6-OH-pyridin-3-yl | H | Schmp. 171°C |
| 2-71 | H | H | Me | Ph | 4-S(O)Me | Schmp. 163°C |
| 2-72 | H | H | H | Ph | H | NMR |
| 2-73 | H | H | H | Ph | 4-Me | NMR |
| 2-74 | H | H | Et | Ph | H | NMR |
| 2-75 | H | H | n-Pr | Ph | H | |
| 2-76 | H | H | CH₂Cl | Ph | H | |
| 2-77 | H | H | CHCl₂ | Ph | H | |
| 2-78 | H | H | CH₂F | Ph | H | |
| 2-79 | H | H | CHF₂ | Ph | H | NMR |
| 2-80 | H | H | Cl | Ph | H | |
| 2-81 | H | H | Et | Ph | 4-Me | |
| 2-82 | H | H | n-Pr | Ph | 4-Me | |
| 2-83 | H | H | CH₂Cl | Ph | 4-Me | |
| 2-84 | H | H | CHCl₂ | Ph | 4-Me | |
| 2-85 | H | H | CH₂F | Ph | 4-Me | |
| 2-86 | H | H | CHF₂ | Ph | 4-Me | |
| 2-87 | H | H | Cl | Ph | 4-Me | |
| 2-88 | H | H | Et | 4-Cl-Ph | H | |
| 2-89 | H | H | n-Pr | 4-Cl-Ph | H | NMR |
| 2-90 | H | H | CH₂Cl | 4-Cl-Ph | H | |
| 2-91 | H | H | CHCl₂ | 4-Cl-Ph | H | |
| 2-92 | H | H | CH₂F | 4-Cl-Ph | H | |
| 2-93 | H | H | CHF₂ | 4-Cl-Ph | H | |
| 2-94 | H | H | Cl | 4-Cl-Ph | H | |
| 2-95 | H | H | Et | 4-Me-Ph | H | |
| 2-96 | H | H | n-Pr | 4-Me-Ph | H | |
| 2-97 | H | H | CH₂Cl | 4-Me-Ph | H | |
| 2-98 | H | H | CHCl₂ | 4-Me-Ph | H | |
| 2-99 | H | H | CH₂F | 4-Me-Ph | H | |
| 2-100 | H | H | CHF₂ | 4-Me-Ph | H | |
| 2-101 | H | H | Cl | 4-Me-Ph | H | |
| 2-102 | H | H | Et | 2-Pyridyl | H | |
| 2-103 | H | H | n-Pr | 2-Pyridyl | H | |
| 2-104 | H | H | CH₂Cl | 2-Pyridyl | H | |
| 2-105 | H | H | CHCl₂ | 2-Pyridyl | H | |
| 2-106 | H | H | CH₂F | 2-Pyridyl | H | |
| 2-107 | H | H | CHF₂ | 2-Pyridyl | H | |
| 2-108 | H | H | Cl | 2-Pyridyl | H | |
| 2-109 | H | H | Me | 2-Pyridyl | H | NMR |
| 2-110 | H | H | Me | 5-Cl-pyridin-2-yl | H | |
| 2-111 | H | H | Me | 5-Br-pyridin-2-yl | H | |
| 2-112 | H | H | Me | 5-F-pyridin-2-yl | H | |
| 2-113 | H | H | Me | 5-Me-pyridin-2-yl | H | |
| 2-114 | H | H | Me | 2,4-Cl₂-Ph | H | NMR |
| 2-115 | H | H | Me | 4-(CH₂COOH)-Ph | 4-Me | NMR |
| 2-116 | H | H | Me | 3,4-Me₂-Ph | 4-Me | NMR |
| 2-117 | H | H | Me | 4-Br-Ph | 4-Me | |
| 2-118 | H | H | Me | 3,4-Me₂-Ph | H | |
| 2-119 | H | H | Me | 3-Me-Ph | H | NMR |
| 2-120 | H | H | Me | 4-F-Ph | H | NMR |
| 2-121 | H | H | Me | 4-(Me-CO)-Ph | H | NMR |
| 2-122 | H | H | Me | 4-tBu-Ph | H | NMR |
| 2-123 | H | H | Me | 4-Cl-3-Me-Ph | H | NMR |
| 2-124 | H | H | n-Pr | 4-Cl-Ph | 4-Me | NMR |
| 2-125 | H | H | Me | 3-Pyridyl | H | NMR |
| 2-126 | H | H | Me | 4-Pyridyl | H | NMR |
| 2-127 | H | H | C(O)OMe | Ph | H | NMR |
| 2-128 | H | H | Me | 6-Me-pyridin-3-yl | H | NMR |
| 2-129 | H | H | Me | 4-Cl-Ph | 4-SO₂Me | |
| 2-130 | H | H | Me | 3-Pyridyl | 4-Me | |
| 2-131 | H | H | Me | 2,3-Cl₂-Ph | 4-Me | Schmp. 140°C |
| 2-132 | H | H | Me | 2-Pyridyl | 4-Me | |
| 2-133 | H | H | H | 4-Cl-Ph | 4-Me | NMR |
| 2-134 | H | H | Me | 6-Cl-pyridin-3-yl | H | NMR |

"NMR" der Beispielverbindungen wurde jeweils als ¹H-NMR-Spektum bei 300 MHz (CDCl₃) (¹H-Kernresonanz-Daten) gemessen. Nachfolgend sind charakterische chemische Verschiebungen δ (ppm) für einige Beispielverbindungen aufgeführt:

### Beisp. Nr.: δ (ppm)

- 2-1:: 3,59 (s, 2H), 3,62 (s, 3H), 7,2-7,8 (m, 12H), 8,44 (dd, 1 H), 8,59 (d, 1 H)
- 2-2:: 2,41 (s, 3H), 3,50 (s, 2H), 3,77 (s, 3H), 7,63 (m, 1 H), 8,50 (dd, 1 H), 8,54 (d, 1 H)
- 2-5:: 2,39 (s, 3H), 3,43 (s, 2H), 3,70 (s, 3H), 3,90 (s, 3H), 6,66 (d, 1 H), 7,98 (d, 1 H)
- 2-6:: 1,42 (d, 3H), 2,03 (s, 3H), 2,38 (s, 3H), 3,68 (s, 3H), 3,73 (q, 1 H)
- 2-7:: 2,37 (s, 3H), 3,40 (s, 2H), 3,70 (s, 3H), 7,57 (dd, 1 H), 8,11 (d, 1 H)
- 2-8:: 2,37 (s, 3H), 3,43 (s, 2H), 3,74 (s, 3H), 7,60 (d, 1 H), 8,43 (s, 1 H), 8,73 (d, 1 H)
- 2-9:: 2,38 (s, 3H), 3,41 (s, 2H), 3,70 (s, 3H), 7,60 (d, 1 H), 7,78 (dd, 1 H), 8,57 (d, 1H)
- 2-10:: 2,03 (s, 3H), 2,38 (s, 3H), 3,43 (s, 2H), 3,76 (s, 3H), 8,40 (m, 2H)
- 2-12:: 2,37 (s, 3H), 3,45 (s, 2H), 3,71 (s, 3H), 7,17-7,37 (m, 6H), 7,59 (m, 1 H)
- 2-13:: 2,02 (s, 3H), 2,37 (s, 3H), 3,45 (s, 2H), 3,74 (s, 3H), 3,78 (s, 3H), 6,80 (d, 2H), 7,07 (d, 2H)
- 2-14:: 2,37 (s, 3H), 3,40 (s, 2H), 3,72 (s, 3H), 3,78 (s, 3H), 6,90 (d, 2H), 7,13 (d, 2H)
- 2-16:: 2,37 (s, 3H), 2,38 (s, 3H), 3,41 (s, 2H), 3,72 (s, 3H), 7,08 (d, 2H), 7,17 (d, 2H)
- 2-17:: 2,37 (s, 3H), 3,40 (s, 2H), 3,71 (s, 3H), 7,08 (dt, 1 H), 7,20-7,38 (m, 4H), 7,58 (m, 1 H), 8,44 (m, 2H)
- 2-18:: 2,38 (s, 3H), 3,40 (s, 2H), 3,73 (s, 3H), 7,39 (d, 1 H), 7,51 (t, 1 H), 7,55 (s,1 H), 7,63 (d, 1 H)
- 2-19:: 2,10 (s, 3H), 2,38 (s, 3H), 3,47 (s, 2H), 3,76 (s, 3H), 7,34 (d, 1 H), 7,41 (t, 1 H), 7,47 (s,1 H), 7,58 (d, 1 H)
- 2-20:: 2,06 (s, 3H), 2,35 (s, 3H), 3,42 (s, 2H), 3,76 (s, 3H), 6,95 (dd, 1H), 7,15 (d, 1 H), 8,41 (d, 1 H)
- 2-21:: 2,07 (s, 3H), 2,35 (s, 3H), 3,43 (s, 2H), 3,75 (s, 3H), 7,00 (dd, 1 H), 7,15 (d, 1 H), 8,40 (d, 1 H)
- 2-22:: 2,25 (s, 3H), 2,38 (s, 3H), 3,35 (d, 1 H), 3,42 (d, 1 H), 3,62 (s, 3H), 7,15 (d, 1H), 8,23 (s, 1 H), 8,33 (d, 1 H)
- 2-23:: 2,22 (s, 3H), 2,38 (s, 3H), 3,30 (d, 1 H), 3,40 (d, 1 H), 3,62 (s, 3H), 8,20 (s, 1 H), 8,35 (d, 1 H)
- 2-25:: 2,02 (s, 3H), 2,37 (s, 3H), 3,43 (s, 2H), 3,75 (s, 3H), 7,10 (d, 2H), 7,26 (d, 2H), 8,38 (s, 1 H), 8,42 (d, 1 H)
- 2-26:: 2,37 (s, 3H), 3,40 (s, 2H), 3,72 (s, 3H), 7,18 (d, 2H), 7,25 (m, 1 H), 7,37 (d, 2H), 7,58 (m, 1 H), 8,46 (m, 2H)
- 2-27:: 2,38 (s, 3H), 3,41 (s, 2H), 3,75 (s, 3H), 7,05 (dd, 1 H), 7,40 (d, 1 H), 7,45 (d, 1 H)
- 2-28:: 2,37 (s, 3H), 3,40 (s, 2H), 3,72 (s, 3H), 7,38 (d, 2H), 7,65 (d, 2H)
- 2-29:: 2,38 (s, 3H), 3,45 (s, 2H), 3,75 (s, 3H), 7,15 (d, 2H), 7,30 (d, 2H), 7,36 (d, 1 H), 8,48 (d, 1 H), 8,55 (s, 1H)
- 2-31:: 2,44 (s, 3H), 3,38 (d, 1 H), 3,48 (d, 1 H), 3,75 (s, 3H), 8,50 (m, 2H)
- 2-32:: 1,25 (s, 9H), 2,06 (s, 3H), 2,37 (s, 3H), 3,49 (s, 2H), 3,76 (s, 3H), 7,05 (d, 2H), 7,15 (d, 1H), 7,30 (d, 2H), 8,37 (s, 1 H), 8,39 (d, 1 H)
- 2-35:: 2,38 (s, 3H), 3,43 (s, 2H), 3,61 (s, 3H), 3,73 (s, 3H), 6,77 (d, 1 H), 7,14 (d, 2H), 7,26 (d, 2H), 8,43 (d, 1 H), 8,46 (s, 1 H)
- 2-36:: 2,04 (s, 3H), 2,30 (s, 3H), 2,37 (s, 3H), 3,43 (s, 2H), 3,75 (s, 3H), 7,02 (d, 2H), 7,08 (d, 2H)
- 2-37:: 2,06 (s, 3H), 2,38 (s, 3H), 3,44 (s, 2H), 3,75 (s, 3H), 6,99 (t, 2H), 8,38 (s, 1 H), 8,40 (d, 1 H)
- 2-38:: 2,05 (s, 3H), 2,25 (s, 3H), 2,37 (s, 3H), 3,47 (s, 2H), 3,74 (s, 3H), 6,92 (d, 1 H), 6,96 (s, 1 H)
- 2-43:: 2,25 (s, 6H), 2,37 (s, 3H), 3,41 (s, 2H), 3,72 (s, 3H), 6,80 (s, 2H), 7,00 (s, 1 H)
- 2-44:: 2,37 (s, 3H), 2,41 (s, 3H), 3,46 (s, 2H), 3,72 (s, 3H), 7,09 (d, 1 H), 8,12 (s, 1 H), 8,38 (d, 1 H)
- 2-45:: 2,37 (s, 3H), 2,41 (s, 3H), 3,43 (s, 2H), 3,74 (s, 3H), 7,09 (d, 1H), 7,18 (d, 2H), 7,27 (d, 2H), 8,14 (s, 1 H), 8,39 (d, 1 H)
- 2-46:: 2,36 (s, 3H), 2,39 (s, 3H), 3,41 (s, 2H), 3,73 (s, 3H), 6,98 (dd, 1 H), 7,21 (d, 1 H), 7,26 (m, 2H)
- 2-47:: 2,38 (s, 3H), 3,40 (s, 2H), 3,73 (s, 3H), 7,30 (m, 2H), 7,51 (d, 1 H)
- 2-48:: 2,09 (s, 3H), 2,38 (s, 3H), 3,43 (s, 2H), 3,77 (s, 3H), 7,23 (dd, 1 H), 7,41 (d, 1 H), 7,59 (d, 1 H)
- 2-49:: 2,08 (s, 3H), 2,34 (s, 3H), 2,36 (s, 3H), 3,43 (s, 2H), 3,77 (s, 3H), 6,90 (dd, 1 H), 7,12 (d, 1 H), 7,19 (d, 1 H)
- 2-50:: 2,30 (s, 3H), 2,37 (s, 3H), 3,42 (s, 2H), 3,71 (s, 3H), 7,53 (t, 1 H), 8,12 (d, 1 H), 8,26 (d, 1 H)
- 2-51: 2,33 (s, 3H), 2,37 (s, 3H), 3,40 (s, 2H), 3,71 (s, 3H), 7,17 (d, 2H), 7,37 (d, 2H), 7,57 (t, 1 H), 8,10 (d, 1 H), 8,30 (d, 1 H)
- 2-53:: 1,98 (s, 3H), 2,20 (s, 3H), 2,37 (s, 3H), 3,43 (s, 2H), 3,67 (s, 3H), 6,79 (d, 1 H), 7,17 (d, 1 H), 7,23 (d, 1 H), 8,30 (s, 1 H), 8,39 (d, 1 H)
- 2-57:: 2,36 (s, 3H), 3,50 (s, 2H), 3,72 (s, 3H), 7,02 (m, 1 H), 7,08 (m, 1 H), 7,28 (m, 1 H), 7,41 (d, 1 H), 7,65 (dt, 1 H), 8,48 (dd, 1 H), 8,54 (d, 1 H)
- 2-58:: 1,86 (s, 3H), 2,37 (s, 3H), 3,42 (breites d, 2H), 3,68 (s, 3H), 6,88 (d, 1 H), 7,23 (dd, 1 H), 7,38 (d, 1 H), 7,61 (dt, 1 H), 8,45 (dd, 1 H), 8,51 (d, 1 H)
- 2-59:: 2,23 (s, 3H), 2,34 (s, 3H), 3,50 (s, 2H), 3,72 (s, 3H), 6,81 (d, 1 H), 6,98 (d, 1 H), 7,28 (dd, 1 H), 7,68 (dt, 1 H), 8,49 (dd, 1 H), 8,55 (d, 1 H)
- 2-60:: 2,35 (s, 3H), 3,49 (s, 2H), 3,74 (s, 3H), 6,82 (d, 1 H), 6,88 (d, 1 H), 7,31 (dd, 1 H), 7,69 (dt, 1 H), 8,53 (dd, 1 H), 8,59 (d, 1 H)
- 2-61:: 2,36 (s, 3H), 2,46 (d, 3H), 3,50 (s, 2H), 3,72 (s, 3H), 6,70 (m, 1 H), 6,80 (d, 1 H), 7,29 (dd, 1 H), 7,70 (dt, 1 H), 8,49 (dd, 1 H), 8,57 (d, 1 H)
- 2-62:: 2,38 (s, 3H), 3,42 (s, 2H), 3,72 (s, 3H), 3,97 (s, 3H), 6,74 (d, 1 H), 7,28 (dd, 1 H), 7,39 (dd, 1 H), 7,62 (dt, 1 H), 8,08 (d, 1 H), 8,49 (m, 2H)
- 2-63:: 2,37 (s, 3H), 3,47 (s, 2H), 3,75 (s, 3H), 6,80 (dd, 1 H), 7,28 (dd, 1 H), 7,34 (dd, 1 H), 7,42 (dd, 1 H), 7,62 (dt, 1 H), 8,49 (dd, 1 H), 8,52 (d, 1 H)
- 2-72:: 3,50 (s, 2H), 3,70 (s, 3H), 7,82 (s, 1 H), 8,47 (dd, 1 H), 8,50 (d, 1 H)
- 2-73:: 2,05 (s, 3H), 3,56 (s, 2H), 3,73 (s, 3H), 7,82 (s, 1 H), 8,38 (s, 1 H), 8,41 (d, 1 H)
- 2-74:: 1,34 (t, 3H), 2,75 (q, 2H), 3,43 (s, 2H), 3,70 (s, 3H)
- 2-79:: 3,60 (s, 2H), 3,73 (s, 3H), 6,84 (t, 1 H, ²*J*_{HF} = 55 Hz)
- 2-89:: 1,03 (t, 3H), 1,77 (sext, 2H), 2,66 (t, 2H), 3,41 (s, 2H), 3,71 (s, 3H), 7,19 (d, 2H), 7,37 (d, 2H)
- 2-109:: 2,37 (s, 3H), 3,60 (s, 2H), 3,66 (s, 3H), 7,25 (m, 3H), 7,66 (m, 2H), 8,39 (d, 1 H), 8,44 (dd, 1 H), 8,60 (m, 1 H)
- 2-114:: 2,38 (s, 3H), 3,25 (d, 1 H), 3,39 (d, 1 H), 3,65 (s, 3H), 7,42 (m, 1 H), 8,40 (m, 1 H), 8,43 (m, 1 H)
- 2-115:: 2,05 (s, 3H), 2,38 (s, 3H), 3,47 (s, 2H), 3,74 (s, 3H), 3,90 (s, 3H), 7,23 (d, 2H), 7,96 (d, 2H)
- 2-116:: 2,05 (s, 3H), 2,18 (s, 3H), 2,20 (s, 3H), 2,35 (s, 3H), 3,46 (s, 2H), 3,74 (s, 3H), 6,92 (s, 1 H), 7,01 (d, 1 H)
- 2-118:: 2,20 (s, 3H), 2,27 (s, 3H), 2,37 (s, 3H), 3,41 (s, 2H), 3,72 (s, 3H), 6,92 (d, 1 H), 6,99 (s, 1 H), 7,12 (d, 1 H)
- 2-119:: 2,32 (s, 3H), 2,38 (s, 3H), 3,41 (s, 2H), 3,72 (s, 3H), 6,99 (d, 1 H), 7,02 (s, 1 H)
- 2-120:: 2,38 (s, 3H), 3,41 (s, 2H), 3,73 (s, 3H), 7,08 (t, 1 H)
- 2-121:: 2,38 (s, 3H), 3,42 (s, 2H), 3,73 (s, 3H), 3,94 (s, 3H), 7,33 (d, 2H), 8,04 (d, 2H)
- 2-122:: 1,32 (s, 9H), 2,38 (s, 3H), 3,42 (s, 2H), 3,72 (s, 3H), 7,13 (d, 2H), 7,38 (d, 2H)
- 2-123:: 2,35 (s, 3H), 2,37 (s, 3H), 3,40 (s, 2H), 3,73 (s, 3H), 6,97 (dd, 1 H), 7,12 (d, 1 H), 7,33 (d, 1 H)
- 2-124:: 1,03 (t, 3H), 1,77 (sext, 2H), 2,06 (s, 3H), 2,67 (t, 2H), 3,43 (s, 2H), 3,72 (s, 3H), 7,10 (d, 2H), 7,14 (d, 1H), 7,26 (d, 2H), 8,38 (s, 1H), 8,41 (d, 1H)
- 2-125:: 2,38 (s, 3H), 3,42 (s, 2H), 3,72 (s, 3H), 7,26 (m, 1 H), 7,34 (m, 1 H), 7,59 (m, 2H), 8,43-8,60 (m, 3H), 8,62 (dd, 1 H)
- 2-126:: 2,38 (s, 3H), 3,43 (s, 2H), 3,73 (s, 3H), 7,18 (d, 2H), 7,27 (dd, 1 H), 7,57 (dt, 1 H), 8,50 (d, 1 H), 8,53 (dd, 1H), 8,63 (d, 2H)
- 2-127:: 3,72 (s, 3H), 3,75 (s, 2H), 3,97 (s, 3H)
- 2-128:: 2,38 (s, 3H), 2,59 (s, 3H), 3,42 (s, 2H), 3,71 (s, 3H), 7,18 (d, 1H), 7,28 (dd, 1 H), 7,44 (dd, 1 H), 7,62 (dt, 1 H), 8,39 (d, 1 H), 8,45 (d, 1 H), 8,49 (dd, 1 H)
- 2-133:: 2,05 (s, 3H), 3,52 (s, 2H), 3,73 (s, 3H), 7,07 (d, 2H), 7,15 (d, 1 H), 7,28 (d, 2H), 7,80 (s, 1H), 8,37 (s, 1H), 8,42 (d, 1 H)
- 2-134:: 2,37 (s, 3H), 3,40 (s, 2H), 3,72 (s, 3H), 7,28 (dd, 1 H), 7,35 (d, 1 H),7,53 (dd, 1 H), 7,59 (dt, 1 H), 8,30 (d, 1 H), 8,45 (d, 1 H), 8,51 (dd, 1 H)

**Tabelle 3: Verbindungen der Formel (I"")**

| | | | | | |
|---|---|---|---|---|---|
| 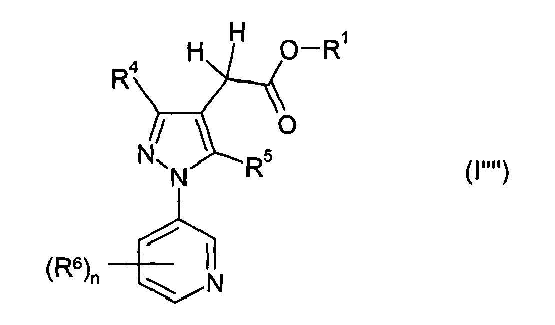 | | | | | |

| Nr. | R¹ | R⁴ | R⁵ | (R⁶)ₙ | Daten |
|---|---|---|---|---|---|
| 3-1 | Et | Me | 4-Cl-Ph | 4-Me | NMR |
| 3-2 | Et | Me | 4-Cl-Ph | 4-Me | |
| 3-3 | Et | Me | 2-Thienyl | 4-Me | |
| 3-4 | Et | Me | 3-Thienyl | 4-Me | |
| 3-5 | Et | Me | 3-Me-2-thienyl | 4-Me | |
| 3-6 | Et | Me | 4-Me-2-thienyl | 4-Me | |
| 3-7 | Et | Me | 5-Cl-2-thienyl | 4-Me | |
| 3-8 | Et | Me | 5-Me-2-thienyl | 4-Me | |
| 3-9 | Et | Me | 3-Pyridyl | 4-Me | |
| 3-10 | Et | Me | 6-MeO-pyridin-3-yl | 4-Me | |
| 3-11 | Et | Me | 6-OH-pyridin-3-yl | 4-Me | |
| 3-12 | Et | Me | 6-Me-pyridin-3-yl | 4-Me | |
| 3-13 | Et | Me | 4-Me-Ph | 4-Me | |
| 3-14 | Et | Me | 4-Br-Ph | 4-Me | |
| 3-15 | Et | Me | 4-F-Ph | 4-Me | |
| 3-16 | Et | Me | 5-Cl-pyridin-2-yl | 4-Me | |
| 3-17 | Et | Me | 5-Br-pyridin-2-yl | 4-Me | |
| 3-18 | Et | Me | 5-F-pyridin-2-yl | 4-Me | |
| 3-19 | Et | Me | 5-Me-pyridin-2-yl | 4-Me | |
| 3-20 | Et | Me | 2-Pyridyl | 4-Me | |
| 3-21 | Et | Me | 4-Pyridyl | 4-Me | |
| 3-22 | Pr | Me | Ph | H | |
| 3-23 | Pr | Me | 4-Cl-Ph | H | |
| 3-24 | Pr | Me | 2-Thienyl | H | |
| 3-25 | Pr | Me | 3-Pyridyl | H | |
| 3-26 | Pr | Me | 6-Me-pyridin-3-yl | H | |
| 3-27 | Pr | Me | 4-Me-Ph | H | |
| 3-28 | Pr | Me | 4-Br-Ph | H | |
| 3-29 | Pr | Me | 4-F-Ph | H | |
| 3-30 | Pr | Me | 5-Cl-pyridin-2-yl | H | |
| 3-31 | Pr | Me | 5-Br-pyridin-2-yl | H | |
| 3-32 | Pr | Me | 5-F-pyridin-2-yl | H | |
| 3-33 | Pr | Me | 5-Me-pyridin-2-yl | H | |
| 3-34 | Pr | Me | 2-Pyridyl | H | |
| 3-35 | Pr | Me | 4-Pyridyl | H | |
| 3-36 | i-Pr | Me | Ph | H | NMR |
| 3-37 | i-Pr | Me | 4-Cl-Ph | H | |
| 3-38 | i-Pr | Me | 2-Thienyl | H | |
| 3-39 | i-Pr | Me | 3-Pyridyl | H | |
| 3-40 | i-Pr | Me | 6-Me-pyridin-3-yl | H | |
| 3-41 | i-Pr | Me | 4-Me-Ph | H | |
| 3-42 | i-Pr | Me | 4-Br-Ph | H | |
| 3-43 | i-Pr | Me | 4-F-Ph | H | |
| 3-44 | i-Pr | Me | 5-Cl-pyridin-2-yl | H | |
| 3-45 | i-Pr | Me | 5-Br-pyridin-2-yl | H | |
| 3-46 | i-Pr | Me | 5-F-pyridin-2-yl | H | |
| 3-47 | i-Pr | Me | 5-Me-pyridin-2-yl | H | |
| 3-48 | i-Pr | Me | 2-Pyridyl | H | |
| 3-49 | i-Pr | Me | 4-Pyridyl | H | |
| 3-50 | CH₂Ph | Me | Ph | H | Schmp. 107°C |
| 3-51 | CH₂Ph | Me | 4-Cl-Ph | H | |
| 3-52 | CH₂Ph | Me | 2-Thienyl | H | |
| 3-53 | CH₂Ph | Me | 3-Pyridyl | H | |
| 3-54 | prop-2-in-1-yl | Me | Ph | H | NMR |
| 3-55 | prop-2-in-1-yl | Me | 4-Cl-Ph | H | NMR |
| 3-56 | prop-2-in-1-yl | Me | 2-Thienyl | H | |
| 3-57 | prop-2-in-1-yl | Me | 3-Thienyl | H | |
| 3-58 | prop-2-in-1-yl | Me | 3-Me-2-thienyl | H | |
| 3-59 | prop-2-in-1-yl | Me | 4-Me-2-thienyl | H | |
| 3-60 | prop-2-in-1-yl | Me | 5-Cl-2-thienyl | H | |
| 3-61 | prop-2-in-1-yl | Me | 5-Me-2-thienyl | H | |
| 3-62 | prop-2-in-1-yl | Me | 3-Pyridyl | H | |
| 3-63 | prop-2-in-1-yl | Me | 6-MeO-pyridin-3-yl | H | NMR |
| 3-64 | prop-2-in-1-yl | H | Ph | H | NMR |
| 3-65 | prop-2-in-1-yl | Me | 6-Me-pyridin-3-yl | H | |
| 3-66 | prop-2-in-1-yl | Me | 4-Me-Ph | H | |
| 3-67 | prop-2-in-1-yl | Me | 4-Br-Ph | H | |
| 3-68 | prop-2-in-1-yl | Me | 4-F-Ph | H | NMR |
| 3-69 | prop-2-in-1-yl | Me | 5-Cl-pyridin-2-yl | H | |
| 3-70 | prop-2-in-1-yl | Me | 5-Br-pyridin-2-yl | H | |
| 3-71 | prop-2-in-1-yl | Me | 5-F-pyridin-2-yl | H | |
| 3-72 | prop-2-in-1-yl | Me | 5-Me-pyridin-2-yl | H | |
| 3-73 | prop-2-in-1-yl | Me | 2-Pyridyl | H | NMR |
| 3-74 | prop-2-in-1-yl | Me | 4-Pyridyl | H | NMR |
| 3-75 | prop-2-in-1-yl | Me | 4-Cl-Ph | 4-Me | NMR |
| 3-76 | prop-2-in-1-yl | Me | Ph | 4-Me | Schmp. 106°C |
| 3-77 | cyclopropylmethyl | Me | Ph | H | NMR |
| 3-78 | cyclopropylmethyl | Me | 4-Cl-Ph | H | NMR |
| 3-79 | cyclopropylmethyl | Me | 2-Thienyl | H | |
| 3-80 | cyclopropylmethyl | Me | 3-Thienyl | H | |
| 3-81 | cyclopropylmethyl | Me | 3-Me-2-thienyl | H | |
| 3-82 | cyclopropylmethyl | Me | 3-Pyridyl | H | |
| 3-83 | cyclopropylmethyl | Me | 5-Cl-2-thienyl | H | |
| 3-84 | cyclopropylmethyl | Me | 5-Me-2-thienyl | H | |
| 3-85 | cyclopropylmethyl | Me | 4-Me-2-thienyl | H | NMR |
| 3-86 | cyclopropylmethyl | Me | 6-MeO-pyridin-3-yl | H | NMR |
| 3-87 | cyclopropylmethyl | Me | 6-OH-pyridin-3-yl | H | NMR |
| 3-88 | cyclopropylmethyl | Me | 6-Me-pyridin-3-yl | H | |
| 3-89 | cyclopropylmethyl | Me | 4-Me-Ph | H | |
| 3-90 | cyclopropylmethyl | Me | 4-Br-Ph | H | |
| 3-91 | cyclopropylmethyl | Me | 4-F-Ph | H | NMR |
| 3-92 | cyclopropylmethyl | Me | 5-Cl-pyridin-2-yl | H | |
| 3-93 | cyclopropylmethyl | Me | 5-Br-pyridin-2-yl | H | |
| 3-94 | cyclopropylmethyl | Me | 5-F-pyridin-2-yl | H | |
| 3-95 | cyclopropylmethyl | Me | 5-Me-pyridin-2-yl | H | |
| 3-96 | cyclopropylmethyl | Me | 2-Pyridyl | H | |
| 3-97 | cyclopropylmethyl | Me | 4-Pyridyl | H | |
| 3-98 | cyclopropylmethyl | Me | 4-Cl-Ph | 4-Me | NMR |
| 3-99 | cyclopropylmethyl | Me | Ph | 4-Me | Schmp. 87°C |
| 3-100 | cyclopropylmethyl | H | Ph | H | NMR |
| 3-101 | 3,3-dichlor-2-fluorprop-2-en-1-yl | Me | Ph | H | NMR |
| 3-102 | 3,3-dichlor-2-fluorprop-2-en-1-yl | Me | 4-Cl-Ph | H | |
| 3-103 | 3,3-dichlor-2-fluorprop-2-en-1-yl | Me | 2-Thienyl | H | |
| 3-104 | 3,3-dichlor-2-fluorprop-2-en-1-yl | Me | 3-Pyridyl | H | |
| 3-105 | (1-methylcyclopropyl)-methyl | Me | Ph | H | NMR |
| 3-106 | (1-methylcyclopropyl)-methyl | Me | 4-Cl-Ph | H | |
| 3-107 | (1-methylcyclopropyl)-methyl | Me | 2-Thienyl | H | |
| 3-108 | (1-methylcyclopropyl)-methyl | Me | 3-Pyridyl | H | |
| 3-109 | 4-chlorbut-2-in-1-yl | Me | Ph | H | NMR |
| 3-110 | 4-chlorbut-2-in-1-yl | Me | 4-Cl-Ph | H | |
| 3-111 | 4-chlorbut-2-in-1-yl | Me | 2-Thienyl | H | |
| 3-112 | 4-chlorbut-2-in-1-yl | Me | 3-Pyridyl | H | |
| 3-113 | (2,2-dichlorcyclopropyl)-methyl | Me | Ph | H | NMR |
| 3-114 | (2,2-dichlorcyclopropyl)-methyl | Me | 4-Cl-Ph | H | |
| 3-115 | (2,2-dichlorcyclopropyl)-methyl | Me | 2-Thienyl | H | |
| 3-116 | (2,2-dichlorcyclopropyl)-methyl | Me | 3-Pyridyl | H | |
| 3-117 | but-2-in-1-yl | Me | Ph | H | NMR |
| 3-118 | but-2-in-1-yl | Me | 4-Cl-Ph | H | NMR |
| 3-119 | but-2-in-1-yl | Me | 2-Thienyl | H | |
| 3-120 | but-2-in-1-yl | Me | 3-Thienyl | H | |
| 3-121 | but-2-in-1-yl | Me | 3-Me-2-thienyl | H | |
| 3-122 | but-2-in-1-yl | Me | 4-Me-2-thienyl | H | |
| 3-123 | but-2-in-1-yl | Me | 5-Cl-2-thienyl | H | |
| 3-124 | but-2-in-1-yl | Me | 5-Me-2-thienyl | H | |
| 3-125 | but-2-in-1-yl | Me | 3-Pyridyl | H | |
| 3-126 | but-2-in-1-yl | Me | 6-MeO-pyridin-3-yl | H | |
| 3-127 | but-2-in-1-yl | H | Ph | H | |
| 3-128 | but-2-in-1-yl | Me | 6-Me-pyridin-3-yl | H | |
| 3-129 | but-2-in-1-yl | Me | 4-Me-Ph | H | |
| 3-130 | but-2-in-1-yl | Me | 4-Br-Ph | H | |
| 3-131 | but-2-in-1-yl | Me | 4-F-Ph | H | |
| 3-132 | but-2-in-1-yl | Me | 5-Cl-pyridin-2-yl | H | |
| 3-133 | but-2-in-1-yl | Me | 5-Br-pyridin-2-yl | H | |
| 3-134 | but-2-in-1-yl | Me | 5-F-pyridin-2-yl | H | |
| 3-135 | but-2-in-1-yl | Me | 5-Me-pyridin-2-yl | H | |
| 3-136 | but-2-in-1-yl | Me | 2-Pyridyl | H | NMR |
| 3-137 | but-2-in-1-yl | Me | 4-Pyridyl | H | |
| 3-138 | but-2-in-1-yl | Me | 4-Cl-Ph | 4-Me | |
| 3-139 | but-2-in-1-yl | Me | Ph | 4-Me | NMR |
| 3-140 | 1-methylprop-2-in-1-yl | Me | Ph | H | NMR |
| 3-141 | 1-methylprop-2-in-1-yl | Me | 4-Cl-Ph | H | |
| 3-142 | 1-methylprop-2-in-1-yl | Me | 2-Thienyl | H | |
| 3-143 | 1-methylprop-2-in-1-yl | Me | 3-Pyridyl | H | |
| 3-144 | 1-cyclopropylethyl | Me | Ph | H | NMR |
| 3-145 | 1-cyclopropylethyl | Me | 4-Cl-Ph | H | |
| 3-146 | 1-cyclopropylethyl | Me | 2-Thienyl | H | |
| 3-147 | 1-cyclopropylethyl | Me | 3-Pyridyl | H | |
| 3-148 | allyl | Me | Ph | H | NMR |
| 3-149 | allyl | Me | 4-Cl-Ph | H | |
| 3-150 | allyl | Me | 2-Thienyl | H | |
| 3-151 | allyl | Me | 3-Pyridyl | H | |
| 3-152 | 3-methylbut-2-en-1-yl | Me | Ph | H | NMR |
| 3-153 | 3-methylbut-2-en-1-yl | Me | 4-Cl-Ph | H | |
| 3-154 | 3-methylbut-2-en-1-yl | Me | 2-Thienyl | H | |
| 3-155 | 3-methylbut-2-en-1-yl | Me | 3-Pyridyl | H | |
| 3-156 | 2-methylprop-2-en-1-yl | Me | Ph | H | NMR |
| 3-157 | 2-methylprop-2-en-1-yl | Me | 4-Cl-Ph | H | |
| 3-158 | 2-methylprop-2-en-1-yl | Me | 2-Thienyl | H | |
| 3-159 | 2-methylprop-2-en-1-yl | Me | 3-Pyridyl | H | |
| 3-160 | (2E)-1-methylbut-2-en-1-yl | Me | Ph | H | NMR |
| 3-161 | (2E)-1-methylbut-2-en-1-yl | Me | 4-Cl-Ph | H | |
| 3-162 | (2E)-1-methylbut-2-en-1-yl | Me | 2-Thienyl | H | |
| 3-163 | (2E)-1-methylbut-2-en-1-yl | Me | 3-Pyridyl | H | |
| 3-164 | 3-phenylprop-2-in-1-yl | Me | Ph | H | NMR |
| 3-165 | 3-phenylprop-2-in-1-yl | Me | 4-Cl-Ph | H | |
| 3-166 | 3-phenylprop-2-in-1-yl | Me | 2-Thienyl | H | |
| 3-167 | 3-phenylprop-2-in-1-yl | Me | 3-Pyridyl | H | |
| 3-168 | cyclobutylmethyl | Me | Ph | H | NMR |
| 3-169 | cyclobutylmethyl | Me | 4-Cl-Ph | H | |
| 3-170 | cyclobutylmethyl | Me | 2-Thienyl | H | |
| 3-171 | cyclobutylmethyl | Me | 3-Pyridyl | H | |
| 3-172 | cyclopentylmethyl | Me | Ph | H | NMR |
| 3-173 | cyclopentylmethyl | Me | 4-Cl-Ph | H | |
| 3-174 | cyclopentylmethyl | Me | 2-Thienyl | H | |
| 3-175 | cyclopentylmethyl | Me | 3-Pyridyl | H | |
| 3-176 | cyclohexylmethyl | Me | Ph | H | |
| 3-177 | cyclohexylmethyl | Me | 4-Cl-Ph | H | NMR |
| 3-178 | cyclohexylmethyl | Me | 2-Thienyl | H | |
| 3-179 | cyclohexylmethyl | Me | 3-Pyridyl | H | |
| 3-180 | but-3-en-1-yl | Me | Ph | H | NMR |
| 3-181 | but-3-en-1-yl | Me | 4-Cl-Ph | H | |
| 3-182 | but-3-en-1-yl | Me | 2-Thienyl | H | |
| 3-183 | but-3-en-1-yl | Me | 3-Pyridyl | H | |
| 3-184 | 2-chloroprop-2-en-1-yl | Me | Ph | H | NMR |
| 3-185 | 2-chloroprop-2-en-1-yl | Me | 4-Cl-Ph | H | NMR |
| 3-186 | 2-chloroprop-2-en-1-yl | Me | 2-Thienyl | H | NMR |
| 3-187 | 2-chloroprop-2-en-1-yl | Me | 3-Thienyl | H | NMR |
| 3-188 | 2-chloroprop-2-en-1-yl | Me | 3-Me-2-thienyl | H | NMR |
| 3-189 | 2-chloroprop-2-en-1-yl | Me | 4-Me-2-thienyl | H | NMR |
| 3-190 | 2-chloroprop-2-en-1-yl | Me | 5-Cl-2-thienyl | H | NMR |
| 3-191 | 2-chloroprop-2-en-1-yl | Me | 5-Me-2-thienyl | H | NMR |
| 3-192 | 2-chloroprop-2-en-1-yl | Me | 3-Pyridyl | H | |
| 3-193 | 2-chloroprop-2-en-1-yl | Me | 6-MeO-pyridin-3-yl | H | NMR |
| 3-194 | 2-chloroprop-2-en-1-yl | Me | 6-OH-pyridin-3-yl | H | |
| 3-195 | 2-chloroprop-2-en-1-yl | Me | 6-Me-pyridin-3-yl | H | NMR |
| 3-196 | 2-chloroprop-2-en-1-yl | Me | 4-Me-Ph | H | |
| 3-197 | 2-chloroprop-2-en-1-yl | Me | 4-Br-Ph | H | |
| 3-198 | 2-chloroprop-2-en-1-yl | Me | 4-F-Ph | H | NMR |
| 3-199 | 2-chloroprop-2-en-1-yl | Me | 5-Cl-pyridin-2-yl | H | |
| 3-200 | 2-chloroprop-2-en-1-yl | Me | 5-Br-pyridin-2-yl | H | |
| 3-201 | 2-chloroprop-2-en-1-yl | Me | 5-F-pyridin-2-yl | H | |
| 3-202 | 2-chloroprop-2-en-1-yl | Me | 5-Me-pyridin-2-yl | H | |
| 3-203 | 2-chloroprop-2-en-1-yl | Me | 2-Pyridyl | H | NMR |
| 3-204 | 2-chloroprop-2-en-1-yl | Me | 4-Pyridyl | H | |
| 3-205 | 2-chloroprop-2-en-1-yl | Me | 4-Cl-Ph | 4-Me | |
| 3-206 | 2-chloroprop-2-en-1-yl | Me | Ph | 4-Me | |
| 3-207 | 2-chloroprop-2-en-1-yl | H | Ph | H | NMR |
| 3-208 | Et | Me | 4-Cl-Ph | H | |
| 3-209 | Et | Me | 2-Thienyl | H | |
| 3-210 | Et | Me | 3-Thienyl | H | |
| 3-211 | Et | Me | 3-Me-2-thienyl | H | |
| 3-212 | Et | Me | 4-Me-2-thienyl | H | |
| 3-213 | Et | Me | 5-Cl-2-thienyl | H | |
| 3-214 | Et | Me | 5-Me-2-thienyl | H | |
| 3-215 | Et | Me | 3-Pyridyl | H | |
| 3-216 | Et | Me | 6-MeO-pyridin-3-yl | H | |
| 3-217 | Et | Me | 6-OH-pyridin-3-yl | H | |
| 3-218 | Et | Me | 6-Me-pyridin-3-yl | H | |
| 3-219 | Et | Me | 4-Me-Ph | H | |
| 3-220 | Et | Me | 4-Br-Ph | H | |
| 3-221 | Et | Me | Ph | H | |
| 3-222 | Et | Me | 4-F-Ph | H | |
| 3-223 | Et | Me | 5-Cl-pyridin-2-yl | H | |
| 3-224 | Et | Me | 5-Br-pyridin-2-yl | H | |
| 3-225 | Et | Me | 5-F-pyridin-2-yl | H | |
| 3-226 | Et | Me | 5-Me-pyridin-2-yl | H | |
| 3-227 | Et | Me | 2-Pyridyl | H | |
| 3-228 | Et | Me | 4-Pyridyl | H | |

"NMR" der Beispielverbindungen wurde jeweils als ¹H-NMR-Spektum bei 300 MHz (CDCl₃) (¹H-Kernresonanz-Daten) gemessen. Nachfolgend sind charakterische chemische Verschiebungen δ (ppm) für einige Beispielverbindungen aufgeführt:

### Beisp. Nr.: δ (ppm)

- 3-1:: 1,27 (t, 3H), 2,04 (s, 3H), 2,38 (s, 3H), 3,41 (s, 2H), 4,18 (q, 2H), 7,10 (d, 2H), 7,25 (d, 2H)
- 3-36:: 1,23 (d, 6H), 2,37 (s, 3H), 3,38 (s, 2H), 5,03 (sept, 1 H)
- 3-54:: 2,37 (s, 3H), 2,49 (t, 1 H), 3,48 (s, 2H), 4,72 (d, 2H)
- 3-55:: 2,38 (s, 3H), 2,51 (t, 1H), 3,44 (s, 2H), 4,72 (d, 2H), 7,19 (d, 2H), 7,38 (d, 2H)
- 3-63:: 2,38 (s, 3H), 2,50 (t, 1 H), 3,45 (s, 2H), 3,95 (s, 3H), 4,72 (d, 2H), 6,75 (d, 1H), 7,28 (dd, 1 H), 7,40 (dd, 1 H), 7,62 (dt, 1 H), 8,08 (d, 1 H), 8,48 (m, 2H)
- 3-64:: 2,49 (t, 1 H), 3,55 (s, 2H), 4,71 (d, 2H), 7,84 (s, 1 H), 8,48 (dd, 1 H), 8,50 (d, 1 H)
- 3-68:: 2,38 (s, 3H), 2,50 (t, 1 H), 3,44 (s, 2H), 4,71 (d, 2H), 7,07 (t, 2H)
- 3-73:: 2,38 (s, 3H), 2,45 (t, 1 H), 3,64 (s, 2H), 4,67 (d, 2H), 7,26 (m, 3H), 7,67 (m, 2H), 8,40 (d, 1 H), 8,46 (dd, 1 H), 8,61 (m, 1 H)
- 3-74:: 2,39 (s, 3H), 2,51 (t, 1 H), 3,50 (s, 2H), 4,73 (d, 2H), 7,17 (d, 2H), 8,63 (d, 2H)
- 3-75:: 2,05 (s, 3H), 2,38 (s, 3H), 2,51 (t, 1 H), 3,50 (s, 2H), 4,73 (d, 2H), 7,10 (d, 2H), 7,28 (d, 2H)
- 3-77:: 0,28 (m, 2H), 0,58 (m, 2H), 1,15 (m, 1 H), 2,38 (s, 3H), 3,42 (s, 2H), 3,96 (d, 2H)
- 3-78:: 0,28 (m, 2H), 0,59 (m, 2H), 1,14 (m, 1 H), 2,39 (s, 3H), 3,41 (s, 2H), 3,97 (d, 2H), 7,20 (d, 2H), 7,37 (d, 2H)
- 3-85:: 0,29 (m, 2H), 0,59 (m, 2H), 1,15 (m, 1 H), 2,23 (s, 3H), 2,37 (s, 3H), 3,50 (s, 2H), 3,96 (d, 2H), 6,86 (d, 1 H), 6,98 (d, 1 H)
- 3-86:: 0,28 (m, 2H), 0,59 (m, 2H), 1,14 (m, 1 H), 2,39 (s, 3H), 2,59 (s, 3H), 3,42 (s, 2H), 3,96 (d, 2H), 7,18 (d, 1 H), 7,27 (dd, 1 H), 7,48 (dd, 1 H), 7,61 (dt, 1 H), 8,39 (d, 1 H), 8,44 (d, 1 H), 8,48 (dd, 1 H)
- 3-87:: 0,30 (m, 2H), 0,60 (m, 2H), 1,16 (m, 1 H), 2,38 (s, 3H), 3,41 (s, 2H), 3,97 (d, 2H), 6,53 (d, 1 H), 7,21 (dd, 1 H), 7,33 (dd, 1 H), 7,58 (d, 1 H), 7,78 (dt, 1 H), 8,53 (dd, 1 H), 8,60 (d, 1 H), 13,38 (breites s, 1 H)
- 3-91:: 0,28 (m, 2H), 0,59 (m, 2H), 1,14 (m, 1 H), 2,38 (s, 3H), 3,41 (s, 2H), 3,95 (d, 2H), 7,07 (t, 2H), 7,58 (dt, 1 H), 8,46 (m, 2H)
- 3-98:: 0,28 (m, 2H), 0,59 (m, 2H), 1,14 (m, 1 H), 2,04 (s, 3H), 2,38 (s, 3H), 3,43 (s, 2H), 3,97 (d, 2H), 7,14 (d, 2H), 7,37 (d, 2H)
- 3-100:: 0,28 (m, 2H), 0,58 (m, 2H), 1,13 (m, 1H), 3,51 (s, 2H), 3,96 (d, 2H), 7,83 (s, 1 H), 8,48 (dd, 1 H), 8,50 (d, 1 H)
- 3-101:: 2,38 (s, 3H), 3,50 (s, 2H), 4,93 (d, 2H)
- 3-105:: 0,38 (m, 2H), 0,48 (m, 2H), 1,10 (s, 3H), 2,40 (s, 3H), 3,45 (s, 2H), 3,93 (s, 2H)
- 3-109:: 2,39 (s, 3H), 3,48 (s, 2H), 4,17 (m, 2H), 4,77 (m, 2H)
- 3-113:: 1,34 (t, 1 H), 1,71 (dd, 1 H), 2,00 (m, 1 H), 2,40 (s, 3H), 3,47 (s, 2H), 4,07 (dd, 1 H), 4,40 (dd, 1 H)
- 3-117:: 1,86 (t, 3H), 2,38 (s, 3H), 3,46 (s, 2H), 4,66 (q, 2H)
- 3-118:: 1,87 (t, 3H), 2,38 (s, 3H), 3,42 (s, 2H), 4,68 (q, 2H), 7,19 (d, 2H), 7,37 (d, 2H)
- 3-138:: 1,87 (t, 3H), 2,05 (s, 3H), 2,38 (s, 3H), 3,47 (s, 2H), 4,68 (q, 2H), 7,12 (d, 2H), 7,27 (d, 2H)
- 3-139:: 1,87 (t, 3H), 2,05 (s, 3H), 2,38 (s, 3H), 3,50 (s, 2H), 4,70 (q, 2H)
- 3-140:: 1,51 (d, 3H), 2,39 (s, 3H), 2,47 (d, 1 H), 3,43 (s, 2H), 5,46 (dq, 1 H)
- 3-144:: 0,23 (m, 1 H), 0,38 (m, 1 H), 0,52 (m, 2H), 1,30 (d, 3H), 2,39 (s, 3H), 3,40 (s, 2H), 4,38 (m, 1 H)
- 3-148:: 2,38 (s, 3H), 3,43 (s, 2H), 4,61 (m, 2H), 5,25 (m, 1 H), 5,30 (m, 1 H), 5,90 (m, 1 H)
- 3-152:: 1,72 (s, 3H), 1,78 (s, 3H), 2,38 (s, 3H), 3,41 (s, 2H), 4,60 (d, 2H), 5,35 (t, 1 H)
- 3-156:: 1,74 (s, 3H), 2,39 (s, 3H), 3,45 (s, 2H), 4,54 (s, 2H), 4,97 (m, 2H)
- 3-160:: 1,28 (d, 3H), 1,70 (m, 3H), 2,38 (s, 3H), 3,40 (s, 2H), 5,34 (quint, 1 H), 5,46 (m, 1 H), 5,71 (dq, 1 H)
- 3-164:: 2,40 (s, 3H), 3,45 (s, 2H),4,94 (s, 2H)
- 3-168:: 1,75 (m, 2H), 1,87 (m, 2H), 2,06 (m, 2H), 2,39 (s, 3H), 2,62 (sept, 1 H), 3,41 (s, 2H), 4,09 (d, 2H)
- 3-172:: 1,22 (m, 2H), 1,58 (m, 4H), 1,72 (m, 2H), 2,20 (sept, 1 H), 2,38 (s, 3H), 3,41 (s, 2H), 4,01 (d, 2H)
- 3-176:: 0,95 (m, 2H), 1,22 (m, 3H), 1,69 (m, 6H), 2,39 (s, 3H), 3,41 (s, 2H), 3,94 (d, 2H)
- 3-180:: 2,05 (s, 3H), 2,37 (s, 3H), 2,40 (q, 2H), 3,47 (s, 2H), 4,20 (t, 2H), 5,10 (m, 2H), 5,78 (m, 1 H)
- 3-184:: 2,39 (s, 3H), 3,50 (s, 2H), 4,70 (s, 2H), 5,40 (m, 2H)
- 3-185:: 2,38 (s, 3H), 3,48 (s, 2H), 4,70 (s, 2H), 5,42 (s, 2H), 7,18 (d, 2H), 7,36 (d, 2H)
- 3-186:: 2,38 (s, 3H), 3,58 (s, 2H), 4,70 (s, 2H), 5,41 (m, 2H), 7,03 (m, 1 H), 7,07 (m, 1 H), 7,29 (dd, 1 H), 7,42 (d, 1 H), 7,66 (dt, 1 H), 8,50 (dd, 1 H), 8,55 (d, 1 H)
- 3-187:: 2,38 (s, 3H), 3,51 (s, 2H), 4,70 (s, 2H), 5,42 (m, 2H), 6,80 (dd, 1 H), 7,28 (dd, 1 H), 7,34 (dd, 1 H), 7,42 (dd, 1 H), 7,61 (dt, 1 H), 8,48 (dd, 1 H), 8,51 (d, 1 H)
- 3-188:: 1,84 (s, 3H), 2,37 (s, 3H), 3,49 (breites d, 2H), 3,68 (s, 3H), 3,57 (s, 2H), 4,64 (s, 2H), 5,39 (s, 2H), 6,87 (d, 1 H), 7,23 (dd, 1 H), 7,38 (d, 1 H), 7,61 (dt, 1 H), 8,43 (dd, 1 H), 8,52 (d, 1 H)
- 3-189:: 2,25 (s, 3H), 2,37 (s, 3H), 3,57 (s, 2H), 4,70 (s, 2H), 5,42 (m, 2H), 6,84 (s, 1 H), 6,99 (s, 1 H), 7,29 (dd, 1 H), 7,69 (dt, 1 H), 8,50 (dd, 1 H), 8,56 (d, 1 H)
- 3-190:: 2,37 (s, 3H), 3,55 (s, 2H), 4,70 (s, 2H), 5,42 (m, 2H), 6,83 (d, 1 H), 6,89 (d, 1 H), 7,31 (dd, 1 H), 7,69 (dt, 1 H), 8,54 (dd, 1 H), 8,59 (d, 1 H)
- 3-191:: 2,36 (s, 3H), 2,45 (s, 3H), 3,56 (s, 2H), 4,70 (s, 2H), 5,42 (m, 2H), 6,70 (d, 1 H), 6,80 (d, 1 H), 7,29 (dd, 1 H), 7,70 (dt, 1 H), 8,49 (dd, 1 H), 8,56 (d, 1 H)
- 3-193:: 2,38 (s, 3H), 3,48 (s, 2H), 3,96 (s, 3H), 4,70 (s, 2H), 5,42 (m, 2H), 6,75 (d, 1 H), 7,28 (dd, 1 H), 7,41 (dd, 1 H), 7,62 (dt, 1 H), 8,08 (d, 1 H), 8,48 (m, 2H)
- 3-195:: 2,39 (s, 3H), 2,59 (s, 3H), 3,48 (s, 2H), 4,70 (s, 2H), 5,42 (m, 2H), 7,18 (d, 1 H), 7,27 (dd, 1H), 7,46 (dd, 1 H), 7,62 (dt, 1 H), 8,39 (d, 1 H), 8,44 (d, 1 H), 8,48 (dd, 1 H)
- 3-198:: 2,38 (s, 3H), 3,47 (s, 2H), 4,70 (s, 2H), 5,42 (m, 2H), 7,07 (t, 2H)
- 3-203:: 2,39 (s, 3H), 3,68 (s, 2H), 4,66 (s, 2H), 5,40 (m, 2H), 7,26 (m, 3H), 7,69 (m, 2H), 8,40 (d, 1 H), 8,47 (dd, 1 H), 8,62 (m, 1 H)
- 3-207:: 3,58 (s, 2H), 4,68 (s, 2H), 5,41 (m, 2H), 7,84 (s, 1 H), 8,47 (dd, 1 H), 8,50 (d, 1 H)

**Tabelle 4: Verbindungen der Formel (III) (Zwischenprodukte)**

| | | | | | | |
|---|---|---|---|---|---|---|
| 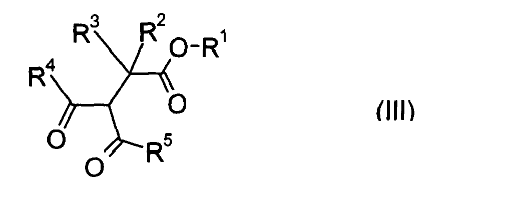 | | | | | | |

| Nr. | R¹ | R² | R³ | R⁴ | R⁵ | Daten |
|---|---|---|---|---|---|---|
| 4-1 | Me | H | H | Ph | Ph | |
| 4-2 | Me | H | H | Me | Ph | |
| 4-3 | Me | H | H | Me | 2-Furyl | |
| 4-4 | Me | Me | H | Me | Ph | |
| 4-5 | Me | H | H | Me | 4-MeO-Ph | |
| 4-6 | Me | H | H | Me | 4-Me-Ph | |
| 4-7 | Me | H | H | Me | 3-CF₃-Ph | |
| 4-8 | Me | H | H | Me | 3,4-Cl₂-Ph | |
| 4-9 | Me | H | H | Me | 3-Cl-Ph | |
| 4-10 | Me | H | H | Me | 2-Cl-Ph | |
| 4-11 | Me | H | H | Me | 2,4-Cl₂-Ph | |
| 4-12 | Me | H | H | Me | 4-CF₃-Ph | |
| 4-13 | Me | H | H | Me | 4-Cl-Ph | |
| 4-14 | Me | H | H | Me | 4-CF₃-Ph | |
| 4-15 | Me | H | H | Me | 4-tBu-Ph | |
| 4-16 | Me | H | H | Me | 3,5-Me₂-Ph | |
| 4-17 | Me | H | H | Me | 4-Me-Ph | |
| 4-18 | Me | H | H | Me | 4-F-Ph | |
| 4-19 | Me | H | H | Me | 3-Me-Ph | |
| 4-20 | Me | H | H | Me | 4-COOH-Ph | |
| 4-21 | Me | H | H | Me | 3-Br-Ph | |
| 4-22 | Me | H | H | Me | 4-Ph-Ph | |
| 4-23 | Me | H | H | Me | 3-Cl-4-Me-Ph | |
| 4-24 | Me | H | H | Me | 3- CF₃-4-chlor-Ph | |
| 4-25 | Me | H | H | Me | 2-Thienyl | |
| 4-26 | Me | H | H | Me | 3-Me-2-thienyl | |
| 4-27 | Me | H | H | Me | 4-Me-2-thienyl | |
| 4-28 | Me | H | H | Me | 5-Cl-2-thienyl | |
| 4-29 | Me | H | H | Me | 3-Thienyl | |
| 4-30 | Me | H | H | Me | 5-Me-2-thienyl | |
| 4-31 | Me | H | H | Me | 6-MeO-pyridin-3-yl | |
| 4-32 | Me | H | H | Me | 3-Thienyl | |
| 4-33 | Me | H | H | Me | 4-Br-Ph | |
| 4-34 | Me | H | H | Me | 1,3-Benzodioxol-5-yl | |
| 4-35 | Me | H | H | Me | 4-J-Ph | |
| 4-36 | Me | H | H | Me | 4-PhO-Ph | |
| 4-37 | Me | H | H | Me | 6-OH-pyridin-3-yl | |
| 4-38 | Me | H | H | H | Ph | |
| 4-39 | Me | H | H | Et | Ph | |
| 4-40 | Me | H | H | n-Pr | Ph | |
| 4-41 | Me | H | H | CH₂Cl | Ph | |
| 4-42 | Me | H | H | CHCl₂ | Ph | |
| 4-43 | Me | H | H | CH₂F | Ph | |
| 4-44 | Me | H | H | CHF₂ | Ph | |
| 4-45 | Me | H | H | Cl | Ph | |
| 4-46 | Me | H | H | n-Pr | 4-Cl-Ph | |
| 4-47 | Me | H | H | CH₂Cl | 4-Cl-Ph | |
| 4-48 | Me | H | H | CHCl₂ | 4-Cl-Ph | |
| 4-49 | Me | H | H | CH₂F | 4-Cl-Ph | |
| 4-50 | Me | H | H | CHF₂ | 4-Cl-Ph | |
| 4-51 | Me | H | H | Cl | 4-Cl-Ph | |
| 4-52 | Me | H | H | Et | 4-Me-Ph | |
| 4-53 | Me | H | H | n-Pr | 4-Me-Ph | |
| 4-54 | Me | H | H | CH₂Cl | 4-Me-Ph | |
| 4-55 | Me | H | H | CHCl₂ | 4-Me-Ph | |
| 4-56 | Me | H | H | CH₂F | 4-Me-Ph | |
| 4-57 | Me | H | H | CHF₂ | 4-Me-Ph | |
| 4-58 | Me | H | H | Cl | 4-Me-Ph | |
| 4-59 | Me | H | H | Et | 2-Pyridyl | |
| 4-60 | Me | H | H | n-Pr | 2-Pyridyl | |
| 4-61 | Me | H | H | CH₂Cl | 2-Pyridyl | |
| 4-62 | Me | H | H | CHCl₂ | 2-Pyridyl | |
| 4-63 | Me | H | H | CH₂F | 2-Pyridyl | |
| 4-64 | Me | H | H | CHF₂ | 2-Pyridyl | |
| 4-65 | Me | H | H | Cl | 2-Pyridyl | |
| 4-66 | Me | H | H | Me | 2-Pyridyl | |
| 4-67 | Me | H | H | Me | 5-Cl-pyridin-2-yl | |
| 4-68 | Me | H | H | Me | 5-Br-pyridin-2-yl | |
| 4-69 | Me | H | H | Me | 5-F-pyridin-2-yl | |
| 4-70 | Me | H | H | Me | 5-Me-pyridin-2-yl | |
| 4-71 | Me | H | H | Me | 2,4-Cl₂-Ph | |
| 4-72 | Me | H | H | Me | 4-CH₂COOH-Ph | |
| 4-73 | Me | H | H | Me | 4-Me(CO)-Ph | |
| 4-74 | Me | H | H | Me | 4-tBu-Ph | |
| 4-75 | Me | H | H | Me | 4-Cl-3-Me-Ph | |
| 4-76 | Me | H | H | n-Pr | 4-Cl-Ph | |
| 4-77 | Me | H | H | Me | 3-Pyridyl | |
| 4-78 | Me | H | H | Me | 4-Pyridyl | |
| 4-79 | Me | H | H | C(O)OMe | Ph | |
| 4-80 | Me | H | H | Me | 6-Me-pyridin-3-yl | |
| 4-81 | Me | H | H | Me | 2,3-Dichlor-Ph | |
| 4-82 | Me | H | H | H | 4-Cl-Ph | |
| 4-83 | Me | H | H | Me | 6-Cl-pyridin-3-yl | |
| 4-84 | Et | H | H | Ph | Ph | |
| 4-85 | Et | H | H | Me | Ph | |
| 4-86 | Et | Me | H | Me | Ph | |
| 4-87 | Et | H | H | Me | 4-MeO-Ph | |
| 4-88 | Et | H | H | Me | 4-Me-Ph | |
| 4-89 | Et | H | H | Me | 3-CF₃-Ph | |
| 4-90 | Et | H | H | Me | 3,4-Cl₂-Ph | |
| 4-91 | Et | H | H | Me | 3-Cl-Ph | |
| 4-92 | Et | H | H | Me | 2-Cl-Ph | |
| 4-93 | Et | H | H | Me | 2,4-Cl₂-Ph | |
| 4-94 | Et | H | H | Me | 4-CF₃-Ph | |
| 4-95 | Et | H | H | Me | 4-Cl-Ph | |
| 4-96 | Et | H | H | Me | 4-CF₃-Ph | |
| 4-97 | Et | H | H | Me | 4-tBu-Ph | |
| 4-98 | Et | H | H | Me | 3,5-Me₂-Ph | |
| 4-99 | Et | H | H | Me | 4-Me-Ph | |
| 4-100 | Et | H | H | Me | 4-F-Ph | |
| 4-101 | Et | H | H | Me | 3-Me-Ph | |
| 4-102 | Et | H | H | Me | 4-COOH-Ph | |
| 4-103 | Et | H | H | Me | 3-Br-Ph | |
| 4-104 | Et | H | H | Me | 4-Ph-Ph | |
| 4-105 | Et | H | H | Me | 3-Cl-4-Me-Ph | |
| 4-106 | Et | H | H | Me | 3- CF₃-4-chlor-Ph | |
| 4-107 | Et | H | H | Me | 2-Thienyl | |
| 4-108 | Et | H | H | Me | 3-Me-2-thienyl | |
| 4-109 | Et | H | H | Me | 4-Me-2-thienyl | |
| 4-110 | Et | H | H | Me | 5-Cl-2-thienyl | |
| 4-111 | Et | H | H | Me | 3-Thienyl | |
| 4-112 | Et | H | H | Me | 5-Me-2-thienyl | |
| 4-113 | Et | H | H | Me | 6-MeO-pyridin-3-yl | |
| 4-114 | Et | H | H | Me | 3-Thienyl | |
| 4-115 | Et | H | H | Me | 4-Br-Ph | |
| 4-116 | Et | H | H | Me | 1,3-Benzodioxol-5-yl | |
| 4-117 | Et | H | H | Me | 4-J-Ph | |
| 4-118 | Et | H | H | Me | 4-PhO-Ph | |
| 4-119 | Et | H | H | Me | 6-OH-pyridin-3-yl | |
| 4-120 | Et | H | H | H | Ph | |
| 4-121 | Et | H | H | Et | Ph | |
| 4-122 | Et | H | H | n-Pr | Ph | |
| 4-123 | Et | H | H | CH₂Cl | Ph | |
| 4-124 | Et | H | H | CHCl₂ | Ph | |
| 4-125 | Et | H | H | CH₂F | Ph | |
| 4-126 | Et | H | H | CHF₂ | Ph | |
| 4-127 | Et | H | H | Cl | Ph | |
| 4-128 | Et | H | H | n-Pr | 4-Cl-Ph | |
| 4-129 | Et | H | H | CH₂Cl | 4-Cl-Ph | |
| 4-130 | Et | H | H | CHCl₂ | 4-Cl-Ph | |
| 4-131 | Et | H | H | CH₂F | 4-Cl-Ph | |
| 4-132 | Et | H | H | CHF₂ | 4-Cl-Ph | |
| 4-133 | Et | H | H | Cl | 4-Cl-Ph | |
| 4-134 | Et | H | H | Et | 4-Me-Ph | |
| 4-135 | Et | H | H | n-Pr | 4-Me-Ph | |
| 4-136 | Et | H | H | CH₂Cl | 4-Me-Ph | |
| 4-137 | Et | H | H | CHCl₂ | 4-Me-Ph | |
| 4-138 | Et | H | H | CH₂F | 4-Me-Ph | |
| 4-139 | Et | H | H | CHF₂ | 4-Me-Ph | |
| 4-140 | Et | H | H | Cl | 4-Me-Ph | |
| 4-141 | Et | H | H | Et | 2-Pyridyl | |
| 4-142 | Et | H | H | n-Pr | 2-Pyridyl | |
| 4-143 | Et | H | H | CH₂Cl | 2-Pyridyl | |
| 4-144 | Et | H | H | CHCl₂ | 2-Pyridyl | |
| 4-145 | Et | H | H | CH₂F | 2-Pyridyl | |
| 4-146 | Et | H | H | CHF₂ | 2-Pyridyl | |
| 4-147 | Et | H | H | Cl | 2-Pyridyl | |
| 4-148 | Et | H | H | Me | 2-Pyridyl | |
| 4-149 | Et | H | H | Me | 5-Cl-pyridin-2-yl | |
| 4-150 | Et | H | H | Me | 5-Br-pyridin-2-yl | |
| 4-151 | Et | H | H | Me | 5-F-pyridin-2-yl | |
| 4-152 | Et | H | H | Me | 5-Me-pyridin-2-yl | |
| 4-153 | Et | H | H | Me | 2,4-Cl₂-Ph | |
| 4-154 | Et | H | H | Me | 4-CH₂COOH-Ph | |
| 4-155 | Et | H | H | Me | 4-Me(CO)-Ph | |
| 4-156 | Et | H | H | Me | 4-tBu-Ph | |
| 4-157 | Et | H | H | Me | 4-Cl-3-Me-Ph | |
| 4-158 | Et | H | H | n-Pr | 4-Cl-Ph | |
| 4-159 | Et | H | H | Me | 3-Pyridyl | |
| 4-160 | Et | H | H | Me | 4-Pyridyl | |
| 4-161 | Et | H | H | C(O)OMe | Ph | |
| 4-162 | Et | H | H | Me | 6-Me-pyridin-3-yl | |
| 4-163 | Et | H | H | Me | 2,3-Dichlor-Ph | |
| 4-164 | Et | H | H | H | 4-Cl-Ph | |
| 4-165 | Et | H | H | Me | 6-Cl-pyridin-3-yl | |

### (B) Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man

| 75 Gewichtsteile | einer Verbindung der Formel (I), |
|---|---|
| 10 | " ligninsulfonsaures Calcium, |
| 5 | " Natriumlaurylsulfat, |
| 3 | " Polyvinylalkohol und |
| 7 | " Kaolin |

mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man 25 Gewichtsteile einer Verbindung der Formel (I),

| | |
|---|---|
| 5 " | 2,2'-dinaphthylmethan-6,6'-disulfonsaures Natrium |
| 2 " | oleoylmethyltaurinsaures Natrium, |
| 1 Gewichtsteil | Polyvinylalkohol, |
| 17 Gewichtsteile | Calciumcarbonat und |
| 50 " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### (C) Biologische Beispiele

### 1. Unkrautwirkung im Vorauflauf

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspension bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. Auflaufschäden erfolgte nach dem Auflaufen der Versuchspflanzen nach einer Versuchszeit von 3 bis 4 Wochen im Vergleich zu unbehandelten Kontrollen. Wie die Testergebnisse zeigten, wiesen die erfindungsgemäßen Verbindungen eine gute herbizide Vorauflaufwirksamkeit gegen ein breites Spektrum von Ungräsern und Unkräutern auf. Beispielsweise zeigten die Beispiele Nr. 1-1, 1-2, 1-5, 1-7, 1-8, 1-9, 1-10, 1-11, 1-12, 1-13, 1-14, 1-16, 1-17, 1-18, 1-19, 1-20, 1-21, 1-22, 1-23, 1-24, 1-25, 1-26, 1-27, 1-28, 1-29, 1-30, 1-31, 1-32, 1-33, 1-34, 1-35, 1-36, 1-37, 1-38, 1-39, 1-40, 1-41, 1-42, 1-43, 1-45, 1-46, 1-47, 1-48, 1-49, 1-50, 1-51, 1-52, 1-53, 1-54, 1-55, 1-56, 1-57, 1-58, 1-59, 1-60, 1-61, 1-62, 1-63, 1-64, 1-70, 1-71, 1-72, 1-73, 1-120, 1-125, 2-1, 2-2, 2-5, 2-6, 2-7, 2-8, 2-9, 2-10, 2-11, 2-12, 2-13, 2-14, 2-16, 2-17, 2-18, 2-19, 2-20, 2-21, 2-22, 2-23, 2-24, 2-25, 2-26, 2-27, 2-28, 2-29, 2-30, 2-31, 2-32, 2-33, 2-35, 2-36, 2-37, 2-38, 2-40, 2-41, 2-43, 2-44, 2-45, 2-46, 2-47, 2-48, 2-49, 2-50, 2-51, 2-52, 2-53, 2-54, 2-55, 2-56, 2-57, 2-58, 2-59, 2-60, 2-61, 2-62, 2-63, 2-64, 2-66, 2-68, 2-70, 2-71, 2-72, 2-73, 2-74, 2-79, 2-89, 2-109, 2-114, 2-115, 2-116, 2-119, 2-120, 2-121, 2-122, 2-123, 2-124, 2-125, 2-126, 2-127, 2-128, 2-131, 2-133, 2-134, 3-1, 3-36, 3-50, 3-54, 3-55, 3-63, 3-64, 3-68, 3-73, 3-74, 3-75, 3-76, 3-77, 3-78, 3-85, 3-86, 3-87, 3-91, 3-98, 3-99, 3-100, 3-101, 3-105, 3-109, 3-113, 3-117, 3-118, 3-136, 3-139, 3-140, 3-144, 3-148, 3-152, 3-156, 3-160, 3-164, 3-168, 3-172, 3-177, 3-180, 3-184, 3-186, 3-187, 3-188, 3-189, 3-190, 3-191, 3-193, 3-195, 3-198, 3-203, 3-207 aus den Tabellen 1 bis 3 im Test sehr gute herbizide Wirkung gegen Schadpflanzen wie Lolium multiflorum, Amaranthus retroflexus, Sinapis alba, Stellaria media und Setaria viridis im Vorauflaufverfahren bei einer Aufwandmenge von 1 kg oder weniger Aktivsubstanz pro Hektar. Teilweise zeigten die Beispielverbindungen dabei auch sehr gute Wirkung bei Schadpflanzen wie Avena sativa und Capsella bursa-pastoris.

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt. Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 I/ha auf die grünen Pflanzenteile gesprüht. Nach ca. 3 bis 4 Wochen Standzeit der Versuchspflanzen im Gewächshaus unter optimalen Wachstumsbedingungen wurde die Wirkung der Präparate optisch im Vergleich zu unbehandelten Kontrollen bonitiert. Die erfindungsgemäßen Mittel wiesen auch im Nachauflauf eine gute herbizide Wirksamkeit gegen ein breites Spektrum wirtschaftlich wichtiger Ungräser und Unkräuter auf. Beispielsweise zeigten die Beispiele Nr. 1-1, 1-2, 1-5, 1-7, 1-8, 1-9, 1-10, 1-11, 1-12, 1-13, 1-14, 1-16, 1-17, 1-18, 1-19, 1-20, 1-21, 1-22, 1-23, 1-24, 1-25, 1-26, 1-27, 1-28, 1-29, 1-30, 1-31, 1-32, 1-33, 1-34, 1-35, 1-36, 1-37, 1-38, 1-39, 1-40, 1-41, 1-42, 1-43, 1-45, 1-46, 1-47, 1-48, 1-49, 1-50, 1-51, 1-52, 1-53, 1-54, 1-55, 1-56, 1-57, 1-58, 1-59, 1-60, 1-61, 1-62, 1-63, 1-64, 1-70, 1-71, 1-72, 1-73, 1-120, 1-125, 2-1, 2-2, 2-5, 2-6, 2-7, 2-8, 2-9, 2-10, 2-11, 2-12, 2-13, 2-14, 2-16, 2-17, 2-18, 2-19, 2-20, 2-21, 2-22, 2-23, 2-24, 2-25, 2-26, 2-27, 2-28, 2-29, 2-30, 2-31, 2-32, 2-33, 2-35, 2-36, 2-37, 2-38, 2-40, 2-41, 2-43, 2-44, 2-45, 2-46, 2-47, 2-48, 2-49, 2-50, 2-51, 2-52, 2-53, 2-54, 2-55, 2-56, 2-57, 2-58, 2-59, 2-60, 2-61, 2-62, 2-63, 2-64, 2-66, 2-68, 2-70, 2-71, 2-72, 2-73, 2-74, 2-79, 2-89, 2-109, 2-114, 2-115, 2-116, 2-119, 2-120, 2-121, 2-122, 2-123, 2-124, 2-125, 2-126, 2-127, 2-128, 2-131, 2-133, 2-134, 3-1, 3-36, 3-50, 3-54, 3-55, 3-63, 3-64, 3-68, 3-73, 3-74, 3-75, 3-76, 3-77, 3-78, 3-85, 3-86, 3-87, 3-91, 3-98, 3-99, 3-100, 3-101, 3-105, 3-109, 3-113, 3-117, 3-118, 3-136, 3-139, 3-140, 3-144, 3-148, 3-152, 3-156, 3-160, 3-164, 3-168, 3-172, 3-177, 3-180, 3-184, 3-186, 3-187, 3-188, 3-189, 3-190, 3-191, 3-193, 3-195, 3-198, 3-203, 3-207 aus den Tabellen 1 bis 3 sehr gute herbizide Wirkung gegen Schadpflanzen wie Lolium multiflorum, Amaranthus retroflexus, Sinapis alba, Stellaria media und Setaria viridis im Nachauflaufverfahren bei einer Aufwandmenge von 500 g und weniger Aktivsubstanz pro Hektar. Teilweise zeigten die Beispielverbindungen dabei auch sehr gute Wirkung bei Schadpflanzen wie Avena sativa und Capsella bursa-pastoris.

### 3. Unkrautwirkung in Reis

Verpflanzter und gesäter Reis sowie typische Reisunkräuter und -ungräser wurden im Gewächshaus bis zum Dreiblattstadium (Echinochloa crus-galli 1,5-Blatt) unter Paddyreis-Bedingungen (Anstauhöhe des Wassers: 2 bis 3 cm) in geschlossenen Plastiktöpfen angezogen. Danach erfolgte die Behandlung mit den erfindungsgemäßen Verbindungen. Hierzu wurden die formulierten Wirkstoffe in Wasser suspendiert, gelöst bzw. emulgiert und mittels Gießapplikation in das Anstauwasser der Testpflanzen in unterschiedlichen Dosierungen ausgebracht. Nach der so durchgeführten Behandlung wurden die Versuchspflanzen im Gewächshaus unter optimalen Wachstumsbedingungen aufgestellt und während der gesamten Versuchszeit so gehalten.

Etwa drei Wochen nach der Applikation erfolgte die Auswertung mittels optischer Bonitur der Pflanzenschäden im Vergleich zu unbehandelten Kontrollen. Beispielsweise zeigten die erfindungsgemäßen Verbindungen Nr. 1-1, 1-2, 1-5, 1-7, 1-8, 1-9, 1-10, 1-11, 1-12, 1-13, 1-14, 1-16, 1-17, 1-18, 1-19, 1-20, 1-21, 1-22, 1-23, 1-24, 1-25, 1-26, 1-27, 1-28, 1-29, 1-30, 1-31, 1-32, 1-33, 1-34, 1-35, 1-36, 1-37, 1-38, 1-39, 1-40, 1-41, 1-42, 1-43, 1-45, 1-46, 1-47, 1-48, 1-49, 1-50, 1-51, 1-52, 1-53, 1-54, 1-55, 1-56, 1-57, 1-58, 1-59, 1-60, 1-61, 1-62, 1-63, 1-64, 1-70, 1-71, 1-72, 1-73, 1-120, 1-125, 2-1, 2-2, 2-5, 2-6, 2-7, 2-8, 2-9, 2-10, 2-11, 2-12, 2-13, 2-14, 2-16, 2-17, 2-18, 2-19, 2-20, 2-21, 2-22, 2-23, 2-24, 2-25, 2-26, 2-27, 2-28, 2-29, 2-30, 2-31, 2-32, 2-33, 2-35, 2-36, 2-37, 2-38, 2-40, 2-41, 2-43, 2-44, 2-45, 2-46, 2-47, 2-48, 2-49, 2-50, 2-51, 2-52, 2-53, 2-54, 2-55, 2-56, 2-57, 2-58, 2-59, 2-60, 2-61, 2-62, 2-63, 2-64, 2-66, 2-68, 2-70, 2-71, 2-72, 2-73, 2-74, 2-79, 2-89, 2-109, 2-114, 2-115, 2-116, 2-119, 2-120, 2-121, 2-122, 2-123, 2-124, 2-125, 2-126, 2-127, 2-128, 2-131, 2-133, 2-134, 3-1, 3-36, 3-50, 3-54, 3-55, 3-63, 3-64, 3-68, 3-73, 3-74, 3-75, 3-76, 3-77, 3-78, 3-85, 3-86, 3-87, 3-91, 3-98, 3-99, 3-100, 3-101, 3-105, 3-109, 3-113, 3-117, 3-118, 3-136, 3-139, 3-140, 3-144, 3-148, 3-152, 3-156, 3-160, 3-164, 3-168, 3-172, 3-177, 3-180, 3-184, 3-186, 3-187, 3-188, 3-189, 3-190, 3-191, 3-193, 3-195, 3-198, 3-203, 3-207 aus den Tabellen 1 bis 3 sehr gute herbizide Wirkung gegen typische Schadpflanzen in Reis auf, wie z.B. Cyperus iria und Echinochloa crus-galli.

### 4. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt. Ein Teil der Töpfe wurde sofort wie unter Abschnitt 1 beschrieben behandelt, die übrigen im Gewächshaus aufgestellt, bis die Pflanzen zwei bis drei echte Blätter entwickelt hatten und dann wie unter Abschnitt 2 beschrieben mit den erfindungsgemäßen Substanzen der Formel (I) in unterschiedlichen Dosierungen besprüht. Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt ließen. In der Regel schonten die Verbindungen bei geeigneten Aufwandmengen auch Gramineen-Kulturen wie z.B. Gerste, Weizen, Roggen, Sorghum, Mais oder Reis. Die Verbindungen der Formel (I) zeigten teilweise eine hohe Selektivität und eignen sich deshalb zur Bekämpfung von unerwünschten Pflanzenwuchs in landwirtschaftlichen Kulturen.

### 5. Versuch zur Pflanzenwachstumsregulation (Halmverkürzung bei Kulturpflanzen)

Die jeweilige Verbindung der Formel (I) wurden in Form wasserdispergierbare Pulver zusammen mit einem oberflächenaktiver Stoff (übliches Adjuvant mit einer Aufwandmenge von 1 I/ha) unter Rühren mit Wasser vermischt, so dass eine homogene Spritzbrühe entstand, und mit einer Wasseraufwandmenge von umgerechnet 300 I/ha verwendet.

Sommerweizensamen der Sorte ,Triso' wurden in einer Tiefe von 1 cm in Pfilanzentöpfen ausgesät und im Freiland bis zur Behandlung kultiviert.

Die Pflanzen wurden auf einer Laborspritzbahn mit Spritzbrühen der Testsubstanzen im Makrostadium 3 nach Erreichen von etwa 10 % des arttypischen maximalen Längenwachstums (Stadium BBCH 31) behandelt und nach der Behandlung wieder im Freiland aufgestellt.

Nach der Ährenausbildung wurden die Halmlängen gemessen. Die Halmverkürzung wurde prozentual zur unbehandelten Kontrolle bewertet.

Die Auswertungen ergaben, dass beispielsweise die erfindungsgemäßen Verbindungen (I) Nr. 1-1, 1-2, 1-5, 1-7, 1-8, 1-9, 1-10, 1-11, 1-12, 1-13, 1-14, 1-16, 1-17, 1-18, 1-19, 1-20, 1-21, 1-22, 1-23, 1-24, 1-25, 1-26, 1-27, 1-28, 1-29, 1-30, 1-31, 1-32, 1-33, 1-34, 1-35, 1-36, 1-37, 1-38, 1-39, 1-40, 1-41, 1-42, 1-43, 1-45, 1-46, 1-47, 1-48, 1-49, 1-50, 1-51, 1-52, 1-53, 1-54, 1-55, 1-56, 1-57, 1-58, 1-59, 1-60, 1-61, 1-62, 1-63, 1-64, 1-70, 1-71, 1-72, 1-73, 1-120, 1-125, 2-1, 2-2, 2-5, 2-6, 2-7, 2-8, 2-9, 2-10, 2-11, 2-12, 2-13, 2-14, 2-16, 2-17, 2-18, 2-19, 2-20, 2-21, 2-22, 2-23, 2-24, 2-25, 2-26, 2-27, 2-28, 2-29, 2-30, 2-31, 2-32, 2-33, 2-35, 2-36, 2-37, 2-38, 2-40, 2-41, 2-43, 2-44, 2-45, 2-46, 2-47, 2-48, 2-49, 2-50, 2-51, 2-52, 2-53, 2-54, 2-55, 2-56, 2-57, 2-58, 2-59, 2-60, 2-61, 2-62, 2-63, 2-64, 2-66, 2-68, 2-70, 2-71, 2-72, 2-73, 2-74, 2-79, 2-89, 2-109, 2-114, 2-115, 2-116, 2-119, 2-120, 2-121, 2-122, 2-123, 2-124, 2-125, 2-126, 2-127, 2-128, 2-131, 2-133, 2-134, 3-1, 3-36, 3-50, 3-54, 3-55, 3-63, 3-64, 3-68, 3-73, 3-74, 3-75, 3-76, 3-77, 3-78, 3-85, 3-86, 3-87, 3-91, 3-98, 3-99, 3-100, 3-101, 3-105, 3-109, 3-113, 3-117, 3-118, 3-136, 3-139, 3-140, 3-144, 3-148, 3-152, 3-156, 3-160, 3-164, 3-168, 3-172, 3-177, 3-180, 3-184, 3-186, 3-187, 3-188, 3-189, 3-190, 3-191, 3-193, 3-195, 3-198, 3-203, 3-207 aus den Tabellen 1 bis 3 bei einer Aufwandmenge von 30 bis 300 g Wirkstoff per Hektar das Längenwachstum des Sommerweizens wirksam verkürzt hatten.

## Patentansprüche

1. Verwendung von Verbindungen der Formel (I) oder deren Salzen, 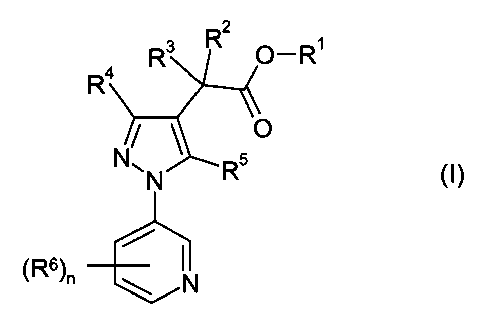 in welcher
R¹ Wasserstoff oder einen hydrolysierbaren Rest,
R² Wasserstoff, Halogen oder (C₁-C₆)Alkyl, das unsubstituiert oder durch einen oder mehrere Reste aus der Gruppe, welche aus Halogen, (C₁-C₄)Alkoxy, (C₁-C₄)Alkylthio und (C₁-C₄)Haloalkoxy besteht, substituiert ist,
R³ Wasserstoff, Halogen oder (C₁-C₆)Alkyl, das unsubstituiert oder durch einen oder mehrere Reste aus der Gruppe, welche aus Halogen, (C₁-C₄)Alkoxy, (C₁-C₄)Alkylthio und (C₁-C₄)Haloalkoxy besteht, substituiert ist, oder
R² und R³ zusammen mit dem C-Atom, an das sie gebunden sind, einen carbocyclischen gesättigten oder teilweise ungesättigten Ring mit 3 bis 6 C-Atomen, der unsubstituiert oder durch einen oder mehrere Reste aus der Gruppe Halogen und (C₁-C₄)Alkyl substituiert ist, und
R⁴ Wasserstoff, Halogen, Cyano, (C₁-C₆)Alkyl, (C₂-C₆)Alkenyl oder (C₂-C₆)Alkinyl, wobei jeder der letztgenannten drei Reste unsubstituiert oder durch einen oder mehrere Reste aus der Gruppe bestehend aus Halogen, Cyano, (C₁-C₄)Alkoxy, (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₉)Cycloalkyl substituiert ist, oder
(C₃-C₉)Cycloalkyl, (C₅-C₉)Cycloalkenyl oder (C₅-C₉)Cycloalkinyl, wobei jeder der 3 letztgenannten Reste unsubstituiert oder durch einen oder mehrere Reste aus der Gruppe bestehend aus Halogen, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy und (C₁-C₄)Alkylthio substituiert ist, oder
Phenyl, das unsubstituiert oder durch einen oder mehrere Reste aus der Gruppe bestehend aus Halogen, Cyano, Nitro, Carboxy, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy, (C₁-C₄)Alkoxy-(C₁-C₄)alkoxy, (C₁-C₄)Alkylthio, (C₁-C₄)Alkanoyl, (C₁-C₄)Haloalkanoyl, [(C₁-C₄)Alkoxy]-carbonyl und [(C₁-C₄)Haloalkoxy]-carbonyl substituiert ist, oder
(C₁-C₆)Alkanoyl, das unsubstituiert oder durch einen oder mehrere Reste aus der Gruppe bestehend aus Halogen, Cyano, (C₁-C₄)Alkoxy, (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₆)Cycloalkyl substituiert ist, oder
[(C₁-C₄)Alkoxy]-carbonyl, unsubstituiert oder durch einen oder mehrere Reste aus der Gruppe bestehend aus Halogen, Cyano, (C₁-C₄)Alkoxy, (C₁-C₄)Haloalkoxy, (C₁-C₄)Alkoxy-(C₁-C₄)alkoxy, (C₁-C₄)Alkylthio und gegebenenfalls durch halogen-, cyano-, (C₁-C₄)alkyl- oder (C₁-C₄)haloalkylsubstituiertes (C₃-C₆)Cycloalkyl substituiert ist, oder
[(C₃-C₉)Cycloalkoxy]-carbonyl, das unsubstituiert oder durch einen oder mehrere Reste aus der Gruppe bestehend aus Halogen, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy und (C₁-C₄)Alkylthio substituiert ist,
R⁵ einen Arylrest, der unsubstituiert oder substituiert ist und inklusive Substituenten 6 bis 30 C-Atome aufweist, oder
einen heteroaromatischen Rest mit 1 bis 4 Heteroringatomen aus der Gruppe N, O und S, der unsubstituiert oder substituiert ist und inklusive Substituenten 1 bis 30 C-Atome aufweist, und
(R⁶)ₙ n Substituenten R⁶, wobei R⁶, im Falle dass n = 1 ist, oder jeder der Substituenten R⁶ unabhängig voneinander, im Falle dass n größer als 1 ist, einen Rest Halogen, Hydroxy, Amino, Nitro, Carboxy, Cyano, Carbamoyl, (C₁-C₆)Alkyl, (C₁-C₆)Haloalkyl, (C₁-C₄)Alkoxy-(C₁-C₄)alkyl, (C₁-C₄)Alkylthio-(C₁-C₄)alkyl, Mono- oder Di-[(C₁-C₄)alkyl]-aminoalkyl, Hydroxy-(C₁-C₄)alkyl, (C₂-C₆)Alkenyl, (C₂-C₆)Haloalkenyl, (C₂-C₆)Alkinyl, (C₂-C₆)Haloalkinyl, (C₁-C₆)Alkoxy, (C₁-C₆)Haloalkoxy, (C₁-C₄)Alkoxy-(C₁-C₄)alkoxy, (C₁-C₆)Alkylthio, [(C₁-C₆)Alkoxy]-carbonyl, [(C₁-C₆)Haloalkoxy]-carbonyl, (C₁-C₆)Alkanoyl, (C₁-C₆)Haloalkanoyl, Mono- oder Di-[(C₁-C₄)alkyl]-aminocarbonyl, Mono- oder Di-[(C₁-C₆)acyl]-amino, Mono- oder Di-[(C₁-C₄)alkyl]-amino, N-[(C₁-C₆)Acyl]-N-[(C₁-C₆)alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, (C₃-C₉)Cycloalkyl oder (C₅-C₉)Cycloalkenyl, wobei jeder der letztgenannten beiden Reste unsubstituiert oder durch einen oder mehrere Reste aus der Gruppe bestehend aus Halogen, (C₁-C₄)Alkyl und (C₁-C₄)Haloalkyl substituiert ist, bedeutet, und
n 0, 1, 2, 3 oder 4
bedeuten,
als Herbizide oder Pflanzenwachstumsregulatoren.

2. Verwendung nach Anspruch 1, **dadurch gekennzeichnet, dass**
R¹ Wasserstoff, Alkyl, Alkenyl, Alkinyl, Cycloalkyl, Cycloalkenyl, Cycloalkinyl oder Aryl, wobei jeder der letztgenannten 7 Reste unsubstituiert oder substituiert ist und inklusive Substituenten bis zu 30 C-Atome
oder
einen Heterocyclylrest mit 3 bis 9 Ringatomen, der 1 bis 4 Heteroatome aus der Gruppe N, O und S enthält und der unsubstituiert oder substituiert ist und der inklusive Substituenten 1 bis 30 C-Atome aufweist,
oder
einen Rest der Formel SiR^{a}R^{b}R^{c}, -NR^{a}R^{b} oder -N=CR^{c}R^{d},
wobei in den letztgenannten 3 Formel jeder der Reste R^{a}, R^{b}, R^{c} und R^{d} unabhängig voneinander Wasserstoff, (C₁-C₄)Alkyl, (C₂-C₄)Alkenyl, (C₂-C₄)Alkinyl, Benzyl, substituiertes Benzyl, Phenyl oder substituiertes Phenyl bedeutet oder R^{a} und R^{b} zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl und (C₁-C₄)Haloalkyl substituiert ist, bedeuten oder R^{c} und R^{d} zusammen mit dem C-Atom einen 3- bis 8-gliedrigen carbocyclischen Rest oder heterocyclischen Rest, welcher 1 bis 3 Heteroringatome aus der Gruppe N, O und S enthalten kann, wobei der carbocyclische oder heterocyclischen Rest unsubstituiert oder durch einen oder mehrere Reste aus der Gruppe (C₁-C₄)Alkyl und (C₁-C₄)Haloalkyl substituiert ist, bedeuten, und
R² Wasserstoff, Halogen oder (C₁-C₄)Alkyl, das unsubstituiert oder durch einen oder mehrere Reste aus der Gruppe Halogen, wie Fluor oder Chlor substituiert ist, und
R³ Wasserstoff, Halogen oder (C₁-C₄)Alkyl, das unsubstituiert oder durch einen oder mehrere Reste aus der Gruppe Halogen, wie Fluor oder Chlor substituiert ist, oder
R² und R³ zusammen mit dem C-Atom, an das sie gebunden sind, (C₃-C₆)Cycloalkyl oder (C₅-C₆)Cycloalkenyl, wobei jeder der letztgenannten 2 Reste unsubstituiert oder durch einen oder mehrere Reste aus der Gruppe Halogen und (C₁-C₄)Alkyl substituiert ist, und
R⁴ Wasserstoff, Halogen, Cyano, (C₁-C₄)Alkyl, (C₂-C₄)Alkenyl oder (C₂-C₄)Alkinyl,
wobei jeder der letztgenannten drei Reste unsubstituiert oder durch einen oder mehrere Reste aus der Gruppe bestehend aus Halogen, wie Fluor und Chlor substituiert ist, oder
(C₃-C₆)Cycloalkyl, das unsubstituiert oder durch einen oder mehrere Reste aus der Gruppe bestehend aus Halogen und (C₁-C₄)Alkyl substituiert ist, oder
Phenyl, das unsubstituiert oder durch einen oder mehrere Reste aus der Gruppe bestehend aus Halogen, Nitro, (C₁-C₄)Alkyl, (C₁-C₄)Haloalkyl, (C₁-C₄)Alkoxy, (C₁-C₄)Haloalkoxy, (C₁-C₄)Alkoxy-(C₁-C₄)alkoxy, (C₁-C₄)Alkylthio, [(C₁-C₄)Alkoxy]-carbonyl und [(C₁-C₄)Haloalkoxy]-carbonyl substituiert ist, oder
(C₁-C₄)Alkanoyl, das unsubstituiert oder durch einen oder mehrere Reste aus der Gruppe bestehend aus Halogen, Cyano, (C₁-C₄)Alkoxy, (C₁-C₄)Haloalkoxy und (C₁-C₂)Alkoxy-(C₁-C₂)alkoxy substituiert ist, oder
[(C₁-C₄)Alkoxy]-carbonyl, das unsubstituiert oder durch einen oder mehrere Reste aus der Gruppe bestehend aus Halogen, wie Fluor und Chlor, substituiert ist, oder
[(C₃-C₆)Cycloalkoxy]-carbonyl, das unsubstituiert oder durch einen oder mehrere Reste aus der Gruppe bestehend aus Halogen und (C₁-C₄)Alkyl substituiert ist, und
R⁵ Phenyl,
das unsubstituiert oder durch einen oder mehrere Reste aus der Gruppe bestehend aus Halogen, Hydroxy, Amino, Nitro, Carboxy, Cyano, Carbamoyl, (C₁-C₆)Alkyl, (C₁-C₆)Haloalkyl, (C₁-C₄)Alkoxy-(C₁-C₄)alkyl, (C₁-C₄)Alkylthio-(C₁-C₄)alkyl, Mono- und Di-[(C₁-C₄)alkyl]-aminoalkyl, Hydroxy-(C₁-C₄)alkyl, Carboxy-(C₁-C₄)alkyl, Cyano-(C₁-C₄)alkyl, (C₁-C₆)Alkoxy, (C₁-C₄)Alkoxy-(C₁-C₄)alkoxy, (C₁-C₆)Alkylthio, [(C₁-C₆)Alkoxy]-carbonyl, [(C₁-C₆)Haloalkoxy]-carbonyl, (C₁-C₆)Alkanoyl, (C₁-C₆)Haloalkanoyl, Mono- und Di-[(C₁-C₄)alkyl]-aminocarbonyl, Mono- und Di-[(C₁-C₆)Acyl]-amino, Mono- und Di-[(C₁-C₄)alkyl]-amino, N-[(C₁-C₆)Acyl]-N-[(C₁-C₆)Alkyl]-amino, (C₁-C₆)Alkylsulfinyl, (C₁-C₆)Haloalkyfsulfinyl, (C₁-C₆)Alkylsulfonyl, (C₁-C₆)Haloalkylsulfonyl, (C₃-C₆)Cycloalkyl, (C₃-C₆)Cycloalkyloxy, Phenyl und Phenoxy,
wobei jeder der letztgenannten 4 Reste unsubstituiert oder durch einen oder mehrere Reste aus der Gruppe bestehend aus Halogen, (C₁-C₄)Alkyl und (C₁-C₄)Haloalkyl substituiert ist,
substituiert ist und wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen und (C₁-C₆)Alkyl substituiert ist,
oder
einen 5- oder 6-gliedrigen heteroaromatischen Rest mit 1 bis 3 Heteroringatomen aus der Gruppe N, O und S,
der unsubstituiert oder durch einen oder mehrere Reste aus der Gruppe bestehend aus Halogen, Hydroxy, Amino, Nitro, Carboxy, Cyano, Carbamoyl, (C₁-C₆)Alkyl, (C₁-C₆)Haloalkyl, (C₁-C₄)Alkoxy-(C₁-C₄)alkyl, (C₁-C₄)Alkylthio-(C₁-C₄)alkyl, Mono- und Di-[(C₁-C₄)alkyl]-aminoalkyl, Hydroxy-(C₁-C₄)alkyl, Carboxy-(C₁-C₄)alkyl, Cyano-(C₁-C₄)alkyl, (C₁-C₆)Alkoxy, (C₁-C₄)Alkoxy-(C₁-C₄)alkoxy, (C₁-C₆)Alkylthio, [(C₁-C₆)Alkoxy]-carbonyl, [(C₁-C₆)Haloalkoxy]-carbonyl, (C₁-C₆)Alkanoyl, (C₁-C₆)Haloalkanoyl, Mono- und Di-[(C₁-C₄)alkyl]-aminocarbonyl, Mono- und Di-[(C₁-C₆)Acyl]-amino, Mono- und Di-[(C₁-C₄)alkyl]-amino, N-[(C₁-C₆)Acyl]-N-[(C₁-C₆)Alkyl]-amino, (C₁-C₆)Alkylsulfinyl, (C₁-C₆)Haloalkylsulfinyl, (C₁-C₆)Alkylsulfonyl, (C₁-C₆)Haloalkylsulfonyl, (C₃-C₆)Cycloalkyl, (C₃-C₆)Cycloalkyloxy, Phenyl und Phenoxy,
wobei jeder der letztgenannten 4 Reste unsubstituiert oder durch einen oder mehrere Reste aus der Gruppe bestehend aus Halogen, (C₁-C₄)Alkyl und (C₁-C₄)Haloalkyl substituiert ist, substituiert ist, und
(R⁶) ₙ Substituenten R⁶, wobei R⁶, im Falle dass n = 1 ist, oder jeder der Substituenten R⁶ unabhängig voneinander, im Falle dass n größer als 1 ist, einen Rest Halogen, (C₁-C₄)Alkyl, (C₁-C₄)Haloalkyl, (C₁-C₄)Alkoxy, (C₁-C₄)Alkylthio, (C₁-C₄)Alkylsulfinyl, (C₁-C₄)Haloalkylsulfinyl, (C₁-C₄)Alkylsulfonyl oder (C₁-C₄)Haloalkylsulfonyl bedeutet, und
n 0, 1, 2, 3 oder 4,
bedeuten.

3. Verwendung nach Anspruch 1 oder 2, **dadurch gekennzeichnet, dass**
R¹ H, (C₁-C₁₈)Alkyl, (C₂-C₁₈)Alkenyl, (C₂-C₁₈)Alkinyl, (C₃-C₉)Cycloalkyl, (C₅-C₉)Cycloalkenyl, (C₅-C₉)Cycloalkinyl, Phenyl,
wobei jeder der letztgenannten 7 Reste unsubstituiert oder durch einen oder mehrere Reste aus der Gruppe Halogen, Cyano, Thio, Nitro, Hydroxy, (C₁-C₈)Alkyl, (C₁-C₈)Haloalkyl, (C₁-C₄)Alkoxy-(C₁-C₄)alkyl, (C₂-C₈)Alkenyl, (C₂-C₈)Haloalkenyl, (C₂-C₈)Alkinyl, (C₂-C₈)Haloalkinyl, die letztgenannten 7 Reste nur im Falle cyclischer Basisreste, (C₁-C₈)Alkoxy, (C₂-C₈)Alkenyloxy, (C₂-C₈)Alkinyloxy, (C₁-C₈)Haloalkoxy, (C₁-C₄)Alkoxy-(C₁-C₄)alkoxy, (C₁-C₈)Alkylthio, (C₂-C₈)Alkenylthio, (C₂-C₈)Alkinylthio, Reste der Formeln -NR*R**, -CO-NR*R** und -O-CO-NR*R**,
wobei jeder der Reste R* und R** in den letztgenannten 3 Formeln unabhängig voneinander H, (C₁-C₈)Alkyl, (C₂-C₈)Alkenyl, (C₂-C₈)Alkinyl, Benzyl, substituiertes Benzyl, Phenyl oder substituiertes Phenyl, oder zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl und (C₁-C₄)Haloalkyl substituiert ist, bedeutet,
und [(C₁-C₈)Alkoxy]-carbonyl, [(C₁-C₈)Alkoxy]-thiocarbonyl, [(C₂-C₈)Alkenyloxy]-carbonyl, [(C₂-C₈)Alkinyloxy]-carbonyl, [(C₁-C₈)Alkylthio]-carbonyl, [(C₂-C₈)Alkenylthio]-carbonyl, [(C₂-C₈)Alkinylthio]-carbonyl, (C₁-C₈)Alkanoyl, [(C₂-C₈)Alkenyl]-carbonyl, [(C₂-C₈)Alkinyl]-carbonyl, (C₁-C₄)Alkylimino, (C₁-C₄)Alkoxyimino, [(C₁-C₈)Alkyl]-carbonylamino, [(C₂-C₈)Alkenyl]-carbonylamino, [(C₂-C₈)Alkinyl]-carbonylamino, [(C₁-C₈)Alkoxy]-carbonylamino, [(C₂-C₈)Alkenyloxy]-carbonylamino, [(C₂-C₈)Alkinyloxy]-carbonylamino, [(C₁-C₈)Alkylamino]-carbonylamino, [(C₁-C₆)Alkyl]-carbonyloxy, [(C₂-C₆)Alkenyl]-carbonyloxy, [(C₂-C₆)Alkinyl]-carbonyloxy, [(C₁-C₈)Alkoxy]-carbonyloxy, [(C₂-C₈)Alkenyloxy]-carbonyloxy, [(C₂-C₈)Alkinyloxy]-carbonyloxy, (C₁-C₈)Alkylsulfinyl und (C₁-C₈)Alkylsulfonyl,
wobei jeder der letztgenannten 27 Reste unsubstituiert oder durch einen oder mehrere Reste aus der Gruppe Halogen, NO₂, (C₁-C₄)Alkoxy und gegebenenfalls substituiertes Phenyl substituiert ist,
und Phenyl, Phenyl-(C₁-C₆)alkoxy, Phenyl-[(C₁-C₆)alkoxy]-carbonyl, Phenoxy, Phenoxy-(C₁-C₆)alkoxy, Phenoxy-[(C₁-C₆)alkoxy]-carbonyl, Phenoxycarbonyl, Phenylcarbonyloxy, Phenylcarbonylamino, Phenyl-[(C₁-C₆)alkyl]-carbonylamino, Phenyl-[(C₁-C₆)alkyl]-carbonyloxy, (C₃-C₇)Cycloalkyl und (C₃-C₇)Cycloalkoxy,
wobei jeder der letztgenannten 13 Reste im Ring unsubstituiert oder durch einen oder mehrere Reste aus der Gruppe Halogen, (C₁-C₄)Alkyl, (C₁-C₄)Alkoxy, (C₁-C₄)Haloalkyl, (C₁-C₄)Haloalkoxy und Nitro substituiert ist,
und Reste der Formeln -SiR'₃, -O-SiR'₃, (R')₃Si-(C₁-C₆)alkoxy, -CO-O-NR'₂, -O-N=CR'₂, -N=CR'₂, -O-NR'₂, -CH(OR')₂ und -O-(CH₂)ₘ-CH(OR')₂,
in welchen jeder der Reste R' unabhängig voneinander H, (C₁-C₄)Alkyl oder Phenyl, welches unsubstituiert oder durch einen oder mehrere Reste aus der Gruppe bestehend aus Halogen, (C₁-C₄)Alkyl, (C₁-C₄)Alkoxy, (C₁-C₄)Haloalkyl, (C₁-C₄)Haloalkoxy und Nitro substituiert ist oder an zwei benachbarten Positionen durch eine (C₂-C₆)Alkylen-Brücke substituiert ist, und m eine ganze Zahl von 0 bis 6 bedeuten,
und Reste der Formel R"O-CHR"'CH(OR")-(C₁-C₆)alkoxy, in welcher jeder der Reste R" unabhängig voneinander H oder (C₁-C₄)Alkyl oder gemeinsam eine (C₁-C₆)Alkylengruppe bedeuten und R'" H oder (C₁-C₄)Alkyl bedeuten,
substituiert ist,
bedeutet.

4. Verwendung nach einem der Ansprüche 1 bis 3, **dadurch gekennzeichnet, dass**
R² Wasserstoff, Methyl oder Ethyl,
R³ Wasserstoff oder Methyl,
R⁴ Wasserstoff, Halogen, (C₁-C₄)Alkyl, (C₁-C₄)Haloalkyl, Cyclopropyl oder Cyclobutyl, wobei jeder der letztgenannten beiden Reste unsubstituiert oder durch einen oder mehrere Reste aus der Gruppe bestehend aus Halogen, wie Fluor und Chlor, und (C₁-C₄)Alkyl substituiert ist, oder
Phenyl, das unsubstituiert oder durch einen oder mehrere Reste aus der Gruppe bestehend aus Halogen, (C₁-C₄)Alkyl, (C₁-C₄)Haloalkyl, (C₁-C₄)Alkoxy und (C₁-C₄)Alkylthio substituiert ist, oder
[(C₁-C₄)Alkoxy]-carbonyl oder [(C₁-C₄)Haloalkoxy]-carbonyl
R⁵ Phenyl, das unsubstituiert oder durch einen oder mehrere Reste aus der Gruppe bestehend aus Halogen, Hydroxy, Nitro, Carboxy, Cyano, (C₁-C₄)Alkyl, (C₁-C₄)Haloalkyl, (C₁-C₄)Alkoxy-(C₁-C₄)alkyl, (C₁-C₄)Alkylthio-(C₁-C₄)alkyl, Hydroxy-(C₁-C₄)alkyl, (C₁-C₄)Alkoxy, (C₁-C₄)Alkoxy-(C₁-C₄)alkoxy, (C₁-C₄)Alkylthio, [(C₁-C₄)Alkoxy]-carbonyl, [(C₁-C₆)Haloalkoxy]-carbonyl, (C₁-C₄)Alkylsulfinyl, (C₁-C₄)Haloalkylsulfinyl, (C₁-C₄)Alkylsulfonyl, (C₁-C₄)Haloalkylsulfonyl, (C₃-C₆)Cycloalkyl, (C₃-C₆)Cycloalkyloxy, Phenyl und Phenoxy,
wobei jeder der letztgenannten 4 Reste unsubstituiert oder durch einen oder mehrere Reste aus der Gruppe bestehend aus Halogen, (C₁-C₄)Alkyl und (C₁-C₄)Haloalkyl substituiert ist,
substituiert ist und wobei zwei benachbarte Substituenten einen ankondensierten 5- oder 6-gliedrigen Ring bilden können, welcher carbocyclisch ist oder noch 1 bis 3 Heteroringatome aus der Gruppe N, O und S enthalten kann und der unsubstituiert oder durch einen oder mehrere Reste aus der Gruppe Halogen und (C₁-C₄)Alkyl substituiert ist,
oder
einen 5- oder 6-gliedrigen heteroaromatischen Rest mit 1 bis 3 Heteroringatomen aus der Gruppe N, O und S,
der unsubstituiert oder durch einen oder mehrere Reste aus der Gruppe bestehend aus Halogen, Hydroxy, Carboxy, Cyano, (C₁-C₄)Alkyl, (C₁-C₄)Haloalkyl, (C₁-C₄)Alkoxy-(C₁-C₄)alkyl, (C₁-C₄)Alkylthio-(C₁-C₄)alkyl, Hydroxy-(C₁-C₄)alkyl, (C₁-C₄)Alkoxy, (C₁-C₄)Alkoxy-(C₁-C₄)alkoxy, (C₁-C₄)Alkylthio, [(C₁-C₆)Alkoxy]-carbonyl, [(C₁-C₆)Haloalkoxy]-carbonyl, (C₁-C₄)Alkylsulfinyl, (C₁-C₄)Haloalkylsulfinyl, (C₁-C₄)Alkylsulfonyl, (C₁-C₄)Haloalkylsulfonyl und (C₃-C₆)Cycloalkyl, das unsubstituiert oder durch einen oder mehrere Reste aus der Gruppe bestehend aus Halogen, (C₁-C₄)Alkyl und (C₁-C₄)Haloalkyl substituiert ist,
substituiert ist, und
(R⁶)ₙ n Substituenten R⁶, wobei R⁶, im Falle dass n = 1 ist, oder jeder der Substituenten R⁶ unabhängig voneinander, im Falle dass n größer als 1 ist, einen Rest Halogen, Methyl, Ethyl, CF₃, Methoxy, Ethoxy, Methylthio, Methylsulfinyl, Ethylsulfinyl, Methylsulfonyl oder Ethylsulfonyl bedeutet, und
n 0, 1, 2, 3 oder 4,
bedeuten.

5. Verbindungen der Formel (I) oder deren Salze wie sie nach einem der Ansprüche 1 bis 4 definiert sind, ausgenommen ausgenommen die Verbindungen
3,5-Diphenyl-1-(3-pyridyl)-pyrazol-4-essigsäure,
3,5-Diphenyl-1-(3-pyridyl)-pyrazol-4-essigsäureethylester,
3-Methyl-5-phenyl-1-(3-pyridyl)-pyrazol-4-essigsäure,
3-Methyl-5-phenyl-1-(3-pyridyl)-pyrazol-4-essigsäureethylester und
Salze der genannten Verbindungen.

6. Verfahren zur Herstellung von Verbindungen der Formel (I) oder deren Salze nach Anspruch 5, **dadurch gekennzeichnet, dass** man
(a) eine Verbindung der Formel (II), 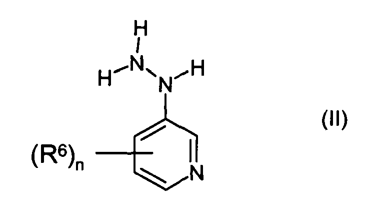 worin (R⁶)ₙ wie in Formel (I) definiert ist,
mit einer Verbindung der Formel (III), 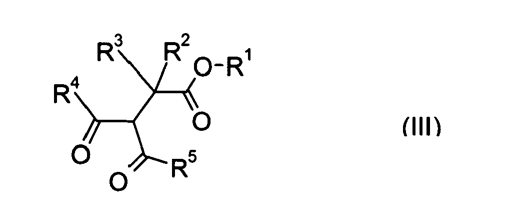 worin R¹, R², R³, R⁴ und R⁵ wie in Formel (I) definiert sind,
zur Verbindung der Formel (I) oder deren Salz umsetzt oder
(b) im Falle, dass R¹ in Formel (I) von Wasserstoff verschieden ist, eine Verbindung der Formel (I'), 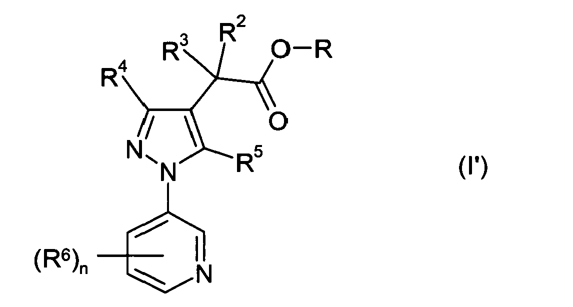 in welcher R¹, R², R³, R⁴ und R⁵ wie in Formel (I) definiert sind und
R einen vom Rest R¹ unterschiedlichen, von Wasserstoff verschiedenen Rest, der aus der Gruppe der Reste, wie sie für R¹ definiert ist, ausgewählt ist, oder ein Anhydrid, Säurehalogenid oder einen aktivierten Ester der Verbindung der Formel (I'), worin R = H bedeutet,
bedeutet,
mit einer Verbindung der Formel (IV),
R¹ - OH (IV)
worin R¹ wie in Formel (I) definiert ist,
zur Verbindung der Formel (I) umsetzt oder
(c) im Falle, dass R¹ in Formel (I) von Wasserstoff verschieden ist, eine Verbindung der Formel (I") 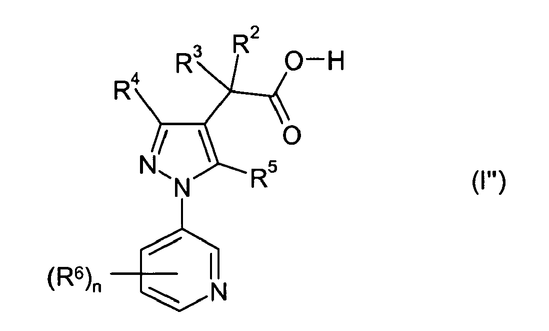 in welcher R², R³, R⁴ und R⁵ wie in Formel (I) definiert sind,
gegebenenfalls nach Aktivierung der Säuregruppe, mit einer Verbindung der Formel (IV),
R¹-OH (IV)
worin R¹ wie in Formel (I) definiert ist,
zur Verbindung der Formel (I) verestert oder
(d) im Falle, dass die Verbindung der Formel (I), worin R = H, oder deren Salz hergestellt wird, eine Verbindung der Formel (I') [siehe Definition in Variante (b)] zur Verbindung der Formel (I) oder deren Salz hydrolysiert.

7. Herbizides oder pflanzenwachstumsregulierendes Mittel, **dadurch gekennzeichnet, dass** es eine oder mehrere Verbindungen der Formel (I) oder deren Salze, wie sie nach einem der Ansprüche 1 bis 5 definiert sind, und im Pflanzenschutz übliche Formulierungshilfsmittel enthält.

8. Verfahren zur Bekämpfung von Schadpflanzen oder zur Wachstumsregulierung von Pflanzen, **dadurch gekennzeichnet, dass** man eine wirksame Menge von einer oder mehreren Verbindungen der Formel (I) oder deren Salzen, wie sie nach einem der Ansprüche 1 bis 5 definiert sind, auf die Pflanzen, Pflanzensamen oder die Anbaufläche appliziert.

9. Verfahren nach Anspruch 8, **dadurch gekennzeichnet, dass** die Verbindungen der Formel (I) oder deren Salze zur Bekämpfung von Schadpflanzen oder zur Wachstumsregulierung in Kulturen von Nutz- oder Zierpflanzen eingesetzt werden.

10. Verfahren nach Anspruch 9, **dadurch gekennzeichnet, dass** die Kulturpflanzen transgene Kulturpflanzen sind.
